(19) Europäisches Patentamt / European Patent Office / Office européen des brevets

(11) **EP 4 520 346 A1**

(12) **EUROPEAN PATENT APPLICATION**

(43) Date of publication:
12.03.2025 Bulletin 2025/11

(21) Application number: 23461647.2

(22) Date of filing: 06.09.2023

(51) International Patent Classification (IPC):
*A61K 39/00* (2006.01)    *C07K 14/705* (2006.01)
*C07K 14/725* (2006.01)

(52) Cooperative Patent Classification (CPC):
**A61K 40/11; A61K 40/31; A61K 40/4202;
A61K 40/4215;** A61K 2239/22; A61K 2239/28;
A61K 2239/48; C07K 14/7051; C07K 14/70521;
C07K 14/70578; C07K 2319/03

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Gyncentrum Sp. z o.o.**
**40-851 Katowice (PL)**

(72) Inventors:
• **Bednarska-Czerwinska, Anna**
 **40-851 Katowice (PL)**
• **Morawiec, Emilia**
 **40-851 Katowice (PL)**
• **Pudelko, Adam**
 **40-851 Katowice (PL)**
• **Nowak, Ewa**
 **40-851 Katowice (PL)**

• **Polak, Agnieszka**
 **40-851 Katowice (PL)**
• **Broncel, Mateusz**
 **40-851 Katowice (PL)**
• **Matczynska, Daria**
 **40-851 Katowice (PL)**
• **Czerwinski, Michal**
 **40-851 Katowice (PL)**
• **Czerwinski, Pawel**
 **40-851 Katowice (PL)**

(74) Representative: **Patpol Kancelaria Patentowa Sp.
z o.o.
Nowoursynowska 162J
02-776 Warszawa (PL)**

Remarks:
The complete document including Reference
Table(s) and the Sequence Listing(s) can be
downloaded from the EPO website

(54) **CELLS, METHODS AND SYSTEMS FOR USE IN THE TREATMENT OF A CANCER WITH
DUAL-CAR-T**

(57)    The present application provides a T cell that co-expresses at least a first chimeric antigen receptor (CAR) and second CAR at the cell surface, each CAR comprising: (i) an extracellular antigen-binding domain; (ii) a spacer; (iii) a linking/trans-membrane domain; and (iv) an endodomain, wherein the antigen binding domains of the first and second CAR bind to different antigens, and wherein the first CAR is directed against the group C group 5 member D orphan G protein-coupled receptor and the second CAR is directed against B cell maturation antigen. Furthermore, the present application discloses a set of at least two nucleic acid sequences, a system of two vectors, a kit comprises thereof, a method for generating said T cell, a composition comprising said T cells, and a method for treating and/or preventing a disease. Additionally, said application provides a system of at least two CARs for expression on a T cell surface.

FIG. 1

EP 4 520 346 A1

**Description**

TECHNICAL FIELD OF INVENTION

The third generation dual-CAR-T containing cell, method and system for use in the cancer treatment, in particular for use in the treatment of multiple myeloma. Namely, the present invention relates to a new manufacturing protocol for multiple myeloma (MM) potential immunotherapy based on third generation dual-CAR-T to offer solutions for relapsed patients especially with BCMA antigen escape mechanism and other current challenges of CAR-T cell therapy in MM.

BACKGROUND OF INVENTION

The development of multiple myeloma is associated with the excessive growth of abnormal plasma cells. Despite the use of triplet and quadruplet induction regimens, autologous stem cell transplant, and maintenance therapy, myeloma remains incurable with relapses invariably occurring at different stages in the course of the disease (Bhatt P, Kloock C, Comenzo R. Relapsed/Refractory Multiple Myeloma: A Review of Available Therapies and Clinical Scenarios Encountered in Myeloma Relapse. Curr Oncol. 2023 Feb 15;30(2):2322-2347).

Therapies using chimeric antigen receptors (CARs) has given new hope for the treatment of B-cell malignancies, however, despite recent therapeutic advances, the prognosis of patients with multiple myeloma remains unfavorable. The main indication for CAR-T cell therapy in MM would be limited to patients with relapsed/refractory MM after at least two lines of prior therapy that included PIs (proteasome inhibitors), iMIDs (immunomodulatory agents, e.g., lenalidomide, pomalidomide), and anti-C38 monoclonal antibodies (N. Kröger et al. (eds.), The EBMT/EHA CAR-T Cell Handbook).

The CAR - chimeric antigen receptor is a genetically modified fusion protein, synthetic molecule that is created by combining the single-chain variable fragment domain (scFv) from an antibody, specific to a target antigen, with a signaling and activating domain of the zeta subunit of the CD3 complex, in a single multidomain polypeptide. Recognition of the target antigen by CAR-containing T cells (CAR-T) leads to their activation independently of the proteins included in the histocompatibility complex. The transmembrane domain (TMD), like the hinge domain (HD), is a component in the CAR structure that connects the antigen recognition moiety to the intracellular signaling domain. It is mainly derived from type-I single-spanning proteins, such as CD3$\zeta$, CD4, CD8$\alpha$, or CD28 (Mazinani, M., Rahbarizadeh, F. CAR-T cell potency: from structural elements to vector backbone components. Biomark Res 10, 70 (2022)).

There are currently four generations of the CARs. The first generation is a fusion protein composed of an intracellular signaling domain (endodomain) containing CD3$\zeta$, a transmembrane domain, and an extracellular domain containing the antigen-specific (murine or humanized) single-chain variable fragment (variable Fab-scFv) of an immunoglobulin (Ig) that binds to the appropriate protein in the membrane of tumor cells, which is followed by a hinge or spacer domain (frequently from the Ig constant heavy regions or from the CD8 molecule). The second generation has an additional costimulatory molecule, i.e. CD28, CD137, CD134, 4-1BB or OX-40, bound to CD3$\zeta$. In the third generation, the signal fragment is associated with two distinct co-stimulatory domains which are fused finally to the signaling. The fourth generation CARs are based on second-generation constructs which are additionally modified with a constitutive or inducible expression cassette containing a transgenic protein such as a cytokine - important from pathophysiological point of view. These are called T cell redirected for universal cytokine-mediated killing (TRUCK) CAR-T (Choi T, Kang Y. Chimeric antigen receptor (CAR) T-cell therapy for multiple myeloma. Pharmacol Ther. 2022 Apr; 232: 108007, Bruno B et al. European Myeloma Network perspective on CAR T-Cell therapies for multiple myeloma. Haematologica. 2021 Aug 1; 106 (8): 2054-2065, Tomasik J, Jasiński M and Basak GW (2022) Next generations of CAR-T cells - new therapeutic opportunities in hematology? Front. Immunol. 13:1034707.

CAR-T therapy relies on genetic modification of patient lymphocytes T to improve the immune system response against e.g., hematological malignances. An artificial receptor, additionally connected to lymphocyte activation domains, involved in the recognition of cancer cells, is introduced into the cells of the immune system by means of an appropriate vector. Effector cells modified in this way are given to the patient to actively search for cancer cells in the patient's body (Huang J, Huang X, Huang J. CAR-T cell therapy for hematological malignancies: Limitations and optimization strategies. Front Immunol. 2022 Sep 28; 13:1019115).

Several CD19-directed CAR-T cell therapies have been approved for clinical trials (Ghobadi 2018; Yassine et al. 2020). CD19 expression at surface of plasma cells is limited or absent, leading to uncertain efficacy in clinical trials that used anti-CD19 CAR-T therapy in MM patients (Garfall et al. 2015, 2019). An extremely important approach is the use of dual constructs, i.e., targeting different antigens presented on the surface of the cancer cell, which reduces or even excludes the potential possibility of multiple myeloma cells escaping from therapy targeting only single cancer antigen (Leow CC, Low MSY. Targeted Therapies for Multiple Myeloma. J Pers Med. 2021 Apr 23; 11(5):334). One of the main mechanisms is antigen downregulation or antigen loss under therapeutic pressure. Thus, targeting multiple different surface antigens is an effective strategy to prevent antigen-negative escape, and new potent alternative targets are continuously being identified (Zhang X, Zhang H, Lan H, Wu J, Xiao Y. CAR-T cell therapy in multiple myeloma: Current limitations and potential strategies. Front Immunol. 2023 Feb 20; 14: 1101495).

Vectors for expressing chimeric antigen receptors and their uses are known in the art.

In particular, WO 2018/200582 A1 discloses chimeric antibody-TCR constructs comprising an antigen-binding module that binds to a target antigen and a T-cell receptor module capable of recruiting at least one TCR-associated signaling molecule. One preferred embodiment of the present invention provides a caTCR comprising an antigen binding module that specifically binds to a cell surface antigen, wherein the cell surface antigen is CD19, CD20, CD22, CD47, GPC-3, ROR1, ROR2, BCMA, GPRC5D or FCRL5, including variants or mutants thereof. Furthermore, in some embodiments, the caTCR further comprises at least one additional intracellular domain comprising a costimulatory T cell signal sequence selected from, but not limited to, CD27, CD28, 4-1BB (CD137), OX40, CD30, CD40, ICOS.

International application WO 2015/142675 A2 relates to the treatment of cancer using chimeric antigen receptors. More specifically, this invention also relates to a chimeric antigen receptor (CAR) specific for a cancer-associated antigen, vectors encoding them, and recombinant T cells containing CAR. Similarly, WO 2017/172981 A2 discloses a composition that comprises a cell comprising nucleic acids encoding a chimeric antigen receptor (CAR) and one or more signaling proteins.

Other approach in CAR therapy is creating binders that are split between two, or more, different CAR constructs. expressed in the same cell.

In particular, US 11,208,455 B2 patent document discloses novel therapeutic immunotherapy compositions comprising at least two vectors, each vector encoding a functional CAR, whereby the combination of vectors results in the expression of two or more non - identical binding domains, wherein each vector encoded binding domain (s) are covalently linked to a transmembrane domain and one or more non - identical intra cellular signaling motifs are provided herein as well as are methods of use of same in a patient -specific immunotherapy that can be used to treat cancers and other diseases and conditions. By "disease" is meant any condition or disorder that damages or interferes with the normal function of a cell, tissue, or organ. Examples of diseases include neoplasia or pathogen infection of cell.

Alike, EP 3 071 223 B1 patent document relates to a cell which comprises more than one chimeric antigen receptor (CAR). The cell is capable of specifically recognizing a target cell, due to a differential pattern of expression (or non-expression) of two or more antigens by the target cell.

Unfortunately, CAR-T infusion is usually associated with the occurrence of severe side effects, such as a drop in blood pressure, fever, muscle pain, respiratory failure or impaired consciousness. It is possible that the sudden increase in the concentration of pro-inflammatory cytokines in the bloodstream and cerebrospinal fluid leads to damage to the blood-brain barrier. Neurotoxicity observed in patients treated with CAR-T cells is manifested by, among others, headaches, speech disorders, visual hallucinations.

Moreover, the patent documents set forth above do not disclose the specific and unique configuration of the individual domains of a given construct with specific properties allowing to overcome the disadvantages known from the state of the art, such as the mentioned adverse effects or even death of the patient, occurrence of antigenic escape, high mortality and reduced T-cell functionality associated with viral transduction.

Since myeloma remains incurable with relapses invariably occurring at different stages of treatment, there is an urgent and long felt need in the art for discovering compositions and methods for treatment of cancer using a CAR -based therapy that can exhibit cancer - specific intended therapeutic attributes without the aforementioned shortcomings, and with improved immune system responses against hematological malignancies.

## SUMMARY OF THE INVENTION

The present invention addresses these needs by providing an engineered T cells expressing the CAR combinations that are exquisitely specific for cancer cells, based on their particular expression of two or more markers.

Thus, the present invention provides A T cell that co-expresses at least a first chimeric antigen receptor (CAR) and second CAR at the cell surface, each CAR comprising:

(i) an extracellular antigen-binding domain; (ii) a spacer; (iii) a linking/trans-membrane domain; and (iv) an endodomain,

wherein the antigen binding domains of the first and second CAR bind to different antigens, and wherein the first CAR is directed against the group C group 5 member D orphan G protein-coupled receptor and the second CAR is directed against B cell maturation antigen. Preferably, T cell is selected from a group comprising CD3+, CD4+, or CD8+ T cells.

The present invention also discloses a set of at least two nucleic acid sequences comprising nucleic acid sequences encoding the first and the second CAR as defined. Preferably, the first CAR encoding sequence comprises a sequence set forth in SEQ NO ID: 2, or a variant thereof having at least 80% sequence identity, and the second CAR encoding sequence comprises a sequence set forth in SEQ NO ID: 4, or a variant thereof having at least 80% sequence identity, and wherein the said first and second CAR encoding sequences, when expressed in a T cell, present the said non-identical first and second CAR independently co-expressed on a T cell surface.

In one embodiment of the invention, the first CAR encoding sequence consists of a sequence set forth in SEQ NO ID: 2, or a variant thereof having at least 80% sequence identity, and the second CAR encoding sequence consists of a sequence set forth in SEQ NO ID: 4, or a variant thereof having at least 80% sequence identity.

The present invention further discloses a system of two vectors, comprising a first vector and a second vector, wherein the first vector comprises a nucleic acid sequence encoding the first CAR as defined above, wherein the said first vector has the following structure

anti-GPRC5D scFv (ET150-8) h (ICOS-4-1BB-CD3ζ), in which anti-GPRC5D scFv is the nucleic acid sequence encoding the antigen binding domain of the first CAR, ICOS is the nucleic acid sequence encoding the transmembrane domain of the first CAR, and 4-1BB-CD3ζ is the nucleic acid sequence encoding the endodomain of the first CAR,

and the second vector comprises a nucleic acid sequence encoding the second CAR as defined, wherein the second vector has the following structure

anti-BCMA scFv (25D2) h (CD28-OX40-CD3ζ), in which anti-BCMA scFv is the nucleic acid sequence encoding the antigen binding domain of the second CAR, CD28 is the nucleic acid sequence encoding the transmembrane domain of the second CAR, and OX40-CD3ζ is the nucleic acid sequence encoding the endodomain of the second CAR. Preferably, the first vector comprises the first nucleic acid sequence comprising a sequence as set forth in SEQ NO ID: 1 or a variant thereof having at least 80% sequence identity, and the second vector comprises the second nucleic acid sequence comprising a sequence as set forth in SEQ NO ID: 3 or a variant thereof having at least 80% sequence identity. More preferably, the first vector comprises the first nucleic acid sequence consisting of a sequence as set forth in SEQ NO ID: 1 or a variant thereof having at least 80% sequence identity, and the second vector comprises the second nucleic acid sequence consisting of a sequence as set forth in SEQ NO ID: 3 or a variant thereof having at least 80% sequence identity.

The present invention discloses a kit which comprises

(i) a first nucleic acid sequence encoding a first CAR as defined, and (ii) a second nucleic acid sequence encoding a second CAR as defined, and wherein the kit further comprises instructions for use. Preferably, the said kit comprises: the first nucleic acid sequence the second nucleic acid sequence as defined.

In one embodiment, the said kit comprises a first vector and a second vector as defined.

The present invention discloses a method for generating a T cell as defined, which comprises a step of introducing into a T cell *ex vivo:* a set of nucleic acid sequences as defined. Preferably, the step of introducing the set of nucleic acid sequences into a T cell *ex vivo* is carried out via a process of viral transduction. More preferably, the viral transduction is carried out using a lentivirus particles and a system of vectors as defined.

In one embodiment, the T cell is from a sample isolated from a subject, preferably said sample is a whole blood sample.

As described herein, the subject, including humans or non-human mammals, is a subject suffering from blood and hematopoietic system cancer.

In another embodiment said method comprises additional steps of cell activation, specific interleukin stimulations, and/or cell expansion following before the step as specified above is carried out.

The present invention further discloses a composition comprising a population of human T cells comprising a plurality of the T cells as defined. Preferably, said T cells comprise a combination of at least two vectors, wherein the first vector comprises a first nucleic acid sequence encoding the first CAR, and a second vector comprises a second nucleic acid sequence encoding the second CAR,

wherein the combination of said vectors results in the co-expression of at least two non-identical CARs at the T cell surface, and

said vectors are used to genetically modify human T cell populations.

The present invention discloses an immunotherapeutic composition comprising an antitumor effective amount of the composition as defined above.

By effective amount is meant an amount sufficient to arrest, ameliorate, or inhibit the continued proliferation of cancer cells, preferably blood and hematopoietic system cancer cells.

Preferably, said composition further comprises a pharmaceutically acceptable carrier, diluent and/or excipient.

The present invention discloses a composition for use in treating and/or preventing a disease. Preferably, the disease is cancer.

The present invention further discloses a method for treating and/or preventing a disease, which comprises a step of administering to a subject the composition as defined. Preferably, said method comprises the following steps:

(i) isolation of a T cell from the subject, (ii) transduction of the T cell obtained in step (i) with at least two nucleic acid sequences encoding the first and second CARs or at least two vectors comprising such nucleic acid sequences so that the first and the second CAR are independently co-expressed on a T cell surface; and (iii) administering the T cell from (ii) to the subject.

The present invention also discloses a system of at least two CARs for expression on a T cell surface, comprising a first CAR and a second CAR, wherein each CAR comprises:

(i) an extracellular antigen-binding domain; (ii) a spacer; (iii) a linking/trans-membrane domain; and (iv) an endodomain,

and wherein the antigen binding domains of the first and second CARs bind to different antigens, and wherein the first CAR is directed against the group C group 5 member D orphan G protein-coupled receptor and the other second CAR is directed against B cell maturation antigen. Preferably, the first CAR is encoded by a nucleic acid sequence comprising a sequence as set forth in SEQ NO ID: 2, or a variant thereof having at least 80% sequence identity, and the second CAR is encoded by a second nucleic acid sequence comprising a sequence as set forth in SEQ NO ID: 4, or a variant thereof having at least 80% sequence identity,

and said the first and second nucleic acid sequences, when expressed in a T cell, encode non-identical said the first and second CARs independently co-expressed on a T cell surface.

In one embodiment, the first CAR encoding sequence consists of a sequence set forth in SEQ NO ID: 2, or a variant thereof having at least 80% sequence identity, and the second CAR encoding sequence consists of a sequence set forth in SEQ NO ID: 4, or a variant thereof having at least 80% sequence identity.

In another embodiment, the first CAR encoding sequence is a part of a first vector having the following structure

anti-GPRC5D scFv (ET150-8) h (ICOS-4-1BB-CD3ζ), in which

anti-GPRC5D scFv is the nucleic acid sequence encoding the antigen binding domain of the first CAR, ICOS is the nucleic acid sequence encoding the transmembrane domain of the first CAR, and 4-1BB-CD3ζ is the nucleic acid sequence encoding the endodomain of the first CAR,

and the second CAR encoding sequence is a part of a second vector having the following structure

anti-BCMA scFv (25D2) h (CD28-OX40-CD3ζ), in which

anti-BCMA scFv is the nucleic acid sequence encoding the antigen binding domain of the second CAR, CD28 is the nucleic acid sequence encoding the transmembrane domain of the second CAR, and OX40-CD3ζ is the nucleic acid sequence encoding the endodomain of the second CAR.

In yet another embodiment, the first vector comprises the first nucleic acid sequence comprising a sequence as set forth in SEQ NO ID: 1 or a variant thereof having at least 80% sequence identity, and the second vector comprises the second nucleic acid sequence comprising a sequence as set forth in SEQ NO ID: 3 or a variant thereof having at least 80% sequence identity.

In one embodiment, the first vector comprises the first nucleic acid sequence consisting of a sequence as set forth in SEQ NO ID: 1 or a variant thereof having at least 80% sequence identity, and the second vector comprises the second nucleic acid sequence consisting of a sequence as set forth in SEQ NO ID: 3 or a variant thereof having at least 80% sequence identity.

BRIEF DESCRIPTION OF THE FIGURES

The following detailed description, given by way of example, but not intended to limit the invention to specific embodiments described, may be understood in conjunction with the accompanying drawings.

Fig. 1 presents immunophenotype of cultured T lymphocytes with TexMACS medium and the IL-7, and IL-15 (BD FASC Melody, Horus software).

Fig. 2 presents CD3+ T cells, EVOS FLoid Imaging System, magnification 20x, 460x (optical).

Fig 3 presents pLenti-EF1a-C-mGFP-P2A-Puro Lentiviral Gene Expression Vector map.

Fig. 4 shows pCDCAR1-Customized-Anti-GPRC5D scFv (ET150-8) CD8 hinge ICOS TM (ICOS-4-1BB-CD3ζ) CAR plasmid map.

Fig 5. shows result of electrophoretic analysis for the Anti-GPRC5D plasmid. A marker is depicted on the left panel.

Fig. 6 presents pCDCAR1-CAR-T-3-X1022-Anti-BCMA scFv (25D2) CD8 hinge CD8 TM (CD28-OX40-CD3ζ) CART plasmid map.

Fig. 7 shows result of electrophoretic analysis for the Anti-BCMA plasmid. A marker is depicted on the left panel.

Fig. 8 presents 293T cells 24h and 48 after transfection Anti-BCMA lentiviral plasmids in comparison to control untreated cells, Zeiss AxiovertAI

Fig. 9 presents 293T cells 24h and 48 after transfection Anti-GPRC5D lentiviral plasmids in comparison to control untreated cells, Zeiss AxiovertAI

Fig 10. shows results of the obtained lentivirus Anti-BCMA titer in the concentrated medium collected from the transfected 293T cells.

Fig. 11 shows results of the obtained lentivirus Anti-GPRC5D titer in the concentrated medium collected from the transfected 293T cells.

Fig. 12 presents immunophenotyping of co-culture of CD4+ and CD8+ cells (BD FACS Melody, Horus software).

Fig. 13 presents immunophenotyping of negative control.

Fig. 14 shows comparison of the efficiency of the co-transduction process depending on the time of the second Anti-GPRC5D transduction.

Fig. 15 presents the distribution of the CAR-T CD3+ population GFP+ and BCMA+ (BD FACS Melody, Horus Software

Fig. 16 shows characterization of Dual CAR-T and GFP CAR-T products. Comparison of the obtained products depending on the transduced T lymphocytes donor (ATCC cells LOT) and depending on different lentivirus titers used for transduction. MOI 50 for Anti GPRC5D lentivirus and MOI 50 for Anti-BCMA lentivirus, MOI 150 for GFP lentivirus.

Fig. 17 presents electropherogram of gDNA amplification products from Dual CAR-T cells with anti-BCMA and anti-GPRC5D sequence-specific primers.

Fig. 18 presents the result of the anti-BCMA sequence align with the reference sequence (pCDCAR1-LX-R100421-1C-1).

Fig. 19 shows the result of the anti-GPRC5D sequence align with the reference sequence (pCDCAR1-LX-R100421-1C-2).

Fig. 20 presents the result of the anti-BCMA sequence align with the reference sequence (pCDCARI-LX-R100421-1C-1).

Fig. 21 presents the result of the anti-GPRC5D sequence align with the reference sequence (pCDCAR1-LX-R100421-1C-2).

Fig. 22 shows the result of the anti-BCMA sequence align with the reference sequence (pCDCAR1-LX-R100421-1C-1).

Fig. 23 presents the result of the anti-GPRC5D sequence align with the reference sequence (pCDCAR1-LX-R100421-1C-2).

Fig. 24 shows the result of the anti-BCMA sequence align with the reference sequence (pCDCAR1-LX-R100421-1C-1).

Fig. 25 presents the result of the anti-GPRC5D sequence align with the reference sequence (pCDCAR1-LX-R100421-1C-2).

Fig 26. shows H929 cells. EVOS FLoid Imaging System, magnification 20x, 460x (optical).

Fig. 27 shows expression of BCMA antigen on H929 cells, flow cytometry (BD FACS Melody, Horus software).

Fig 28. shows expression of GPRC5D antigen on H929 cells, flow cytometry (BD FACS Melody, Horus software).

Fig 29. presents cells in co-culture and all control cells (1-21) after 24 hours of culture.

Fig 30. presents cells in co-culture and all control cells (1-21) after 48 hours of culture.

Fig 31. shows the profile of cytokines released by the cells present in the co-culture was analyzed after 24 h and 48 h. In this experiment, the cytokine release profile was assessed for non-transduced T cells, CAR-T cells (50.28 % transduced) and H929 cells (negative controls).

Fig 32. shows comparison of pro-inflammatory and anti-inflammatory cytokine secretion (concentration [pg/ml]) Mono Anti-BCMA CAR-T and Dual CAR-T in relation to control GFP+ CAR-T in co-culture with B lymphocytes isolated from malignant exudate of a 62-year-old patient with myeloma plasma cell-line H929, NCI-H929, CRL-9068™.

## DETAILED DESCRIPTION OF THE INVENTION

Current challenges in the more widespread and effective adaptation of CAR therapy for cancer relate to a paucity of compelling targets.

CAR-encoding nucleic acids may be transferred to T cells using, for example, retroviral vectors. Lentiviral vectors may be employed. In this way, a large number of cancer-specific T cells can be generated for adoptive cell transfer. When the CAR binds the target-antigen, this results in the transmission of an activating signal to the T-cell it is expressed on. Thus, the CAR directs the specificity and cytotoxicity of the T cell towards tumor cells expressing the targeted antigen.

Importantly, the dual CAR specificity can be obtained either by simultaneous transduction of two separate CAR constructs into T cells or using a single bicistronic vector containing two separate CAR constructs. However, the last option usually is connected with low transduction efficiencies because of the size of the construct.

The present disclosure relates to an immunological cell which co-expresses the first CAR and second CAR at the cell surface such that an immunological cell can recognize a desired pattern of expression on target cells. Each CAR comprise an extracellular antigen-binding domain; a spacer; a linking/trans-membrane domain; and an endodomain/intracellular signaling domain.

Numerous antigen-binding domains are known in the art, including those based on the antigen binding site of an antibody, antibody mimetics, and T-cell receptors. For example, the antigen-binding domain may comprise: a single-chain variable fragment (scFv) derived from a monoclonal antibody; a natural ligand of the target antigen; a peptide with sufficient affinity for the target; a single domain antibody; an artificial single binder such as a Darpin (designed ankyrin repeat protein); or a single-chain derived from a T-cell receptor. Moreover, the antigen binding domain may be based on a natural ligand of the antigen.

The antigen binding domains of the first and second CARs of the present invention, bind to different antigens, in particular, the first CAR is directed against the group C group 5 member D orphan G protein-coupled receptor and the other CAR is directed against B cell maturation antigen.

Therefore, the present invention relates to chimeric antigen receptors, BCMA/CD28/OX40 and GPRC5D/4-1BB/ICOS encoded on two separate CAR constructs, that simultaneously target two different antigens present on the surface of multiple myeloma (MM) cells with increased co-stimulation and metabolic efficiency of T cells. The last is realized by independent activation of the CD28, OX40 and ICOS, 4-1BB pathways, and fine-tuning CD3ζ-chain-mediated signaling, thereby demonstrating unexpected benefit in preventing MM cell escape from anti-BCMA CAR-T targeted therapy. The second therapeutic target, GPRC5D was chosen after deep analysis of proteomic databases and open access transcriptome datasets, based on the obtained ranking system for possible single and dual antigen immunotherapy targets, and based on criteria related to the abundance and specificity for the surface of plasma cells. The present invention concerns the use of four costimulatory domains i.e., third-generation CAR particles in a dual-CAR systems i.e., using two independent constructs with high therapeutic potential for multiple myeloma.

The B cell maturation antigen (BCMA, alternative name is CD269) is highly expressed in both malignant plasma cells and normal plasma cells. BCMA is a type III transmembrane glycoprotein belonging to the tumor necrosis factor receptor superfamily. Its function is to regulate the proliferation, maturation, survival and differentiation of B cells into plasma cells by binding their ligands, proliferation-inducing ligand (APRIL) and B-cell activating factor (BAFF) (Yu B, Jiang T, Liu D. BCMA-targeted immunotherapy for multiple myeloma. J Hematol Oncol. 2020 Sep 17; 13(1): 125). Compared to normal plasma cells, BCMA expression on malignant plasma cells is much elevated especially during disease progression from monoclonal gammopathy of undetermined significance to active MM. Studies from BCMA-knockdown mice further indicate that BCMA is most important for long-lived plasma cells survival but is dispensable for overall B cell homeostasis. A recent study showed that an enzyme, $\gamma$-secretase can cleave membrane BCMA, leading to protein shedding and formation of soluble form BCMA (sBCMA).

Early clinical results with CAR-T cell therapy targeting the B-cell maturation antigen (BCMA) for multiple myeloma are promising, but relapses associated with low or negative BCMA-expressing MM cell remnants have been reported, requiring the identification of additional therapeutic targets. Multispecific CAR-T cells or combinations of monospecific targeted immunotherapies are going to overcome antigen loss in future trials (N. Kröger et al. (eds.), The EBMT/EHA CAR-T Cell Handbook,). In terms of CAR-T cell therapy, there are currently two FDA-approved CAR-T products, both of which target anti-B cell maturation antigen (BCMA), idecabtagene vicleucel (ide-cel) and ciltacabtagene autoleucel (cilta-cel). As a second-generation CAR T-cell, ide-cel contains a co-stimulatory domain from 4-1BB, which allows for better cytokine production and proliferation and persistence of the CAR T-cells (Anderson LD Jr. Idecabtagene vicleucel (ide-cel) CAR T-cell therapy for relapsed and refractory multiple myeloma. Future Oncol. 2022 Jan;18(3):277-289). The endodomain or a signaling cytoplasmic domain of cilta-cel entail a T-cell activation domain CD3$\zeta$ and a costimulatory domain 4-1BB >Chekol Abebe E, Yibeltal Shiferaw M, Tadele Admasu F, Asmamaw Dejenie T. Ciltacabtagene autoleucel: The second anti-BCMA CAR T-cell therapeutic armamentarium of relapsed or refractory multiple myeloma. Front Immunol. 2022 Sep 2; 13:991092, Rajkumar SV. Multiple myeloma: 2022 update on diagnosis, risk stratification, and management. Am J Hematol. 2022 Aug; 97(8): 1086-1107).

Costimulatory signals are necessary for the proper functioning, maintenance of durability and stabilization of the anticancer activity of T lymphocytes modified with chimeric antigen receptors. Activation of intracellular signaling domains is achieved by introducing at least one, and usually more than one, T-cell costimulatory molecule within the CAR construct. The structure of the intracellular domain of costimulatory receptors determines, at least in part, their functional performance. Therefore, it is important to carefully choose and place co-stimulatory domains in the CAR construct, which affects the activity of modified lymphocytes, cytotoxic function, and, subsequently, the safety of therapy based on their use. So far, it has been shown that the secretion profile of T lymphocytes modified with CAR particles containing sequences encoding CD28 and OX40 (TNF receptor superfamily members CD134) co-stimulatory domains are associated with effective control of cancer cells eradication (including lymphomas), as well as with stable expression of the CAR related molecules on the cell membrane of T cells and high production of cytokines IFN-$\gamma$, TNF-$\alpha$ and IL2 (Th1 profile). However, such co-stimulatory domains are not free from defects. CD28 may augment early exhaustion which limits the associated anti-tumor efficacy and could be responsible for their short-lived persistence *in vivo.* However, the 4-1BB co-stimulatory domain ameliorates the onset of CAR-T cell exhaustion (Honikel MM, Olejniczak SH. Co-Stimulatory Receptor Signaling in CAR-T Cells. Biomolecules. 2022 Sep 15; 12(9): 1303, Guercio M, et al. CD28.OX40 co-stimulatory combination is associated with long in vivo persistence and high activity of CAR.CD30 T-cells. Haematologica 2020; 106(4): 987-999;).

CD4+ and CD8+ T cells have been shown to exhibit different phenotypic persistence depending on the introduced signaling domains used to generate CARs. It is also important that at least two stimulatory signals are required for optimal T cell activation. The first is delivered by the T-cell receptor (TCR) complex after antigen recognition, and further, additional co-stimulatory signals are provided by the involvement of co-stimulatory receptors, including the co-stimulatory receptor CD28 and ligand B7-1 (CD80). This surface glycoprotein specific for T cells is involved in their activation after ligation by B7-1 and B7-2 (CD86) on antigen presenting cells (APCs), induces cell proliferation and cytokine production, enhances IL-4 and IL-10 production in T cells in combination with TCR/CD3 ligation and CD40L co-stimulation. According to literature data, the CD28 molecule is expressed in approximately 80% of human CD4+ T cells and 50% of CD8+ T cells. The percentage of CD28-positive T cells in humans decreases with age. Therefore, it is interesting to use CD28 costimulatory domain in the CAR-T therapy of multiple myeloma in the elderly. CD28 co-stimulation has various effects on T lymphocyte function, e.g., on biochemical changes in the immune synapse, phosphorylation and other post-translational modifications, transcriptional changes and remodeling of the cytoskeleton. CD28 signals increase the rate of cell glycolysis, allowing cells to generate the energy necessary for their growth and proliferation. CD28 mediates myriad functions through two motifs in its cytoplasmic tail: YMNM and PYAP42. The proximal YMNM motif associates with the p85 subunit of phosphatidylinositol kinase 3 (PI3K), a common signaling intermediate, to initiate targeting of AKT (protein kinase B, PKB), which then activates several downstream molecules. The CD28-PI3K-AKT pathway promotes T cell proliferation and survival by activating downstream targets nuclear factor-κB (NF-κB), nuclear factor-activated T lymphocytes (NFAT), BCL-XL, mTOR, GLUT1, and others (Cappell, K.M., Kochenderfer, J.N. A comparison of chimeric antigen receptors containing CD28 versus 4-1BB costimulatory domains. Nat Rev Clin Oncol 18, 715-727 (2021)). In addition, it is also important that the family of the discussed glycoprotein CD28 also includes other molecules: ICOS, CTLA4, PD1, PD1H, TIGIT and BTLA, some of which exert an opposing effect on T-cell stimulation. CD28 exerts an activating signal, while CTLA4, which also binds to B7-1, exerts an inhibitory signal showing dichotomous mode of action based on specific signaling.

Another receptor is the OX40 receptor (TNFSF4), a member of the TNF superfamily of receptors, which activates NF-kappaB by interacting with the adapter proteins: TRAF2 and TRAF5. OX40 is expressed on activated T cells, primarily and lower expressions are reported on natural killer cells (NK) and natural killer T cells. The ligand of OX40, OX40L (CD134L or CD252) - tumor necrosis factor superfamily member 4 (TNFSF4), is expressed in antigen-presenting cells (APCs). In addition, OX40 is a costimulatory molecule involved in long-term T-cell immunity, it also promotes the expression of the apoptosis inhibitors BCL2 and BCL21L1/BCL2-XL and thus promotes lymphocytes survival (Aliya I Sani, Zil-e-Rubab, Shumaila Usman, Syed Zaryab Ahmed, Mervyn Hosein. Role of OX40 and its ligand as costimulatory modulators in cancer immunotherapy. AIMS Molecular Science, 2021, 8(3): 161-173). Moreover, it is already known that the CD28 and OX40 co-stimulatory combination is ultimately responsible for the anti-tumor efficacy of the approach, paving the way to translate this therapeutic strategy into clinical use for patients with CD30+ Hodgkin lymphoma (HL) and non-Hodgkin lymphoma (NHL) (Guercio M, et al. CD28.OX40 co-stimulatory combination is associated with long in vivo persistence and high activity of CAR.CD30 T-cells. Haematologica 2020; 106(4):987-999).

Costimulatory receptors have been shown to recruit multiple proximal signaling molecules to activate several downstream pathways, often via multiple routes, therefore the function of costimulatory receptors depends not only on the spectrum of proximal signaling molecules recruited but also on the versatility of the distal signaling pathways activated.

The differential expression of BCMA on the surface of multiple myeloma cells leads to a differential response to targeted therapy and, in some cases, to relapse. Therefore, it is necessary to use alternative targets in the treatment of relapsed or refractory multiple myeloma. GPRC5D expression is BCMA independent, makes it significant for preventing possible MM relapse. In particular, the second construct, anti-GPRC5D, is directed against the group C group 5 member D orphan G protein-coupled receptor, which is a relatively new target for multiple myeloma (MM) immunotherapy. Normally, its expression is restricted to hair follicles, but is abundantly expressed in multiple myeloma cells.

Although, GPRC5D has already been proposed as a suitable target for CD3-mediated redirection of T cells, this approach does not solve the problems known from the state of the art, related to the phenomenon of antigen escape, recurrence of the disease and the inability to achieve long-term effectiveness of a given therapy.

The first an anti-GPRC5D and CD3 bispecific antibody, talquetamab, facilitates the interaction of T cells and GPRC5D-expressing myeloma cells and subsequently leads to the death of the multiple myeloma cells. Preclinical studies and xenografts have revealed promising anticancer activity of talquetamab. In a phase I, dose-escalation study, MCARH109, a GPRC5D-targeted CAR T-cells product, was administered at four dose levels to patients with heavily pretreated multiple myeloma, including also patients with relapse after BCMA CAR T-cells treatment. This is a second-generation CAR T-cell treatment with a human B-cell-derived GPRC5D single-chain variable fragment, a 4-1BB costimulatory domain, and a CD3ζ signaling domain (Mailankody S et al. Phase I First-in-Class Trial of MCARH109, a G Protein Coupled Receptor Class C Group 5 Member D (GPRC5D) Targeted CAR T Cell Therapy in Patients with Relapsed or Refractory Multiple Myeloma. Blood 2021; 138 (Supplement 1): 827). Another clinical trial is the CAR-GPRC5D with a hypoimmunogenic human single-chain variable fragment (ScFv) that promotes CAR-T activation, proliferation, cytokine secretion and target cell killing through the CD3ζ domain. The amplification and persistence of CAR T are enhanced by 4-1BB costimulation signal (Wang Z, Chen C, Wang L, Jia Y, Qin Y. Chimeric antigen receptor T-cell therapy for multiple myeloma. Front Immunol. 2022 Dec 22; 13: 1050522).

However, the methods of treating multiple myeloma used so far do not allow to achieve a minimized risk of antigenic escape. Mono CAR-T systems, e.g., BCMA or GPRC5D mono CAR-T despite their relatively high initial effectiveness, very often cause recurrence of the disease and the need for another therapy.

Surprisingly, the inventors of the present invention have shown that the specific selection and number of co-stimulating domains in third-generation CARs according to the present invention, namely in dual CAR-T system comprising the first and the second CAR encoded by a nucleic acid sequence having the following structure - anti-GPRC5D scFv (ET150-8) h (ICOS-4-1BB-CD3ζ) and anti-BCMA scFv (25D2) h (CD28-OX40-CD3ζ), translate into increased stability of the molecules and, consequently, more effective anticancer activity of the entire constructs associated with the increased viability and activity of the modified T cells. Thus, the approach used in the present invention allows for a better chance of a complete cure of blood and hematopoietic system cancer and reduces the risk of recurrence of the disease, while ensuring long-term efficacy.

In particular, the TNFRSF family of receptors also includes 4-1BB, CD27, CD30, DR3, GITR, and HVEM, which interact with TCR-CD3 signaling to promote cell cycle progression, cytokine production, and T cell survival. On activated T cells, the three TNF receptor monomers interact with trimerized ligands on APC to form trimeric ligand-receptor complexes that configure and assemble the cytoplasmic tails of the receptor to facilitate the recruitment of TNF receptor-associated factor (TRAF) adapter proteins. OX40 and CD27 can bind TRAF2, TRAF3 and TRAF5 (Tan, J., Jia, Y., Zhou, M. et al. Chimeric antigen receptors containing the OX40 signalling domain enhance the persistence of T cells even under repeated stimulation with multiple myeloma target cells. J Hematol Oncol 15, 39 (2022). The 4-1BB (CD137) protein encoded by TNFRSF9 is a critical co-stimulatory receptor primarily expressed by activated CD8+ T cells, and is a member of the tumor necrosis factor receptor super family, described above. Following engagement by natural ligand 4-1BBL, 4-1BB transduces the activation signal of NF-$\kappa$B, which induces preferential expansion and activation of CD8+ compared to CD4+ T cells. 4-1BB can bind TRAF1, TRAF2 and TRAF3. This receptor contributes to T cell clonal expansion, survival and development. It can also induce proliferation in peripheral monocytes, enhance T cell apoptosis induced by TCR/CD3-triggered activation, and regulate CD28 co-stimulation to promote Th1 cell response. The expression of this receptor is induced by lymphocyte activation (Cappell KM, Kochenderfer JN. A comparison of chimeric antigen receptors containing CD28 versus 4-1BB costimulatory domains. Nat Rev Clin Oncol. 2021 Nov;18(11):715-727). Although the 4-1BB/4-1BBL signaling pathway can transduce the activation signal in both CD4+ and CD8+ T cells *in vitro,* it preferentially affects the development and activation of CD8+ T cells (Dai Q, et al. 4-1BB Signaling Boosts the Anti-Tumor Activity of CD28-Incorporated 2nd Generation Chimeric Antigen Receptor-Modified T Cells. Front. Immunol. 11:539654).

Costimulation by 4-1BB and OX40 is known to promote T cell survival by upregulating anti-apoptotic factors including BCL-2, BCL-XL and BFL1 (BCL2A1). Furthermore, costimulation by 4-1BB and OX40 activates AKT to promote the cell cycle by regulating cyclins and cyclin-dependent kinases. OX40 has also been shown to upregulate TRAF6, leading to activation of non-canonical NF-$\kappa$B signaling and induction of IL-9 production in CD4+ T cells Mascarelli DE, Rosa RSM, Toscaro JM, Semionatto IF, Ruas LP, Fogagnolo CT, Lima GC and Bajgelman MC (2021) Boosting Antitumor Response by Costimulatory Strategies Driven to 4-1BB and OX40 T-cell Receptors. Front. Cell Dev. Biol. 9: 692982).

Inducible T-cell costimulator - ICOS (Inducible T-cell Costimulator) protein belongs to the CD28 surface receptor family described above. ICOS and CD28 and CTLA4 described earlier can compete for a similar binding site on B7-H2, importantly B7-H2 has been shown to co-stimulate T cells by both CD28 and ICOS, but ICOS binds to B7-H2 with much higher affinity than CD28 or CTLA4. Unlike the CD80 and CD86 ligands that interact with CD28 and CTLA-4, B7 H/ICOS-L binds only to ICOS. ICOS lacks the specific MYPPPY motif present in CD28 and CTLA-4, which has been shown to be necessary for interaction with CD80 and CD86, and B7H shares only 20% homology with B7-1 (CD80) and B7-2 (CD86). ICOS contains a unique SH2 YMFM binding motif that recruits both p85 and p50$\alpha$ PI3K subunits. p50$\alpha$ is more active than p85 therefore its recruitment by ICOS results in enhanced AKT signaling compared to CD28. ICOS plays an important role in cell signaling, immune responses and regulation of cell proliferation. It enhances the basic response of T cells to a foreign antigen, i.e. proliferation, secretion of lymphokines, overproduction of molecules mediating intercellular interactions and stimulation of antibody secretion by B cells. It is necessary for both efficient interaction between T and B cells and for the correct antibody response to antigens T-cell-dependent and further prevents apoptosis of pre-activated T cells. In addition, PI3K signaling of ICOS and consequent induction of IL-4, IL-10 and IL 21 expression are crucial for the development of CD4+ T helper 2 cells (TH2 cells) and follicular helper T cells (TFH cells).

The constructs of the present invention contain two independent coding sequences i.e., anti-BCMA and anti-GPRC5D that effectively enhance the ability of T lymphocytes to recognize multiple myeloma cells. Importantly, both constructs further contain independent and not used in that arrangement so far sequences encoding different co-stimulatory domains surprisingly improving the activity and proliferation of T lymphocytes, increasing the chance for a complete cure of blood and hematopoietic system cancer and reducing the risk of recurrence of the disease by avoiding i.a. antigenic escape, ensuring long-term effectiveness. The dual CAR-T constructs being the subject of the present invention contain BCMA/CD28/OX40 and GPRC5D/4-1BB/ICOS domains, respectively, what is an innovation with high therapeutic potential, confirmed *in vitro* (see EXMAPLES).

In one embodiment, CARs comprise a spacer sequence to connect the antigen-binding domain with the transmembrane domain and spatially separate the antigen-binding domain from the endodomain. A flexible spacer allows the antigen-binding domain to orient in different directions to facilitate binding.

Furthermore, the present invention relates to a nucleic acid sequence encoding a first CAR and a second CAR. The nucleic acid sequence may comprise one of the following sequences, or a variant thereof.

A nucleic acid sequence encoding chimeric antigen receptor (CAR) as defined herein, comprises the first CAR that has the following structure - anti-GPRC5D scFv (ET150-8) h (ICOS-4-1BB-CD3$\zeta$), and the second CAR that has the following structure - anti-BCMA scFv (25D2) h (CD28-OX40-CD3$\zeta$), wherein the first chimeric antigen receptor (CAR) is encoded by the first nucleic acid sequence as set forth in SEQ NO ID: 2 , and second CAR is encoded by second nucleic acid sequence as set forth in SEQ NO ID: 4.

In one embodiment, the first CAR may comprise a variant of said CAR-encoding part of the sequence shown as SEQ ID No. 2 having at least 80, 85, 90, 95, 98 or 99% sequence identity, provided that the variant sequence is a CAR having the required properties.

In other embodiment, the second CAR may comprise a variant of said CAR-encoding part of the sequence shown as SEQ ID No. 4 having at least 80, 85, 90, 95, 98 or 99% sequence identity, provided that the variant sequence is a CAR having the required properties.

Methods of sequence alignment are well known in the art and are accomplished using suitable alignment programs. The % sequence identity refers to the percentage of amino acid or nucleotide residues that are identical in the two sequences when they are optimally aligned. Nucleotide and protein sequence homology or identity may be determined using standard algorithms such as a BLAST program.

The CARs of the T cell of the present invention may comprise a signal peptide so that when the CAR is expressed inside a cell, such as a T-cell, the nascent protein is directed to the endoplasmic reticulum and subsequently to the cell surface, where it is expressed. The core of the signal peptide may contain a long stretch of hydrophobic amino acids that has a tendency to form a single alpha-helix. The signal peptide may begin with a short positively charged stretch of amino acids, which helps to enforce proper topology of the polypeptide during translocation. At the end of the signal peptide there is typically a stretch of amino acids that is recognized and cleaved by signal peptidase. Signal peptidase may cleave either during or after completion of translocation to generate a free signal peptide and a mature protein. The free signal peptides are then digested by specific proteases. In one embodiment, the signal peptide may be at the amino terminus of the molecule. Moreover, the signal peptide for the first CAR may have a different sequence from the signal peptide of the second CAR.

Signal peptide coding sequences for the first and second CARs are disclosed herein. The first signal peptide coding sequence is a part of the sequence set forth in SEQ NO ID: 2, and second signal peptide coding sequence is a part of the sequence set forth in set forth in SEQ NO ID: 4, respectively.

In one embodiment, the first signal peptide coding sequence may comprise a variant of said signal peptide-encoding part of the sequence having at least 80, 85, 90, 95, 98 or 99% sequence identity, provided that the variant sequence is a CAR having the required properties.

In other embodiment, the second signal peptide coding sequence may comprise a variant of said signal peptide-encoding part of the sequence having at least 80, 85, 90, 95, 98 or 99% sequence identity, provided that the variant sequence is a CAR having the required properties.

The present invention provides a vector comprising a nucleic acid encoding the first and second CAR, or kit of vectors which comprises a first vector which comprises the first nucleic acid sequence and a second vector which comprises the second nucleic acid sequence. Such a vector may be used to introduce the nucleic acid sequence(s) into a host cell so that it expresses the first and second CARs. The vector may, for example, be a plasmid or a viral vector, such as a retroviral vector or a lentiviral vector, or a transposon based vector or synthetic mRNA.

Moreover, the present invention relates to immunological cell, namely T cell or NK cell, which co-expresses a first CAR and a second CAR at the cell surface. T cells or T lymphocytes are a type of lymphocyte that play a central role in cell-mediated immunity. They can be distinguished from other lymphocytes, such as B cells and natural killer cells (NK cells), by the presence of a T-cell receptor (TCR) on the cell surface.

In certain embodiments, the CAR cells of the invention may be any of the cell types mentioned above. CAR-expressing cells, such as CAR-expressing T or NK cells, may either be created ex vivo either from a patient's own peripheral blood, or in the setting of a hematopoietic stem cell transplant from donor peripheral blood, or peripheral blood from an unconnected donor.

In certain embodiments, the present invention also provides a cell composition comprising CAR expressing T cells and/or CAR expressing NK cells according to the present invention. The cell composition may be made by transducing or transfecting a blood-sample *ex vivo* with a nucleic acid according to the present invention. Alternatively, CAR-expressing cells may be derived from ex vivo differentiation of inducible progenitor cells or embryonic progenitor cells to the relevant cell type, such as T cells. Alternatively, an immortalized cell line such as a T-cell line which retains its lytic function and could act as a therapeutic may be used.

In one embodiment, a CAR T cell of the invention may be an *ex vivo* T cell from a subject. The T cell may be isolated from a peripheral blood mononuclear cell (PBMC) sample. T cells may be activated and/or expanded prior to being transduced with CAR-encoding nucleic acid, for example by treatment with the colloidal, polymeric nanomatrix, conjugated to recombinant humanized CD3 and CD28 agonists. In other embodiment, T cells may be activated by interleukins. In one embodiment, in the presence of IL-7 and IL-15, CAR-T cells surprisingly possess a naive/$T_{SCM}$ (memory stem T cell) phenotype with improved proliferation upon antigenic-rechallenge compared to IL-2-treated CARs. Obtaining an increased proliferative potential of the cells is extremely important in the approach of generating a T cells which co-expresses at least the first chimeric antigen receptor (CAR) and second CAR at the cell surface according to the invention.

A CAR T cell of the invention may be generated by isolation of a T cell-containing sample from a subject or other sources listed above; and transduction of the T cells with one or more nucleic acid sequences encoding the first and second CAR. Then, after expansion, cells are immunophenotyped and sorted, for example, selected on the basis of co-expression of the first and second CAR.

The present invention also includes manufacturing protocol for both parts i.e., production of infectious virus after being packaged with the vectors described above and preparation of dual CAR-T product.

The present invention also relates to a pharmaceutical composition containing a plurality of CAR-expressing cells according to the first aspect of the invention. The pharmaceutical composition may additionally comprise a pharmaceutically acceptable carrier, diluent or excipient. The pharmaceutical composition may optionally comprise one or more further pharmaceutically active polypeptides and/or compounds. Such a formulation may, for example, be in a form suitable for intravenous infusion.

Furthermore, the T cells of the present invention may be capable of killing target cells, such as cancer cells (multiple myeloma). The target cell is recognisable by a defined pattern of antigen expression.

Treatment with the T cells of the invention may help prevent the escape or release of tumour cells which often occurs with standard approaches.

The invention will now be further described by way of Examples, which are meant to serve to assist one of ordinary skill in the art in carrying out the invention and are not intended in any way to limit the scope of the invention.

## EXAMPLES

## EXAMPLE 1

## GENERATION OF ANTI-BCMA AND ANTI-GPRC5D VECTORS

## CELL LINES CULTURE

### CD4+, CD8+ cells culture condition

For the study, commercially purchased CD4+ and CD8+ T cell lines were used. The immunophenotype and handling specification of each line was determined and additionally confirmed *in vitro.* The particular conditions for the growth of lymphocytes were established, taking into account the specified culture medium and the selection of appropriate interleukins. The multi-parameter flow cytometry technique was used to determine the immunophenotype of cells.

Both cell lines (CD4+ T cells and CD8+ T cells) were purchased from ATCC (American Type Culture Collection). Cells were isolated by negative selection with a purity class over 90% (CD4+ catalog number: PCS-800-016; CD8+ catalog number: PCS-800-017). The doubling time of CD4+ and CD8+ lymphocyte cells was estimated for about 40 hours, however it depended on cell activation and interleukin stimulation. The diameter of the cells is approximately 7 $\mu$m - 10 $\mu$m (the size of the cells changes depending on their current metabolic state, cells were significantly smaller before activation).

To recreate the physiological conditions in the experiments, CD4+ and CD8+ T cells were co-cultured in a 1:1 ratio at the beginning of culture. Two types of culture media were tested RPMI-1640 (ATCC cat. number 30-2001) enriched with 10% FBS (fetal bovine serum) and TexMACS (Miltenyi Biotec cat. number: 130-097-196). The effect of specific interleukins on cell proliferation and their immunophenotype was studied (for results see Table 1):

- RPMI-1640 medium (with 10% FBS) enriched with interleukin 2 (IL-2) with a concentration of 50 UI/ml = 50 ng/ml
- RPMI-1640 medium (with 10% FBS) enriched with interleukin 7 (IL-7) and interleukin 15 (IL-15) with a concentration of 155 UI/ml = 3,1 ng/ml (for IL-7) and 290 UI/ml = 58 ng/ml (for IL-15)
- TexMACS medium enriched with interleukin 2 (IL-2) with a concentration of 50 UI/ml = 50 ng/ml
- TexMACS medium enriched with interleukin 7 (IL-7) and interleukin 15 (IL-15) with a concentration of 155 UI/ml = 3,1 ng/ml (for I1-7) and 290 UI/ml = 58 ng/ml (for IL-15).

All used cytokines were purchased from Miltenyi Biotec (IL-2 cat. number: 130-097-742; IL-7 cat. number: 130-093-937; IL-15 cat. number: 130-093-955). Unexpectedly, in the presence of IL-7 and IL-15, CAR-T cells possess a naive/$T_{SCM}$ (memory stem T cell) phenotype with improved proliferation upon antigenic-rechallenge (see Table 1) compared to IL-2-treated CARs (Front. Immunol., 20 February 2019 Sec. Cancer Immunity and Immunotherapy, Volume 10-2019).

During the experiment, the presence of CD3+, CD4+, CD8+ antigens on the cell surface was assayed with the flow cytometry. In addition, the expression of the CD62L antigen on the surface of cells was monitored to indicate the cells naive immunophenotype. BD FACS Melody 9 color cell sorter was used for the analyses. The performed analyzes showed the best cell growth for cell culture with TexMACS medium enriched with interleukin 7 (IL-7) and interleukin 15 (IL-15) with a concentration of 155 UI/ml = 3,1 ng/ml (for IL-7) and 290 UI/ml = 58 ng/ml (for IL-15) without serum supplementation. Experimental determination of the IL-7 and IL15 interleukins concentration allowed to obtain non-obvious data resulting in, compared to protocols known from the state of the art (e. g. with a concentration of 5 ng/ml IL-7, IL-15 treatment (Cieri N, et al), additional induction of increased cell proliferation compared to the use of interleukin 2 (Blood 2013; 121 (4): 573-584). For example, other known protocol suggests supplementation medium with IL-7 (10 ng/ml) plus IL-15 (10 ng/ml) or IL-2 (200 U/ml) (Zhou J, Jin L, Wang F, Zhang Y, Liu B, Zhao T. Chimeric antigen receptor T (CAR-T) cells expanded with IL-7/IL-15 mediate superior antitumor effects. Protein Cell. 2019 Oct; 10 (10): 764-769 PMID: 31250350; PMCID: PMC6776495). In another, CAR T cells were generated with IL-2 (100 U/ml) and IL-7 (200 pg/ml) or IL-7 and IL-15 (10 ng/ml). Additionally, it is a common knowledge that concentration 50 IU/ml of IL-2 significantly increase the expansion rate of T cells in comparison to 20 IU/ml and there is no difference between cells cultivated in 500 IU/ml compared to 50, 100 or 200 IU/ml (Ghaffari, S., Torabi-Rahvar, M., Aghayan, S. et al. Optimizing interleukin-2 concentration, seeding density and bead-to-cell ratio of T-cell expansion for adoptive immunotherapy. BMC Immunol 22, 43 (2021)). Moreover, it was proved that cells cultured in the presence of increasing concentrations of hIL-15 (while maintaining hIL-7 at 10 ng/ml) achieved significant increases in fold expansion in the presence of 50 ng/ml or 100 ng/ml hIL-15 (Lanitis E, Rota G, Kosti P, Ronet C, Spill A, Seijo B, Romero P, Dangaj D, Coukos G, Irving M. Optimized gene engineering of murine CAR-T cells reveals the beneficial effects of IL-15 coexpression. J Exp Med. 2021 Feb 1; 218 (2): e20192203 PMID: 33156338; PMCID: PMC7653685).

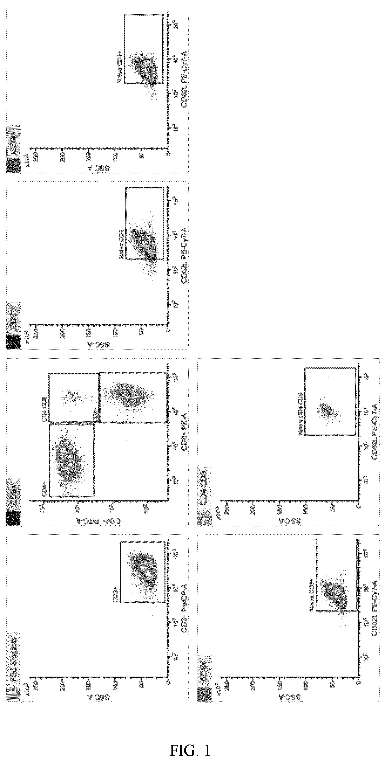

What is important, non-specific population of CD8+ T cells expressing the CD4+ antigen (double positive population) cultured with RPMI-1640 medium supplemented with IL-2 was observed. Additionally, the presence of CD62L antigen on the cell surface was observed in all tested samples, which indicates their naive immunophenotype (Fig. 1). Cell viability was determined always using 0.4 % sterile filtered Trypan Blue (PAN Biotech, Cat. No. P08-34100) in 1:1 concentration on a DeNovix Cell Drop instrument.7

Table 1. Results of immunophenotyping of cells grown in different culture media with addition of interleukins.

| Experiment conditions | | | | |
|---|---|---|---|---|
| Tested antygen/ cell viability | RPMI-1640 + IL-2 | RPMI-1640 + IL-7, IL-15 | TexMACS + IL-2 | TexMACS + IL-7, IL-15 |
| CD4+ | 29.7% | 59% | 64.23% | 59.24% |
| CD8+ | 40.59% | 39.69% | 33.63% | 38.25% |
| CD4+, CD8+ (double positive population) | 26.51% | 0.81% | 1.61% | 1.95% |
| CD62L+ | 99.5% | 98.74% | 97.68% | 97.44% |
| Cell Viability | 89.9% | 92.2% | 88.65% | 89.6% |

Lymphocytes were cultured in vessels intended for suspension cell culture. The cells were passaged every other day where the cell density after the passage is $1 \times 10^6$ cells/ml. Details on the exact handling of the cells are described in the protocols below.

**Principles of handling lymphocyte cells**

**I. Thawing cells:**

Cryo tubes containing 1 ml of cells were taken from a liquid nitrogen dewar and placed in a water bath (37°C, approx. 1-2 min). Then they were combined with 9 ml of culture medium previously heated to 37°C. The cells were centrifuged at 300 RCF, 5 min, the supernatant was discarded and the pellet was resuspended in 1 ml of medium. 20 μl of cell suspension was taken for cell counting in triplicate with 20 μl Trypan Blue. Cells were counted using a DeNovix Cell Drop. Depending on the amount, the cells were cultured in an appropriately sized suspension cell culture bottle at a density of $1 \times 10^6$ cells/ml of culture medium. Cells were cultured in a 5% $CO_2$ incubator on a cell shaker at an orbital speed of 80 RPM.

**II. T cell culture supplements**

- IL-7 concentration: 155 U/ml = 3.1 ng/ml
- IL-15 concentration: 290 U/ml = 58 ng/ml

**III. Medium exchange protocol**

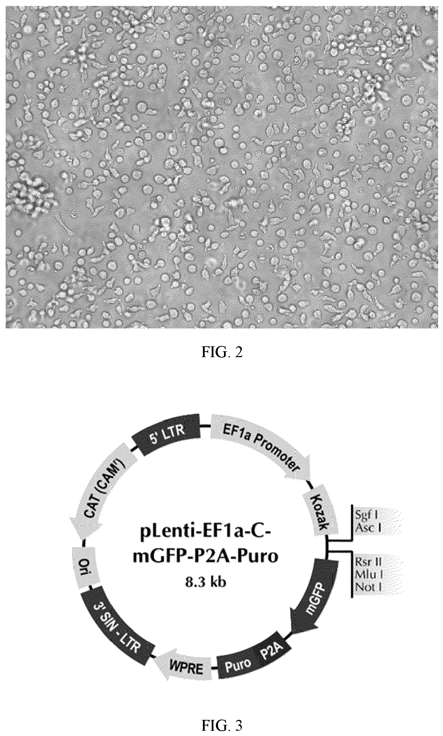

Every 2 days, T cells were thoroughly mixed and evaluated with the FLoid™ Cell Imaging Station (Fig. 2). Cells were counted using a DeNovix instrument (procedure described above). Depending on the results obtained, the volume of fresh medium to be used was recalculated to maintain a ratio of $1 \times 10^6$ cells/ml. The T cell culture was centrifuged at 300 RCF for 5 min. The supernatant was carefully removed. The appropriate amount of medium and interleukins in the proportions given above were added and mixed gently. The T cell culture was placed in an incubator with a cell shaker, speed 80 RPM.

## IV. Cell freezing protocol

The number of cells was measured using the DeNovix device (described above). The T cell culture was centrifuged at 300 RCF for 5 min. The supernatant was carefully removed. The appropriate amount of FreezeMe One freezing medium, 1 ml per $3 \times 10^6$ - 10 x $10^6$ cells, was added to the cells and gently mixed with the cells. The cell suspension was distributed 1 ml into labeled cryotubes: cell line, date of freezing, number of frozen cells, viability, no. transitions. The cryotubes were transferred to a freezing container filled with isopropanol and placed at - 80°C for at least 5 hours. After this time, the cells were transferred to the appropriate racks in a Dewar dish with liquid nitrogen (-196°C).

## V. Labeling cells with antibodies for immunophenotyping

1. $1 \times 10^6$ cells were centrifuged into a polystyrene cytometric tube (5 ml round bottom tube), at 300 RCF, 5 min.
3. The supernatant was discarded and cells were washed with PBS.
4. In a separate tube the antibodies were prepared and mixed well.
5. Pellet was resuspended in 100 µl PBS.
6. The prepared antibodies were added to the cells (Table 2), mixed thoroughly and incubated for 30 min RT in the dark.
7. Cells were washed twice with PBS.
8. Pellet was resuspended in 300 µl BD Stain Buffer (cat. number: 554656).

Table 2. Multicolored antibodies panel used for cytometric immunophenotyping assay.

| Antibody | Fluorochrome | Concentration | Cat. No. | Meaning |
|---|---|---|---|---|
| CD3 | APC-H7 | 100 µg/ml | 51-9007098 | T lymphocyte antigen |
| CD4 | PerCp-Cy5.5 | 100 µg/ml | 51-9007099 | Helper T lymphocyte antigen |
| CD8 | Negative population from CD4 | | | Cytotoxic T lymphocytes |
| CD25 | PE | 200 µg/ml | 567214 | Activation of T cells (IL-2 receptor) |
| CD69 | BV786 | 400 µg/ml | 563834 | Early activation of T cells |
| CD45RA | V450 | 400 µg/ml | 560362 | Determining the level of cell differentiation (Effector, Naive, Central Memory, Effector Memory) |
| CD62L | PE-Cy7 | 200 µg/ml | 565535 | |
| BCMA (protein) | APC | 0.5 µg/ml | BCA-HA2H4 | Determination of BCMA CAR-T expression level |
| PD-1 | RealBlue780 (Pe-Cy7) | 100 µg/ml | 568703 | Determination of exhausted T cells |
| CD45 | FITC | 25 µg/ml | 555482 | Leukocyte antigen |
| CD38 | V450 | 200 µg/ml | 561378 | Multiple myeloma antigen |

## 293T cells culture condition

The commercially available 293T cell line (ECACC: European Collection of Cell Cultures, cat. no. 12022001) was selected for efficient viral transfection. This is a line obtained by transfecting a subline of adenovirus-immortalized human embryonic kidney cells with the gene encoding the SV40-T antigen and the neomycin resistance gene. The cell line is competent to replicate the SV40 origin of replication vectors. It exhibited an adherent growth pattern and morphologies of small, slightly rounded, diffusely growing cells. The cell doubling time was 12 to 20 hours. The average cell size was 11 to

14 $\mu$m. Cells required passage at 70-80% confluence, which was approximately every 48 hours. The optimal seeding density was $4 \times 10^5$ cells/cm$^2$. Cells grown on culture plates or bottles TC Flask T25, T75, T175 Standard, Vent Cap.

Cells were cultured in Dulbecco's Modified Eagle Medium (DMEM) High Glucose (4.5 g/l), w/o L-Glutamine, with Sodium Pyruvate (Capricorn Scientific, catalog number: DMEM-HPXA) with Heat-Inactivated European Grade Foetal Bovine Serum 0.1 $\mu$m Membrane Filtered, Mycoplasma Tested, Virus Screened (BI Biological Industries, catalog number: 04-127-1A) and 2 mmol/l L-Glutamine, 200 mM (Capricorn Scientific, catalog number: GLN-B). For passage, the cells were rinsed with PBS without magnesium and calcium (EURX, catalog number: E0282-02) and then detached from the culture vessel using Accutatase Cell Detachment Solution (Capricorn Scientific, catalog number: ACC-1B) according to the manufacturer's instructions.

To freeze 293T cells in liquid nitrogen the FreezeMe One, Cyropreservation Medium with Fetal Bovine Serum (Capricorn Scientific, catalog number: FM1-F) were used and the cells were frozen at a concentration of 3,5 x 10$^6$, 5 x 10$^6$ and 10 x 10$^6$/ml.

Viability and cell amount were determined using the Cell Drop device (DeNovix, described above).

## LENTIVIRUS PRODUCTION AND TITRATION

### Lentivirus packaging, virus concentration and titer determination

The present invention also includes the nucleotide sequences of two vectors encoding an antigen binding receptor proteins expressed on the surface of the genetically engineered T cells with two different pairs of costimulatory domains. The vector used in the present invention is a lentiviral plasmid vector anti-GPRC5D scFv (ET150-8) h (ICOS-4-1BB-CD3$\zeta$) CART - the nucleotide sequence of generated vector is set forth as SEQ ID NO: 1, and anti-BCMA scFv (25D2) h (CD28-OX40-CD3$\zeta$) CART - the nucleotide sequence of generated vector is set forth as SEQ ID NO: 3.

Surprisingly, the approach described herein, as co-transduction with usage of said two lentiviral vectors, has an effect on the antitumor activity of the modified cells in *in vitro* tests with commercially available multiple myeloma cells in cell co-cultures. Moreover, said dual CAR-T system indicates more effective activity in relation to the mono CAR-T system *in vitro* (see EXAMPLE 12).

### *Lentiviral Packaging Kits (OriGene Technologies, catalog number: TR30037)*

A packaging system was used to package third-generation anti-GPRC5D scFv (ET150-8) h (ICOS-4-1BB-CD3$\zeta$) CART and Anti-BCMA scFv (25D2) h (CD28-OX40-CD3$\zeta$) CART lentivectors into high-titer virus particles. The expression of the lentiviral construct was carried out in mammalian 293T cells. The procedure was modified for high packing efficiency (see Table 3). The produced lentivirus integrated into the host genome, allowing stable cell selection. To increase the safety of the lentivirus, the components necessary for the production of the virus were divided into:

- The lentiviral transfer plasmid encoding the CAR-T inserts. The transgene sequence is flanked by long terminal repeat (LTR) sequences that facilitate integration of the transfer plasmid sequence into the host genome.
- Packing plasmids
- Envelope plasmid

The third generation packaging system is split into two plasmids: one encoding Rev and another encoding Gag and Pol. In this system, Tat is eliminated by adding the chimeric 5' LTR used for gene expression linked to a heterologous promoter on the transfer plasmid. Transgene expression from this promoter is not dependent on Tat transactivation. The chemical composition of the transfection reagent and the physical properties of the transfection complexes are critical for successful gene transfer. Uptake by the transfected cell is affected by the ratio of the charges carried by the transfection complex, defined as the arithmetic ratio of the positively charged moieties of the transfection reagent to the negative charges of the DNA. Modifying the charge ratio is critical when improving a transfection protocol and involves determining the volume of transfection reagent per microgram of DNA, and the selected reagent to DNA ratio often depends on the cell type. Serum contains nucleases and other components that may interfere with complex formation, use only serum-free media. Similarly, antibiotics (and other highly charged molecules) can disrupt the charge balance, thereby inhibiting complex formation. Once nucleic acids are complexed with transfection reagents, they are protected from the activity of nucleases and charged species.

Lipofectamine 3000 Transfection Reagent (Invitrogen, catalog number: L3000001) is based on cationic lipids, suitable for the transfection of nucleic acids into eukaryotic cells. The use of Lipofectamine 3000 to transfect 293T cells ensures the highest transfection efficiency in 293T cells, DNA-Lipofectamine 3000 complexes can be added directly to cells in culture medium in the presence of serum, removal of complexes or change of medium or addition after transfection are not required, although complexes can be removed after 4-6 hours without loss of activity.

Modification of the transfection process was performed using a control lentiviral plasmid encoding GFP pLenti-EF1a-C-mGFP-P2A-Puro Lentiviral Gene Expression Vector (OriGene, PS100121) (Fig. 3) with Lenti-vpak Lentiviral Packaging Kit (OriGene, TR30037) with the Lipofectamine 3000™ Invitrogen to determine transfection efficiency and toxicity (the negative control: no DNA, no Lipofectamine 3000 reagent was included). The 293T cell lipofection protocol has been refined to obtain the most efficient transfection procedure, therefore said modified transfection protocol is another aspect according to the present invention (see Table 3).

Lentiviral particles were generated using designed and synthesized anti-GPRC5D scFv (ET150-8) h (ICOS-4-1BB-CD3ζ) CART, pCDCAR1 and anti-BCMA scFv (25D2) h (CD28-OX40-CD3ζ) CART, pCDCAR1 vectors transfected into 293T cells using Lenti-vpak Packaging Kit and transfection reagents according to the modified Lipofectamine3000 transfection protocol from Invitrogen.

**CAR CONSTRUCTION - LENTIVIRAL PLASMID MAPS**

**Lentiviral transfer vector, anti-GPRC5D scFv (ET150-8) h (ICOS-4-1BB-CD3ζ) CART, pCDCAR1**

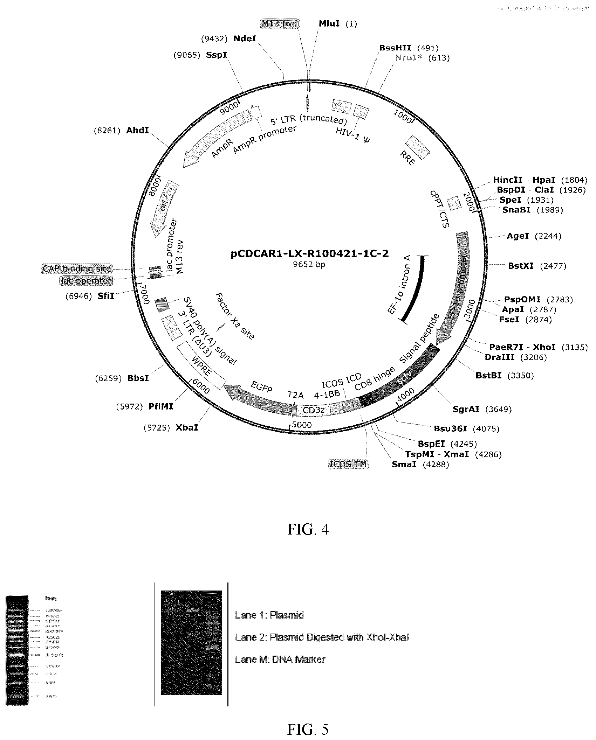

The used viral vector is lentiviral transfer vector, anti-GPRC5D scFv (ET150-8) h (ICOS-4-1BB-CD3ζ) CART, pCDCAR1 with cloning sties BstBI (TTCGAA)-XbaI(TCTAGA) and target GPRC5D (for plasmid map see Fig. 4). The above construct contains a promoter EF1a. CAR construction was EFla promoter- scFv-CD8 hinge-ICOS ICS-4-1BB-CD3ζ with EGFP marker. Product size was 10ug/vial. The electrophoretic analysis for said plasmid was shown in Fig. 5.

Full sequence of plasmid pCDCAR1-Customized-Anti-GPRC5D scFv (ET150-8) CD8 hinge ICOS TM (ICOS-4-1BB-CD3ζ) CAR, pCDCAR1(SEQ NO ID: 1) is provided below:

ACGCGTGTAGTCTTATGCAATACTCTTGTAGTCTTGCAACATGGTAACGATGAGTT

AGCAACATGCCTTACAAGGAGAGAAAAAGCACCGTGCATGCCGATTGGTGGAAG

TAAGGTGGTACGATCGTGCCTTATTAGGAAGGCAACAGACGGGTCTGACATGGAT

TGGACGAACCACTGAATTGCCGCATTGCAGAGATATTGTATTTAAGTGCCTAGCT

CGATACAATAAACGGGTCTCTCTGGTTAGACCAGATCTGAGCCTGGGAGCTCTCT

GGCTAACTAGGGAACCCACTGCTTAAGCCTCAATAAAGCTTGCCTTGAGTGCTTC

AAGTAGTGTGTGCCCGTCTGTTGTGTGACTCTGGTAACTAGAGATCCCTCAGACC
CTTTTAGTCAGTGTGGAAAATCTCTAGCAGTGGCGCCCGAACAGGGACTTGAAAG
CGAAAGGGAAACCAGAGGAGCTCTCTCGACGCAGGACTCGGCTTGCTGAAGCGC
GCACGGCAAGAGGCGAGGGGCGGCGACTGGTGAGTACGCCAAAAATTTTGACTA
GCGGAGGCTAGAAGGAGAGAGATGGGTGCGAGAGCGTCAGTATTAAGCGGGGG
AGAATTAGATCGCGATGGGAAAAAATTCGGTTAAGGCCAGGGGGAAAGAAAAA
ATATAAATTAAAACATATAGTATGGGCAAGCAGGGAGCTAGAACGATTCGCAGT
TAATCCTGGCCTGTTAGAAACATCAGAAGGCTGTAGACAAATACTGGGACAGCTA
CAACCATCCCTTCAGACAGGATCAGAAGAACTTAGATCATTATATAATACAGTAG
CAACCCTCTATTGTGTGCATCAAAGGATAGAGATAAAAGACACCAAGGAAGCTTT
AGACAAGATAGAGGAAGAGCAAAACAAAAGTAAGACCACCGCACAGCAAGCGG
CCACTGATCTTCAGACCTGGAGGAGGAGATATGAGGGACAATTGGAGAAGTGAA
TTATATAAATATAAAGTAGTAAAAATTGAACCATTAGGAGTAGCACCCACCAAG
GCAAAGAGAAGAGTGGTGCAGAGAGAAAAAAGAGCAGTGGGAATAGGAGCTTT
GTTCCTTGGGTTCTTGGGAGCAGCAGGAAGCACTATGGGCGCAGCGTCAATGACG
CTGACGGTACAGGCCAGACAATTATTGTCTGGTATAGTGCAGCAGCAGAACAATT
TGCTGAGGGCTATTGAGGCGCAACAGCATCTGTTGCAACTCACAGTCTGGGGCAT
CAAGCAGCTCCAGGCAAGAATCCTGGCTGTGGAAAGATACCTAAAGGATCAACA
GCTCCTGGGGATTTGGGGTTGCTCTGGAAAACTCATTTGCACCACTGCTGTGCCTT
GGAATGCTAGTTGGAGTAATAAATCTCTGGAACAGATTTGGAATCACACGACCTG
GATGGAGTGGGACAGAGAAATTAACAATTACACAAGCTTAATACACTCCTTAATT
GAAGAATCGCAAAACCAGCAAGAAAGAATGAACAAGAATTATTGGAATTAGAT
AAATGGGCAAGTTTGTGGAATTGGTTTAACATAACAAATTGGCTGTGGTATATAA
AATTATTCATAATGATAGTAGGAGGCTTGGTAGGTTTAAGAATAGTTTTTGCTGT
ACTTTCTATAGTGAATAGAGTTAGGCAGGGATATTCACCATTATCGTTTCAGACC
CACCTCCCAACCCCGAGGGGACCCGACAGGCCCGAAGGAATAGAAGAAGAAGGT
GGAGAGAGAGACAGAGACAGATCCATTCGATTAGTGAACGGATCTCGACGGTAT
CGGTTAACTTTTAAAAGAAAGGGGGGATTGGGGGGTACAGTGCAGGGGAAAGA
ATAGTAGACATAATAGCAACAGACATACAAACTAAAGAATTACAAAAACAAATT
ACAAAATTCAAAATTTTATCGATACTAGTATTATGCCCAGTACATGACCTTATGG
GACTTTCCTACTTGGCAGTACATCTACGTATTAGTCATCGCTATTACCATGgagtaattc
atacaaaaggactcgcccctgccttggggaatcccagggaccgtcgttaaactcccactaacgtagaacccagagatcgctgcgttcc
cgccccctcacccgcccgctctcgtcatcactgaggtggagaagagcatgcgtgaggctccggtgcccgtcagtgggcagagcgca
catcgcccacagtccccgagaagttggggggaggggtcggcaattgaaccggtgcctagagaaggtggcgcggggtaaactggga

aagtgatgtcgtgtactggctccgccttttttcccgagggtgggggagaaccgtatataagtgcagtagtcgccgtgaacgttcttttttcgc

aacgggtttgccgccagaacacaggtaagtgccgtgtgtggttcccgcgggcctggcctctttacgggttatggcccttgcgtgccttg

aattacttccacgcccctggctgcagtacgtgattcttgatcccgagcttcgggttggaagtgggtgggagagttcgaggccttgcgctt

aaggagccccttcgcctcgtgcttgagttgaggcctggcttgggcgctggggccgccgcgtgcgaatctggtggcaccttcgcgcctg

tctcgctgctttcgataagtctctagccatttaaaattttttgatgacctgctgcgacgcttttttttctggcaagatagtcttgtaaatgcgggcc

aagatctgcacactggtatttcggttttttggggccgcgggcggcgacggggcccgtgcgtcccagcgcacatgttcggcgaggcggg

gcctgcgagcgcggccaccgagaatcggacggggggtagtctcaagctggccggcctgctctggtgcctggcctcgcgccgccgtgt

atcgccccgccctgggcggcaaggctggcccggtcggcaccagttgcgtgagcggaaagatggccgcttcccggccctgctgcag

ggagctcaaaatggaggacgcggcgctcgggagagcgggcgggtgagtcacccacacaaaggaaaagggcctttccgtcctcag

ccgtcgcttcatgtgactccacggagtaccgggcgccgtccaggcacctcgattagttctcgagcttttggagtacgtcgtctttaggttg

ggggaggggtttatgcgatggagtttccccacactgagtgggtggagactgaagttaggccagcttggcacttgatgtaattctcctt

ggaatttgcccttttgagtttggatcttggttcattctcaagcctcagacagtggttcaaagttttttttcttccatttcaggtgtcgtgaTTC

GAAGCCGCCACCATGGCCTTACCAGTGACCGCCTTGCTCCTGCCGCTGGCCTTGC

TGCTCCACGCCGCCAGGCCGAGCTCCGAGCTGACCCAGGATCCCGCCGTGAGCGT

GGCCCTGGGACAGACAGTGAGGATCACCTGTCAGGGCGACTCCCTGAGATCCTAC

TACGCCTCCTGGTACCAGCAGAAGCCCGGCCAGGCCCCCGTGCTGGTTATCTACG

GCAAGAATAATAGGCCTAGCGGCATCCCTGATAGATTTTCCGGCAGCTCCAGCGG

CAATACCGCCAGCCTGACCATCACCGGCGCCCAGGCTGAGGACGAGGCCGATTA

CTACTGCAATTCCAGGGACAGCAGCGGCAATCCTCCTGTGGTGTTTGGCGGCGGC

ACCAAGCTGACAGTGCTGGGCAGCAGGGGCGGCGGAGGATCTGGAGGAGGCGG

ATCCGGCGGCGGAGGTTCTCTGGAGATGGCCCAGGTGCAGCTGGTGGAGAGCGG

CGGAGGCCTGGTGCACCCAGGAGGATCCCTGAGACTGAGCTGTGCCGCCTCCGGC

TTCACCTTTAGGTCCCACAGCATGAATTGGGTGAGACAGGCCCCTGGCAAGGGCC

TGGAGTGGGTGTCCAGCATCAGCAGCGACAGCACCTACACCTACTACGCCGATAG

CGTGAAGGGCAGGTTCACCATCTCCAGAGACAACGCCAAGAATAGCCTGTACCT

GCAGATGAACAGCCTGAGGGCCGAGGATACCGCCGTGTACTACTGCGCCAGAAG

CGGCGGCCAGTGGAAGTACTACGACTACTGGGGCCAGGGCACACTGGTGACAGT

GAGCTCCACCACTACCCCAGCACCGAGGCCACCCACCCCGGCTCCTACCATCGCC

TCCCAGCCTCTGTCCCTGCGTCCGGAGGCATGTAGACCCGCAGCTGGTGGGGCCG

TGCATACCCGGGGTCTTGACTTCGCCTGCGATTTCTGGCTGCCCATCGGCTGCGCC

GCCTTCGTGGTGGTGTGTATCCTGGGCTGTATCCTGATCTGTTGGCTGACAAAGA

AGAAGTACTCCAGCTCCGTGCACGATCCCAATGGCGAGTACATGTTTATGAGAGC

CGTGAATACCGCCAAGAAGAGCAGACTGACCGACGTGACCCTGAAGCGCGGTCG

GAAGAAGCTGCTGTACATCTTTAAGCAACCCTTCATGAGGCCTGTGCAGACTACT

CAAGAGGAGGACGGCTGTTCATGCCGGTTCCCAGAGGAGGAGGAAGGCGGCTGC
GAACTGCGCGTGAAATTCAGCCGCAGCGCAGATGCTCCAGCCTACAAGCAGGGG
CAGAACCAGCTCTACAACGAACTCAATCTTGGTCGGAGAGAGGAGTACGACGTG
CTGGACAAGCGGAGAGGACGGGACCCAGAAATGGGCGGGAAGCCGCGCAGAAA
GAATCCCCAAGAGGGCCTGTACAACGAGCTCCAAAAGGATAAGATGGCAGAAGC
CTATAGCGAGATTGGTATGAAAGGGGAACGCAGAAGAGGCAAAGGCCACGACG
GACTGTACCAGGGACTCAGCACCGCCACCAAGGACACCTATGACGCTCTTCACAT
GCAGGCCCTGCCGCCTCGGGAGGGCAGAGGCAGCCTGCTGACATGTGGCGACGT
GGAAGAGAACCCTGGCCCCATGGTGAGCAAGGGCGAGGAGCTGTTCACCGGGGT
GGTGCCCATCCTGGTCGAGCTGGACGGCGACGTAAACGGCCACAAGTTCAGCGT
GTCCGGCGAGGGCGAGGGCGATGCCACCTACGGCAAGCTGACCCTGAAGTTCAT
CTGCACCACCGGCAAGCTGCCCGTGCCCTGGCCCACCCTCGTGACCACCCTGACC
TACGGCGTGCAGTGCTTCAGCCGCTACCCCGACCACATGAAGCAGCACGACTTCT
TCAAGTCCGCCATGCCCGAAGGCTACGTCCAGGAGCGCACCATCTTCTTCAAGGA
CGACGGCAACTACAAGACCCGCGCCGAGGTGAAGTTCGAGGGCGACACCCTGGT
GAACCGCATCGAGCTGAAGGGCATCGACTTCAAGGAGGACGGCAACATCCTGGG
GCACAAGCTGGAGTACAACTACAACAGCCACAACGTCTATATCATGGCCGACAA
GCAGAAGAACGGCATCAAGGTGAACTTCAAGATCCGCCACAACATCGAGGACGG
CAGCGTGCAGCTCGCCGACCACTACCAGCAGAACACCCCCATCGGCGACGGCCC
CGTGCTGCTGCCCGACAACCACTACCTGAGCACCCAGTCCGCCCTGAGCAAAGAC
CCCAACGAGAAGCGCGATCACATGGTCCTGCTGGAGTTCGTGACCGCCGCCGGG
ATCACTCTCGGCATGGACGAGCTGTACAAGTAATCTAGAAATCAACCTCTGGATT
ACAAAATTTGTGAAAGATTGACTGGTATTCTTAACTATGTTGCTCCTTTTACGCTA
TGTGGATACGCTGCTTTAATGCCTTTGTATCATGCTATTGCTTCCCGTATGGCTTTC
ATTTTCTCCTCCTTGTATAAATCCTGGTTGCTGTCTCTTTATGAGGAGTTGTGGCCC
GTTGTCAGGCAACGTGGCGTGGTGTGCACTGTGTTTGCTGACGCAACCCCCACTG
GTTGGGGCATTGCCACCACCTGTCAGCTCCTTTCCGGGACTTTCGCTTTCCCCCTC
CCTATTGCCACGGCGGAACTCATCGCCGCCTGCCTTGCCCGCTGCTGGACAGGGG
CTCGGCTGTTGGGCACTGACAATTCCGTGGTGTTGTCGGGGAAATCATCGTCCTTT
CCTTGGCTGCTCGCCTGTGTTGCCACCTGGATTCTGCGCGGGACGTCCTTCTGCTA
CGTCCCTTCGGCCCTCAATCCAGCGGACCTTCCTTCCCGCGGCCTGCTGCCGGCTC
TGCGGCCTCTTCCGCGTCTTCGCCTTCGCCCTCAGACGAGTCGGATCTCCCTTTGG
GCCGCCTCCCCGCCTGGTACCTTTAAGACCAATGACTTACAAGGCAGCTGTAGAT
CTTAGCCACTTTTTAAAAGAAAAGGGGGGACTGGAAGGGCTAATTCACTCCCAAC

GAAGATAAGATCTGCTTTTTGCTTGTACTGGGTCTCTCTGGTTAGACCAGATCTGA

GCCTGGGAGCTCTCTGGCTAACTAGGGAACCCACTGCTTAAGCCTCAATAAAGCT

TGCCTTGAGTGCTTCAAGTAGTGTGTGCCCGTCTGTTGTGTGACTCTGGTAACTAG

AGATCCCTCAGACCCTTTTAGTCAGTGTGGAAAATCTCTAGCAGTAGTAGTTCAT

GTCATCTTATTATTCAGTATTTATAACTTGCAAAGAAATGAATATCAGAGAGTGA

GAGGAACTTGTTTATTGCAGCTTATAATGGTTACAAATAAAGCAATAGCATCACA

AATTTCACAAATAAAGCATTTTTTTCACTGCATTCTAGTTGTGGTTTGTCCAAACT

CATCAATGTATCTTATCATGTCTGGCTCTAGCTATCCCGCCCCTAACTCCGCCCAT

CCCGCCCCTAACTCCGCCCAGTTCCGCCCATTCTCCGCCCCATGGCTGACTAATTT

TTTTTATTTATGCAGAGGCCGAGGCCGCCTCGGCCTCTGAGCTATTCCAGAAGTA

GTGAGGAGGCTTTTTGGAGGCCTAGACTTTGCAGAGACCAAATTCGTAATCAT

GTCATAGCTGTTTCCTGTGTGAAATTGTTATCCGCTCACAATTCCACACAACATAC

GAGCCGGAAGCATAAAGTGTAAAGCCTGGGGTGCCTAATGAGTGAGCTAACTCA

CATTAATTGCGTTGCGCTCACTGCCCGCTTTCCAGTCGGGAAACCTGTCGTGCCAG

CTGCATTAATGAATCGGCCAACGCGCGGGGAGAGGCGGTTTGCGTATTGGGCGCT

CTTCCGCTTCCTCGCTCACTGACTCGCTGCGCTCGGTCGTTCGGCTGCGGCGAGCG

GTATCAGCTCACTCAAAGGCGGTAATACGGTTATCCACAGAATCAGGGGATAAC

GCAGGAAAGAACATGTGAGCAAAAGGCCAGCAAAAGGCCAGGAACCGTAAAAA

GGCCGCGTTGCTGGCGTTTTTCCATAGGCTCCGCCCCCCTGACGAGCATCACAAA

AATCGACGCTCAAGTCAGAGGTGGCGAAACCCGACAGGACTATAAAGATACCAG

GCGTTTCCCCCTGGAAGCTCCCTCGTGCGCTCTCCTGTTCCGACCCTGCCGCTTAC

CGGATACCTGTCCGCCTTTCTCCCTTCGGGAAGCGTGGCGCTTTCTCATAGCTCAC

GCTGTAGGTATCTCAGTTCGGTGTAGGTCGTTCGCTCCAAGCTGGGCTGTGTGCA

CGAACCCCCCGTTCAGCCCGACCGCTGCGCCTTATCCGGTAACTATCGTCTTGAGT

CCAACCCGGTAAGACACGACTTATCGCCACTGGCAGCAGCCACTGGTAACAGGA

TTAGCAGAGCGAGGTATGTAGGCGGTGCTACAGAGTTCTTGAAGTGGTGGCCTAA

CTACGGCTACACTAGAAGAACAGTATTTGGTATCTGCGCTCTGCTGAAGCCAGTT

ACCTTCGGAAAAAGAGTTGGTAGCTCTTGATCCGGCAAACAAACCACCGCTGGTA

GCGGTGGTTTTTTTGTTTGCAAGCAGCAGATTACGCGCAGAAAAAAGGATCTCA

AGAAGATCCTTTGATCTTTTCTACGGGGTCTGACGCTCAGTGGAACGAAAACTCA

CGTTAAGGGATTTTGGTCATGAGATTATCAAAAGGATCTTCACCTAGATCCTTTT

AAATTAAAAATGAAGTTTTAAATCAATCTAAAGTATATATGAGTAAACTTGGTCT

GACAGTTACCAATGCTTAATCAGTGAGGCACCTATCTCAGCGATCTGTCTATTTCG

TTCATCCATAGTTGCCTGACTCCCCGTCGTGTAGATAACTACGATACGGGAGGGC

TTACCATCTGGCCCCAGTGCTGCAATGATACCGCGAGACCCACGCTCACCGGCTC

CAGATTTATCAGCAATAAACCAGCCAGCCGGAAGGGCCGAGCGCAGAAGTGGTC

CTGCAACTTTATCCGCCTCCATCCAGTCTATTAATTGTTGCCGGGAAGCTAGAGTA

AGTAGTTCGCCAGTTAATAGTTTGCGCAACGTTGTTGCCATTGCTACAGGCATCGT

GGTGTCACGCTCGTCGTTTGGTATGGCTTCATTCAGCTCCGGTTCCCAACGATCAA

GGCGAGTTACATGATCCCCATGTTGTGCAAAAAGCGGTTAGCTCCTTCGGTCC

TCCGATCGTTGTCAGAAGTAAGTTGGCCGCAGTGTTATCACTCATGGTTATGGCA

GCACTGCATAATTCTCTTACTGTCATGCCATCCGTAAGATGCTTTTCTGTGACTGG

TGAGTACTCAACCAAGTCATTCTGAGAATAGTGTATGCGGCGACCGAGTTGCTCT

TGCCCGGCGTCAATACGGGATAATACCGCGCCACATAGCAGAACTTTAAAAGTGC

TCATCATTGGAAAACGTTCTTCGGGGCGAAAACTCTCAAGGATCTTACCGCTGTT

GAGATCCAGTTCGATGTAACCCACTCGTGCACCCAACTGATCTTCAGCATCTTTTA

CTTTCACCAGCGTTTCTGGGTGAGCAAAAACAGGAAGGCAAAATGCCGCAAAAA

AGGGAATAAGGGCGACACGGAAATGTTGAATACTCATACTCTTCCTTTTTCAATA

TTATTGAAGCATTTATCAGGGTTATTGTCTCATGAGCGGATACATATTTGAATGTA

TTTAGAAAAATAAACAAATAGGGGTTCCGCGCACATTTCCCCGAAAAGTGCCACC

TGACGTCTAAGAAACCATTATTATCATGACATTAACCTATAAAAATAGGCGTATC

ACGAGGCCCTTTCGTCTCGCGCGTTTCGGTGATGACGGTGAAAACCTCTGACACA

TGCAGCTCCCGGAGACGGTCACAGCTTGTCTGTAAGCGGATGCCGGGAGCAGAC

AAGCCCGTCAGGGCGCGTCAGCGGGTGTTGGCGGGTGTCGGGGCTGGCTTAACTA

TGCGGCATCAGAGCAGATTGTACTGAGAGTGCACCATATGCGGTGTGAAATACCG

CACAGATGCGTAAGGAGAAAATACCGCATCAGGCGCCATTCGCCATTCAGGCTG

CGCAACTGTTGGGAAGGGCGATCGGTGCGGGCCTCTTCGCTATTACGCCAGCTGG

CGAAAGGGGGATGTGCTGCAAGGCGATTAAGTTGGGTAACGCCAGGGTTTTCCC

AGTCACGACGTTGTAAAACGACGGCCAGTGCCAAGCTG

Signal peptide, scfv Anty-GPRC5D, CD8 hinge, ICOS TM, ICOS ICD, 4-1BB, CD3ζ, T2A (SEQ NO ID: 2) coding sequence is provided below:

TTCGAAGCCGCCACCATGGCCTTACCAGTGACCGCCTTGCTCCTGCCGCTGGCCTT

GCTGCTCCACGCCGCCAGGCCGAGCTCCGAGCTGACCCAGGATCCCGCCGTGAGC

GTGGCCCTGGGACAGACAGTGAGGATCACCTGTCAGGGCGACTCCCTGAGATCCT

ACTACGCCTCCTGGTACCAGCAGAAGCCCGGCCAGGCCCCCGTGCTGGTTATCTA

CGGCAAGAATAATAGGCCTAGCGGCATCCCTGATAGATTTTCCGGCAGCTCCAGC

GGCAATACCGCCAGCCTGACCATCACCGGCGCCCAGGCTGAGGACGAGGCCGAT

TACTACTGCAATTCCAGGGACAGCAGCGGCAATCCTCCTGTGGTGTTTGGCGGCG

GCACCAAGCTGACAGTGCTGGGCAGCAGGGGCGGCGGAGGATCTGGAGGAGGCG

GATCCGGCGGCGGAGGTTCTCTGGAGATGGCCCAGGTGCAGCTGGTGGAGAGCG

GCGGAGGCCTGGTGCACCCAGGAGGATCCCTGAGACTGAGCTGTGCCGCCTCCG

GCTTCACCTTTAGGTCCCACAGCATGAATTGGGTGAGACAGGCCCCTGGCAAGGG

CCTGGAGTGGGTGTCCAGCATCAGCAGCGACAGCACCTACACCTACTACGCCGAT

AGCGTGAAGGGCAGGTTCACCATCTCCAGAGACAACGCCAAGAATAGCCTGTAC

CTGCAGATGAACAGCCTGAGGGCCGAGGATACCGCCGTGTACTACTGCGCCAGA

AGCGGCGGCCAGTGGAAGTACTACGACTACTGGGGCCAGGGCACACTGGTGACA

GTGAGCTCCACCACTACCCCAGCACCGAGGCCACCCACCCCGGCTCCTACCATCG

CCTCCCAGCCTCTGTCCCTGCGTCCGGAGGCATGTAGACCCGCAGCTGGTGGGGC

CGTGCATACCCGGGGTCTTGACTTCGCCTGCGATTTCTGGCTGCCCATCGGCTGCG

CCGCCTTCGTGGTGGTGTGTATCCTGGGCTGTATCCTGATCTGTTGGCTGACAAAG

AAGAAGTACTCCAGCTCCGTGCACGATCCCAATGGCGAGTACATGTTTATGAGAG

CCGTGAATACCGCCAAGAAGAGCAGACTGACCGACGTGACCCTGAAGCGCGGTC

GGAAGAAGCTGCTGTACATCTTTAAGCAACCCTTCATGAGGCCTGTGCAGACTAC

TCAAGAGGAGGACGGCTGTTCATGCCGGTTCCCAGAGGAGGAGGAAGGCGGCTG

CGAACTGCGCGTGAAATTCAGCCGCAGCGCAGATGCTCCAGCCTACAAGCAGGG

GCAGAACCAGCTCTACAACGAACTCAATCTTGGTCGGAGAGAGGAGTACGACGT

GCTGGACAAGCGGAGAGGACGGGACCCAGAAATGGGCGGGAAGCCGCGCAGAA

AGAATCCCCAAGAGGGCCTGTACAACGAGCTCCAAAAGGATAAGATGGCAGAAG

CCTATAGCGAGATTGGTATGAAAGGGGAACGCAGAAGAGGCAAAGGCCACGACG

GACTGTACCAGGGACTCAGCACCGCCACCAAGGACACCTATGACGCTCTTCACAT

GCAGGCCCTGCCGCCTCGGGAGGGCAGAGGCAGCCTGCTGACATGTGGCGACGT

GGAAGAGAACCCTGGCCCC

**Lentiviral transfer vector, anti-BCMA scFv (25D2) h (CD28-OX40-CD3ζ) CART, pCDCAR1**

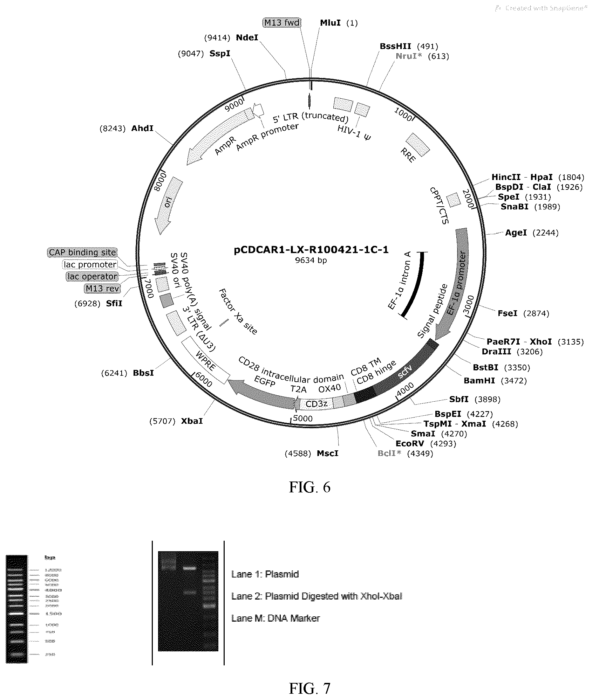

The used viral vector is lentiviral transfer vector, anti-BCMA scFv (25D2) h (CD28-OX40-CD3ζ) CART, pCDCAR1 with cloning sties BstBI(TTCGAA)-XbaI(TCTAGA) and target BCMA (for plasmid map see Fig. 6). The above construct contains a promoter EF1a. CAR construction was EFla promoter- scFv - CD8 hinge - CD8 TM - CD28 ICD - OX40 - CD3ζ with EGFP marker. Product size was 10 μg/vial. The electrophoretic analysis for said plasmid was shown in Fig. 7.

Full sequence of plasmid pCDCARI-CAR-T-3-X1022-Anti-BCMA scFv (25D2) CD8 hinge CD8 TM (CD28-OX40-CD3ζ) CART, pCDCAR1 (SEQ ID NO: 3) is provided below:

ACGCGTGTAGTCTTATGCAATACTCTTGTAGTCTTGCAACATGGTAACGATGAGTT

AGCAACATGCCTTACAAGGAGAGAAAAGCACCGTGCATGCCGATTGGTGGAAG

TAAGGTGGTACGATCGTGCCTTATTAGGAAGGCAACAGACGGGTCTGACATGGAT

TGGACGAACCACTGAATTGCCGCATTGCAGAGATATTGTATTTAAGTGCCTAGCT

CGATACAATAAACGGGTCTCTCTGGTTAGACCAGATCTGAGCCTGGGAGCTCTCT

GGCTAACTAGGGAACCCACTGCTTAAGCCTCAATAAAGCTTGCCTTGAGTGCTTC

AAGTAGTGTGTGCCCGTCTGTTGTGTGACTCTGGTAACTAGAGATCCCTCAGACC

CTTTTAGTCAGTGTGGAAAATCTAGCAGTGGCGCCCGAACAGGGACTTGAAAG

CGAAAGGGAAACCAGAGGAGCTCTCGACGCAGGACTCGGCTTGCTGAAGCGC

GCACGGCAAGAGGCGAGGGGCGGCGACTGGTGAGTACGCCAAAAATTTTGACTA

GCGGAGGCTAGAAGGAGAGATGGGTGCGAGAGCGTCAGTATTAAGCGGGGG

AGAATTAGATCGCGATGGGAAAAAATTCGGTTAAGGCCAGGGGGAAAGAAAAA

ATATAAATTAAAACATATAGTATGGGCAAGCAGGGAGCTAGAACGATTCGCAGT

TAATCCTGGCCTGTTAGAAACATCAGAAGGCTGTAGACAAATACTGGGACAGCTA

CAACCATCCCTTCAGACAGGATCAGAAGAACTTAGATCATTATATAATACAGTAG

CAACCCTCTATTGTGTGCATCAAAGGATAGAGATAAAAGACACCAAGGAAGCTTT

AGACAAGATAGAGGAAGAGCAAAACAAAAGTAAGACCACCGCACAGCAAGCGG

CCACTGATCTTCAGACCTGGAGGAGGAGATATGAGGGACAATTGGAGAAGTGAA

TTATATAAATATAAAGTAGTAAAAATTGAACCATTAGGAGTAGCACCCACCAAG

GCAAAGAGAAGAGTGGTGCAGAGAGAAAAAAGAGCAGTGGGAATAGGAGCTTT

GTTCCTTGGGTTCTTGGGAGCAGCAGGAAGCACTATGGGCGCAGCGTCAATGACG

CTGACGGTACAGGCCAGACAATTATTGTCTGGTATAGTGCAGCAGCAGAACAATT

TGCTGAGGGCTATTGAGGCGCAACAGCATCTGTTGCAACTCACAGTCTGGGGCAT

CAAGCAGCTCCAGGCAAGAATCCTGGCTGTGGAAAGATACCTAAAGGATCAACA

GCTCCTGGGGATTTGGGGTTGCTCTGGAAAACTCATTTGCACCACTGCTGTGCCTT

GGAATGCTAGTTGGAGTAATAAATCTCTGGAACAGATTTGGAATCACACGACCTG

GATGGAGTGGGACAGAGAAATTAACAATTACACAAGCTTAATACACTCCTTAATT

GAAGAATCGCAAAACCAGCAAGAAAGAATGAACAAGAATTATTGGAATTAGAT

AAATGGGCAAGTTTGTGGAATTGGTTTAACATAACAAATTGGCTGTGGTATATAA

AATTATTCATAATGATAGTAGGAGGCTTGGTAGGTTTAAGAATAGTTTTTGCTGT

ACTTTCTATAGTGAATAGAGTTAGGCAGGGATATTCACCATTATCGTTTCAGACC

CACCTCCCAACCCCGAGGGGACCCGACAGGCCCGAAGGAATAGAAGAAGAAGGT
GGAGAGAGAGACAGAGACAGATCCATTCGATTAGTGAACGGATCTCGACGGTAT
CGGTTAACTTTTAAAAGAAAAGGGGGGATTGGGGGGTACAGTGCAGGGGAAAGA
ATAGTAGACATAATAGCAACAGACATACAAACTAAAGAATTACAAAAACAAATT
ACAAAATTCAAAATTTTATCGATACTAGTATTATGCCCAGTACATGACCTTATGG
GACTTTCCTACTTGGCAGTACATCTACGTATTAGTCATCGCTATTACCATGgagtaattc
atacaaaaggactcgcccctgccttggggaatcccagggaccgtcgttaaactcccactaacgtagaacccagagatcgctgcgttcc
cgcccctcacccgcccgctctcgtcatcactgaggtggagaagagcatgcgtgaggctccggtgcccgtcagtgggcagagcgca
catcgcccacagtccccgagaagttggggggaggggtcggcaattgaaccggtgcctagagaaggtggcgcggggtaaactggga
aagtgatgtcgtgtactggctccgccttttcccgagggtgggggagaaccgtatataagtgcagtagtcgccgtgaacgttcttttcgc
aacgggtttgccgccagaacacaggtaagtgccgtgtgtggttcccgcgggcctggcctctttacgggttatggcccttgcgtgccttg
aattacttccacgcccctggctgcagtacgtgattcttgatcccgagcttcgggttggaagtgggtgggagagttcgaggccttgcgctt
aaggagccccttcgcctcgtgcttgagttgaggcctggcttgggcgctggggccgccgcgtgcgaatctggtggcaccttcgcgcctg
tctcgctgctttcgataagtctctagccatttaaaattttgatgacctgctgcgacgcttttttctggcaagatagtcttgtaaatgcgggcc
aagatctgcacactggtatttcggttttttggggccgcgggcggcgacggggcccgtgcgtcccagcgcacatgttcggcgaggcggg
gcctgcgagcgcggccaccgagaatcggacggggggtagtctcaagctggccggcctgctctggtgcctggcctcgcgccgccgtgt
atcgccccgccctgggcggcaaggctggcccggtcggcaccagttgcgtgagcggaaagatggccgcttcccggccctgctgcag
ggagctcaaaatggaggacgcggcgctcgggagagcgggcgggtgagtcacccacacaaaggaaaagggcctttccgtcctcag
ccgtcgcttcatgtgactccacggagtaccgggcgccgtccaggcacctcgattagttctcgagcttttggagtacgtcgtctttaggttg
ggggggaggggttttatgcgatggagtttccccacactgagtgggtggagactgaagttaggccagcttggcacttgatgtaattctcctt
ggaatttgcccttttttgagtttggatcttggttcattctcaagcctcagacagtggttcaaagttttttttcttccatttcaggtgtcgtgaTTC
GAAGCCGCCACCATGGCCTTACCAGTGACCGCCTTGCTCCTGCCGCTGGCCTTGC
TGCTCCACGCCGCCAGGCCGGAGGTGCAGCTGCTGGAGTCCGGCGGCGGACTGG
TGCAGCCAGGAGGATCCCTGAGGCTGTCCTGCGCCGCCAGCGGATTCACTTTCAG
ATCCTACGCCATGAGCTGGGTGAGACAGGCCCCTGGCAAGGGCCTGGAGTGGGT
GTCCGCCATCTCCGGCGGCGGTGGAAATACATTCTACGCCGACTCCGTGAAGGGC
AGGTTCACAATCAGCAGGGACAATTCCAAGAACACCCTGTACCTGCAGATGAACT
CCCTGAGGGCCGAGGACACCGCCGTGTACTACTGCGCCAAGGTGAGGCCCTTTTG
GGGCACCTTTGATTACTGGGGCCAGGGCACCCTGGTGACAGTGTCCAGCGGCGGC
GGCGGAAGCGGAGGAGGAGGATCTGGCGGCGGCGGTAGCGAGATCGTGCTGACC
CAGTCCCCCGGCACACTGTCCCTGTCCCCCGGAGAGAGAGCCACCCTGTCCTGCA
GGGCCTCCCAGTCCGTGAGCTCCAGCTACCTGGCCTGGTACCAGCAGAAGCCCGG
CCAGGCCCCCAGGCTGCTGATCTACGGCGCCTCCTCCAGAGCCACAGGCATCCCT
GACAGGTTCAGCGGCAGCGGCTCCGGCACAGACTTTACACTGACAATCTCCAGAC

TGGAGCCTGAGGATTTCGCCGTGTACTATTGTCAGCAGTACTTCAACCCTCCTGA

GTACACATTCGGCCAGGGCACAAAGGTGGAGATCAAGAGAACCACTACCCCAGC

ACCGAGGCCACCCACCCCGGCTCCTACCATCGCCTCCCAGCCTCTGTCCCTGCGTC

CGGAGGCATGTAGACCCGCAGCTGGTGGGGCCGTGCATACCCGGGGTCTTGACTT

CGCCTGCGATATCTACATTTGGGCCCCTCTGGCTGGTACTTGCGGGGTCCTGCTGC

TTTCACTCGTGATCACTCTTTACTGTAGGAGCAAGCGGAGCAGACTGCTGCACAG

CGACTACATGAACATGACCCCCCGGAGGCCTGGCCCCACCCGGAAGCACTACCA

GCCCTACGCCCCTCCCAGGGATTTCGCCGCCTACCGGAGCAGAGACCAGAGACTG

CCCCCTGACGCCCACAAGCCCCCCGGAGGAGGAAGCTTTAGAACACCCATCCAG

GAGGAGCAGGCCGACGCCCACAGCACACTGGCCAAGATCCGCGTGAAATTCAGC

CGCAGCGCAGATGCTCCAGCCTACAAGCAGGGGCAGAACCAGCTCTACAACGAA

CTCAATCTTGGTCGGAGAGAGGAGTACGACGTGCTGGACAAGCGGAGAGGACGG

GACCCAGAAATGGGCGGGAAGCCGCGCAGAAAGAATCCCCAAGAGGGCCTGTAC

AACGAGCTCCAAAAGGATAAGATGGCAGAAGCCTATAGCGAGATTGGTATGAAA

GGGGAACGCAGAAGAGGCAAAGGCCACGACGGACTGTACCAGGGACTCAGCAC

CGCCACCAAGGACACCTATGACGCTCTTCACATGCAGGCCCTGCCGCCTCGGGAG

GGCAGAGGCAGCCTGCTGACATGTGGCGACGTGGAAGAGAACCCTGGCCCCATG

GTGAGCAAGGGCGAGGAGCTGTTCACCGGGGTGGTGCCCATCCTGGTCGAGCTG

GACGGCGACGTAAACGGCCACAAGTTCAGCGTGTCCGGCGAGGGCGAGGGCGAT

GCCACCTACGGCAAGCTGACCCTGAAGTTCATCTGCACCACCGGCAAGCTGCCCG

TGCCCTGGCCCACCCTCGTGACCACCCTGACCTACGGCGTGCAGTGCTTCAGCCG

CTACCCCGACCACATGAAGCAGCACGACTTCTTCAAGTCCGCCATGCCCGAAGGC

TACGTCCAGGAGCGCACCATCTTCTTCAAGGACGACGGCAACTACAAGACCCGCG

CCGAGGTGAAGTTCGAGGGCGACACCCTGGTGAACCGCATCGAGCTGAAGGGCA

TCGACTTCAAGGAGGACGGCAACATCCTGGGGCACAAGCTGGAGTACAACTACA

ACAGCCACAACGTCTATATCATGGCCGACAAGCAGAAGAACGGCATCAAGGTGA

ACTTCAAGATCCGCCACAACATCGAGGACGGCAGCGTGCAGCTCGCCGACCACT

ACCAGCAGAACACCCCCATCGGCGACGGCCCCGTGCTGCTGCCCGACAACCACTA

CCTGAGCACCCAGTCCGCCCTGAGCAAAGACCCCAACGAGAAGCGCGATCACAT

GGTCCTGCTGGAGTTCGTGACCGCCGCCGGGATCACTCTCGGCATGGACGAGCTG

TACAAGTAATCTAGAAATCAACCTCTGGATTACAAAATTTGTGAAAGATTGACTG

GTATTCTTAACTATGTTGCTCCTTTTACGCTATGTGGATACGCTGCTTTAATGCCTT

TGTATCATGCTATTGCTTCCCGTATGGCTTTCATTTTCTCCTCCTTGTATAAATCCT

GGTTGCTGTCTCTTTATGAGGAGTTGTGGCCCGTTGTCAGGCAACGTGGCGTGGT

GTGCACTGTGTTTGCTGACGCAACCCCCACTGGTTGGGGCATTGCCACCACCTGT
CAGCTCCTTTCCGGGACTTTCGCTTTCCCCCTCCCTATTGCCACGGCGGAACTCAT
CGCCGCCTGCCTTGCCCGCTGCTGGACAGGGGCTCGGCTGTTGGGCACTGACAAT
TCCGTGGTGTTGTCGGGGAAATCATCGTCCTTTCCTTGGCTGCTCGCCTGTGTTGC
CACCTGGATTCTGCGCGGGACGTCCTTCTGCTACGTCCCTTCGGCCCTCAATCCAG
CGGACCTTCCTTCCCGCGGCCTGCTGCCGGCTCTGCGGCCTCTTCCGCGTCTTCGC
CTTCGCCCTCAGACGAGTCGGATCTCCCTTTGGGCCGCCTCCCCGCCTGGTACCTT
TAAGACCAATGACTTACAAGGCAGCTGTAGATCTTAGCCACTTTTTAAAAGAAAA
GGGGGGACTGGAAGGGCTAATTCACTCCCAACGAAGATAAGATCTGCTTTTTGCT
TGTACTGGGTCTCTCTGGTTAGACCAGATCTGAGCCTGGGAGCTCTCTGGCTAACT
AGGGAACCCACTGCTTAAGCCTCAATAAAGCTTGCCTTGAGTGCTTCAAGTAGTG
TGTGCCCGTCTGTTGTGTGACTCTGGTAACTAGAGATCCCTCAGACCCTTTTAGTC
AGTGTGGAAAATCTCTAGCAGTAGTAGTTCATGTCATCTTATTATTCAGTATTTAT
AACTTGCAAAGAAATGAATATCAGAGAGTGAGAGGAACTTGTTTATTGCAGCTTA
TAATGGTTACAAATAAAGCAATAGCATCACAAATTTCACAAATAAAGCATTTTTT
TCACTGCATTCTAGTTGTGGTTTGTCCAAACTCATCAATGTATCTTATCATGTCTG
GCTCTAGCTATCCCGCCCCTAACTCCGCCCATCCCGCCCCTAACTCCGCCCAGTTC
CGCCCATTCTCCGCCCCATGGCTGACTAATTTTTTTTATTTATGCAGAGGCCGAGG
CCGCCTCGGCCTCTGAGCTATTCCAGAAGTAGTGAGGAGGCTTTTTTGGAGGCCT
AGACTTTTGCAGAGACCAAATTCGTAATCATGTCATAGCTGTTTCCTGTGTGAAAT
TGTTATCCGCTCACAATTCCACACAACATACGAGCCGGAAGCATAAAGTGTAAAG
CCTGGGGTGCCTAATGAGTGAGCTAACTCACATTAATTGCGTTGCGCTCACTGCC
CGCTTTCCAGTCGGGAAACCTGTCGTGCCAGCTGCATTAATGAATCGGCCAACGC
GCGGGGAGAGGCGGTTTGCGTATTGGGCGCTCTTCCGCTTCCTCGCTCACTGACT
CGCTGCGCTCGGTCGTTCGGCTGCGGCGAGCGGTATCAGCTCACTCAAAGGCGGT
AATACGGTTATCCACAGAATCAGGGGATAACGCAGGAAAGAACATGTGAGCAAA
AGGCCAGCAAAAGGCCAGGAACCGTAAAAAGGCCGCGTTGCTGGCGTTTTTCCA
TAGGCTCCGCCCCCCTGACGAGCATCACAAAAATCGACGCTCAAGTCAGAGGTG
GCGAAACCCGACAGGACTATAAAGATACCAGGCGTTTCCCCCTGGAAGCTCCCTC
GTGCGCTCTCCTGTTCCGACCCTGCCGCTTACCGGATACCTGTCCGCCTTTCTCCC
TTCGGGAAGCGTGGCGCTTTCTCATAGCTCACGCTGTAGGTATCTCAGTTCGGTGT
AGGTCGTTCGCTCCAAGCTGGGCTGTGTGCACGAACCCCCCGTTCAGCCCGACCG
CTGCGCCTTATCCGGTAACTATCGTCTTGAGTCCAACCCGGTAAGACACGACTTA
TCGCCACTGGCAGCAGCCACTGGTAACAGGATTAGCAGAGCGAGGTATGTAGGC

GGTGCTACAGAGTTCTTGAAGTGGTGGCCTAACTACGGCTACACTAGAAGAACAG TATTTGGTATCTGCGCTCTGCTGAAGCCAGTTACCTTCGGAAAAAGAGTTGGTAG CTCTTGATCCGGCAAACAAACCACCGCTGGTAGCGGTGGTTTTTTTGTTTGCAAGC AGCAGATTACGCGCAGAAAAAAGGATCTCAAGAAGATCCTTTGATCTTTTCTAC GGGGTCTGACGCTCAGTGGAACGAAAACTCACGTTAAGGGATTTTGGTCATGAGA TTATCAAAAAGGATCTTCACCTAGATCCTTTTAAATTAAAAATGAAGTTTTAAATC AATCTAAAGTATATATGAGTAAACTTGGTCTGACAGTTACCAATGCTTAATCAGT GAGGCACCTATCTCAGCGATCTGTCTATTTCGTTCATCCATAGTTGCCTGACTCCC CGTCGTGTAGATAACTACGATACGGGAGGGCTTACCATCTGGCCCCAGTGCTGCA ATGATACCGCGAGACCCACGCTCACCGGCTCCAGATTTATCAGCAATAAACCAGC CAGCCGGAAGGGCCGAGCGCAGAAGTGGTCCTGCAACTTTATCCGCCTCCATCCA GTCTATTAATTGTTGCCGGGAAGCTAGAGTAAGTAGTTCGCCAGTTAATAGTTTG CGCAACGTTGTTGCCATTGCTACAGGCATCGTGGTGTCACGCTCGTCGTTTGGTAT GGCTTCATTCAGCTCCGGTTCCCAACGATCAAGGCGAGTTACATGATCCCCCATG TTGTGCAAAAAAGCGGTTAGCTCCTTCGGTCCTCCGATCGTTGTCAGAAGTAAGT TGGCCGCAGTGTTATCACTCATGGTTATGGCAGCACTGCATAATTCTCTTACTGTC ATGCCATCCGTAAGATGCTTTTCTGTGACTGGTGAGTACTCAACCAAGTCATTCTG AGAATAGTGTATGCGGCGACCGAGTTGCTCTTGCCCGGCGTCAATACGGGATAAT ACCGCGCCACATAGCAGAACTTTAAAAGTGCTCATCATTGGAAAACGTTCTTCGG GGCGAAAACTCTCAAGGATCTTACCGCTGTTGAGATCCAGTTCGATGTAACCCAC TCGTGCACCCAACTGATCTTCAGCATCTTTTACTTTCACCAGCGTTTCTGGGTGAG CAAAAACAGGAAGGCAAAATGCCGCAAAAAAGGGAATAAGGGCGACACGGAAA TGTTGAATACTCATACTCTTCCTTTTTCAATATTATTGAAGCATTTATCAGGGTTAT TGTCTCATGAGCGGATACATATTTGAATGTATTTAGAAAAATAAACAAATAGGGG TTCCGCGCACATTTCCCCGAAAAGTGCCACCTGACGTCTAAGAAACCATTATTAT CATGACATTAACCTATAAAAATAGGCGTATCACGAGGCCCTTTCGTCTCGCGCGT TTCGGTGATGACGGTGAAAACCTCTGACACATGCAGCTCCCGGAGACGGTCACAG CTTGTCTGTAAGCGGATGCCGGGAGCAGACAAGCCCGTCAGGGCGCGTCAGCGG GTGTTGGCGGGTGTCGGGGCTGGCTTAACTATGCGGCATCAGAGCAGATTGTACT GAGAGTGCACCATATGCGGTGTGAAATACCGCACAGATGCGTAAGGAGAAAATA CCGCATCAGGCGCCATTCGCCATTCAGGCTGCGCAACTGTTGGGAAGGGCGATCG GTGCGGGCCTCTTCGCTATTACGCCAGCTGGCGAAAGGGGGATGTGCTGCAAGGC GATTAAGTTGGGTAACGCCAGGGTTTTCCCAGTCACGACGTTGTAAAACGACGGC CAGTGCCAAGCTG

Signal peptide, scfv Anty-BCMA, CD8 hinge, CD8 TM, CD28 ICD, OX40, CD3ζ, T2A (SEQ NO ID: 4) coding sequence is provided below:

TTCGAAGCCGCCACCATGGCCTTACCAGTGACCGCCTTGCTCCTGCCGCTGGCCTT

GCTGCTCCACGCCGCCAGGCCGGAGGTGCAGCTGCTGGAGTCCGGCGGCGGACT

GGTGCAGCCAGGAGGATCCCTGAGGCTGTCCTGCGCCGCCAGCGGATTCACTTTC

AGATCCTACGCCATGAGCTGGGTGAGACAGGCCCCTGGCAAGGGCCTGGAGTGG

GTGTCCGCCATCTCCGGCGGCGGTGGAAATACATTCTACGCCGACTCCGTGAAGG

GCAGGTTCACAATCAGCAGGGACAATTCCAAGAACACCCTGTACCTGCAGATGA

ACTCCCTGAGGGCCGAGGACACCGCCGTGTACTACTGCGCCAAGGTGAGGCCCTT

TTGGGGCACCTTTGATTACTGGGGCCAGGGCACCCTGGTGACAGTGTCCAGCGGC

GGCGGCGGAAGCGGAGGAGGAGGATCTGGCGGCGGCGGTAGCGAGATCGTGCTG

ACCCAGTCCCCCGGCACACTGTCCCTGTCCCCCGGAGAGAGAGCCACCCTGTCCT

GCAGGGCCTCCCAGTCCGTGAGCTCCAGCTACCTGGCCTGGTACCAGCAGAAGCC

CGGCCAGGCCCCCAGGCTGCTGATCTACGGCGCCTCCTCCAGAGCCACAGGCATC

CCTGACAGGTTCAGCGGCAGCGGCTCCGGCACAGACTTTACACTGACAATCTCCA

GACTGGAGCCTGAGGATTTCGCCGTGTACTATTGTCAGCAGTACTTCAACCCTCCT

GAGTACACATTCGGCCAGGGCACAAAGGTGGAGATCAAGAGAACCACTACCCCA

GCACCGAGGCCACCCACCCCGGCTCCTACCATCGCCTCCCAGCCTCTGTCCCTGC

GTCCGGAGGCATGTAGACCCGCAGCTGGTGGGGCCGTGCATACCCGGGGTCTTGA

CTTCGCCTGCGATATCTACATTTGGGCCCCTCTGGCTGGTACTTGCGGGGTCCTGC

TGCTTTCACTCGTGATCACTCTTTACTGTAGGAGCAAGCGGAGCAGACTGCTGCA

CAGCGACTACATGAACATGACCCCCCGGAGGCCTGGCCCCACCCGGAAGCACTA

CCAGCCCTACGCCCCTCCCAGGGATTTCGCCGCCTACCGGAGCAGAGACCAGAGA

CTGCCCCCTGACGCCCACAAGCCCCCCGGAGGAGGAAGCTTTAGAACACCCATCC

AGGAGGAGCAGGCCGACGCCCACAGCACACTGGCCAAGATCCGCGTGAAATTCA

GCCGCAGCGCAGATGCTCCAGCCTACAAGCAGGGGCAGAACCAGCTCTACAACG

AACTCAATCTTGGTCGGAGAGAGGAGTACGACGTGCTGGACAAGCGGAGAGGAC

GGGACCCAGAAATGGGCGGGAAGCCGCGCAGAAGAATCCCCAAGAGGGCCTGT

ACAACGAGCTCCAAAAGGATAAGATGGCAGAAGCCTATAGCGAGATTGGTATGA

AAGGGGAACGCAGAAGAGGCAAAGGCCACGACGGACTGTACCAGGGACTCAGC

ACCGCCACCAAGGACACCTATGACGCTCTTCACATGCAGGCCCTGCCGCCTCGGG

AGGGCAGAGGCAGCCTGCTGACATGTGGCGACGTGGAAGAGAACCCTGGCCCC

**293T CELL TRANSFECTION PROTOCOL**

For efficient transfection, 293T cultured to the 4th to 8th generation cells must reached 90% confluency. The most efficient ratio of Lipofectamine 3000 to DNA concentration was determined experimentally and amounts to 3-3.14 : 1, therefore said modified ratio is another aspect according to the present invention. This protocol was intended for transfection performed on a 75 $cm^2$ culture bottle with an optimized seeding density of $0.5 \times 10^4$ - $1 \times 10^5$ cells/$cm^2$. Two test tubes were prepared:

1. Tube A: to 1984 $\mu$l Opti-MEM I Reduced Serum Medium, GlutaMAX Supplement (Gibco, catalog number: 51985034) 71.4 $\mu$l Lipofectamine 3000 was added - vortex.
2. Tube B: to 2005.8 $\mu$l Opti-MEM I Reduced Serum Medium, GlutaMAX Supplement, 34 $\mu$l Packaging plasmids [0.5 $\mu$g/$\mu$l], 46.3 $\mu$l P3000 and 6.7 $\mu$l Anti-BCMA plasmid [0.85 $\mu$g/$\mu$l] or Anti-GPRC5D plasmid [0.85 $\mu$g/$\mu$l] were added.
3. Tube A (with lipofectamine) (2055.4 $\mu$l) were added to tube B (with plasmid DNA) (2092.8 $\mu$l).
4. Incubate 30 minutes at room temperature in the dark.
5. During this time the half amount (~ 5 ml) of culture medium was removed from the culture bottles.
6. 4148.2 $\mu$l of the mixture was added to the bottle.
7. **After 8 hours, the transfection medium was removed and fresh complete culture medium was added (10 ml) - the time with transfection mix was estimated experimentally and the most efficient was 8 - 10 hours, not longer. 12 hours caused more cytotoxic effect on cells.**

After 18 hours from transfection the first part of supernatant was collected and fresh DMEM + 10% FBS medium (10 ml) was added. The second part of supernatant was collected 48 hours after transfection. The collected supernatants were mixed together and filter with a 0.45 $\mu$m filter.

Table 3. Comparison of 293T transfection results.

| Lipofectamine to DNA ratio | % singlets | % live cells (PI) | % GFP+ live cells | % dead cells (PI +) | % GFP+ dead cells |
|---|---|---|---|---|---|
| Lipofect.: DNA 3.14:1 $1x10^5$ cells/$cm^2$ | 97.92% | 94.65% | **57.98 %** | 5.09% | 75.31% |
| Lipofect.: DNA 2.3:1 $1\times10^5$ cells/$cm^2$ | 98.1% | 94.92% | **44.82 %** | 4.79% | 63.55% |
| Lipofect.: DNA 3.14:1 $7.5x10^4$ cells/$cm^2$ | 97.47% | 94.13% | **58.9 %** | 5.63% | 81.7% |
| Lipofect.: DNA 2.3:1 $7.5x10^4$ cells/$cm^2$ | 97.66% | 93.26% | **45.58%** | 6.54% | 68.88% |
| Lipofect.: DNA 3.14:1 $5x10^4$/$cm^2$ | 97.64% | 92.51% | **58.88 %** | 7.15% | 88.74% |
| Lipofect.: DNA 2.3:1 $5x10^4$/$cm^2$ | 97.56% | 92.29% | **47.01%** | 7.48% | 73.13% |
| Control cells $5x10^4$/$cm^2$ | 96.05% | 99.4% | **0%** | 0.56% | 0% |
| Control Lipofectamine $5x10^4$/$cm^2$ | 97.07% | 98.99% | **0.06%** | 0.96% | 0.78% |
| Control cells $7.5\times10^4$/$cm^2$ | 98.14% | 97.25% | **0%** | 2.5% | 0% |
| Control Lipofectamine $7.5x10^4$/$cm^2$ | 96.56% | 99.09% | **0%** | 0.87% | 0% |
| Control cells $1x10^5$/$cm^2$ | 98.4% | 97.85% | **0%** | 1.96% | 0% |

(continued)

| Lipofectamine to DNA ratio | % singlets | % live cells (PI) | % GFP+ live cells | % dead cells (PI +) | % GFP+ dead cells |
|---|---|---|---|---|---|
| Control Lipofectamine $1 \times 10^5/cm^2$ | 98.06% | 97.25% | 0% | 2.52% | 0% |

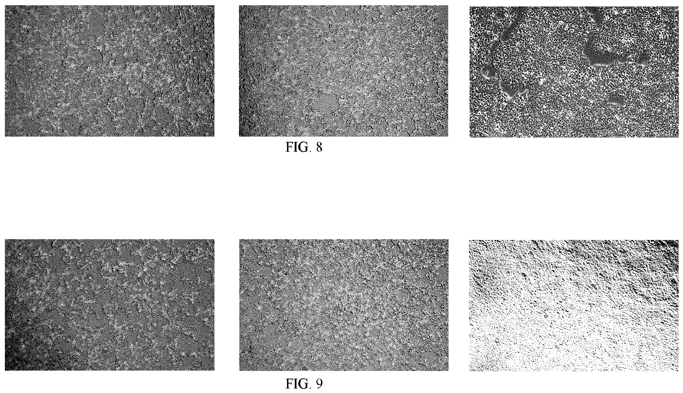

Transfection of packaging cells of the 293T line with an expression plasmid containing a recombinant gene encoding the Anti-BCMA and Anti-GPRC5D chimeric antigen receptor (Fig. 8-9), according to the table below:

| A. | Opti-MEM medium | 1984 μl |
|---|---|---|
|  | Lipofectamine 3000 | 71.4 μl |
| B. | Opti-MEM medium | 1984 μl |
|  | Packaging plasmids | 34 μl |
|  | Anti-BCMA or Anti-GPRC5D plasmid | 28.5 μl |
|  | P3000 | 46.3 μl |

## LENTIVIRAL TITER DETERMINATION

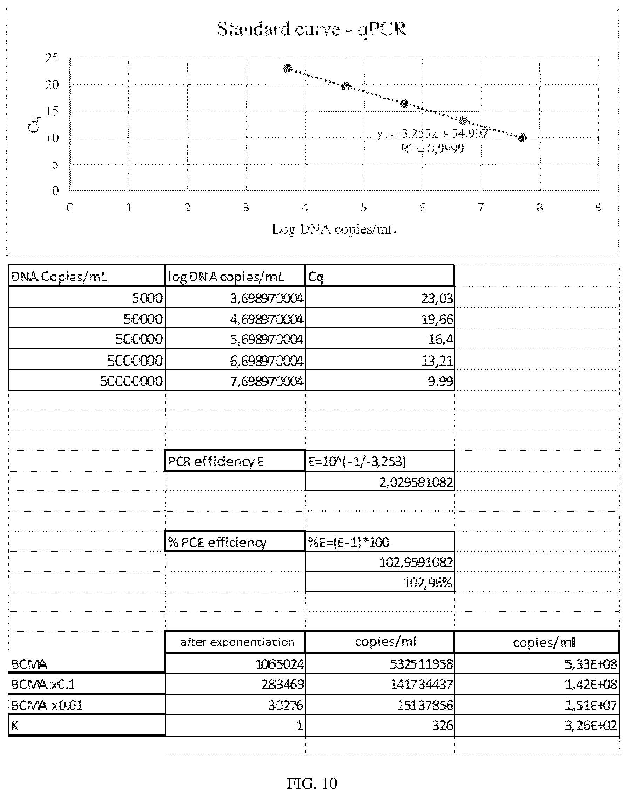

Post transfection, the produced viral particles were harvested from the cell supernatant (obtained as described in section "293T CELL TRANSFECTION PROTOCOL"). The purified cell supernatant was transferred to sterile falcon tube and 1 volume of Lenti-X Concentrator was added to 3 volumes of the purified filtered lentiviral supernatant. Since lentiviruses are sensitive to freeze-thawing and the titer drops with repeated freeze-thawing, the viral stock is aliquoted and stored at - 80°C for long-term usage. The sample from a particular transfection process of lentivirus particles production was thawed and the viral titer was assessed. The total titer of the entire viral mixture from one transfection procedure is determined. Material isolation was performed using the GeneMATRIX Viral RNA/DNA Purification Kit (EURX, cat. no. E3592-02). 100 μl of viral supernatant concentrated with **Lenti-X Concentrator** (Takara Bio, cat. no.: 631231) supplemented with NaCl to 200 μl was used for isolation. Virus titer was determined by qRT-PCR using the Lenti-X™ qRT-PCR Titration kit (Takara Bio, cat. no. 631235). The number of copies of lentiviral vectors was determined using the standard curve and the formula proposed by the manufacturer. Results of the obtained lentivirus anti-BCMA titer in the concentrated medium collected from the transfected 293T cells are presented on Fig. 10. Namely, lentivirus anti-BCMA titer is 5.33 x $10^8$/ml, which was experimentally selected for further procedure. Examples of lentivirus anti-BCMA titers after different transfection procedures: 1.42 x $10^8$/ml, 3.26 x $10^8$/ml, 5.33 x $10^8$/ml, 6.79 x $10^8$/ml, 4.16 x $10^8$/ml.

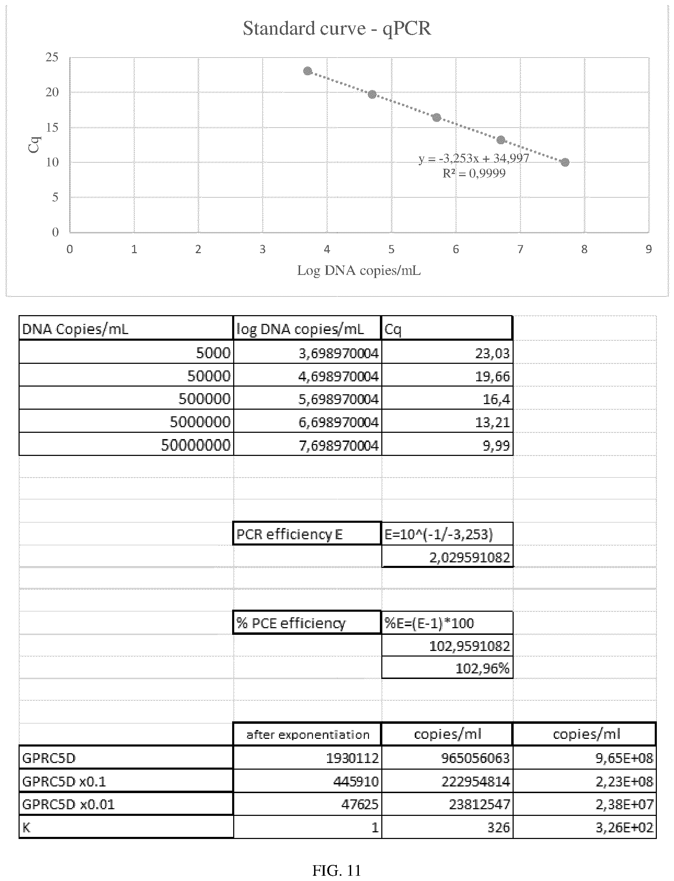

Results of the obtained lentivirus anti-GPRC5D titer in the concentrated medium collected from the transfected 293T cells are presented on Fig.11. Namely, lentivirus anti-GPRC5D titer is 9.65 x $10^8$/ml, which was experimentally selected for further procedure. Examples of lentivirus anti-GPRC5D titers after different transfection procedures: 3.68 x $10^8$/ml, 5.36 x $10^8$/ml, 8.16 x $10^8$/ml, 8.33 x $10^8$/ml, 9.65 x $10^8$/ml.

MOI (multiplicity of infection) is the ratio of the number of viral particles used to infect cells to the actual number of cells. Lentiviral particle titers are reported in transduction units (TU) per ml. 1 TU = 1 infectious particle, so if $10^6$ TUs are used to infect $10^6$ cells, then the MOI = 1. To determine the most effective MOI for a given viral vector, serial dilutions of viral vectors in the range of MOI 1 - MOI 150 were required using co-culture of CD4+, CD8+ lymphocytic cell lines.

To determine efficient transduction, on day 0, cells (co-culture of CD4+, CD8+ lymphocytic cell lines) were plated at $1 \times 10^6$ cells/ml TexMACS medium and activated with T Cell TransAct at 10 μl/ml medium. On the first day, cells were transduced with various MOI variants (1-200) with the viral vectors to be tested and Vectofusin-1 (Miltenyi Biotec, cat. no. 130-111-163) reagent (10 μl/ml). It has been found that transduction shows better efficiency when spinoculation is used for transduction. For this purpose, cells (co-culture of CD4+, CD8+ lymphocytic cell lines) with viral vectors (obtained anti-GPRC5D and anti-BCMA viral vector) and a transduction enhancer were centrifuged for 1 hour at 32°C at 400 RCF. The cells were then placed in an incubator at 37°C, 5% $CO_2$ for 18 hours. After this time, the medium was changed to TexMACS culture medium supplemented with interleukin 7 (IL-7) and interleukin 15 (IL-15) at a concentration of 155 UI/ml (for IL-7) and 290 UI/ml (for IL-15). In the following days, the medium was changed every 48 hours, and additionally, on days 9 and 11, their immunophenotype and GFP fluorescence were checked by flow cytometry. The culture was carried out for 14 days, after 14 days the immunophenotype and fluorescence level were checked again. From these results, the most effective MOI for the anti-BCMA viral vector of 100 and the MOI for the anti-GPRC5D viral vector of 75 were determined. When two vectors were used simultaneously, the MOI of 50 for the anti-BCMA vector and the MOI of 50 for the anti-GPRC5D vector were the most efficient.

The available literature suggests using an MOI in the range of 1-20 depending on the cell type and viral vector to obtain efficient and stable transduction with no significant differences in transduction efficiency after stimulation with IL-2, IL-7, or IL-15 (Simona Cavalieri, Sabrina Cazzaniga, Massimo Geuna, Zulma Magnani, Claudio Bordignon, Luigi Naldini, Chiara Bonini; Human T lymphocytes transduced by lentiviral vectors in the absence of TCR activation maintain an intact immune competence. Blood 2003; 102 (2): 497-505). Importantly, different methods of transduction spinoculation or overnight incubation, different viral vectors under the control of the phosphoglycerate kinase promoter or a synthetic promoter that contains the U3 region of a modified Moloney murine leukemic virus (MoMuLV) long terminal repeat (LTR) or different culture media used after transduction resulted in different efficiency and percentage of transduced cells (Lo Presti V, Cornel AM, Plantinga M, Dünnebach E, Kuball J, Boelens JJ, Nierkens S, van Til NP. Efficient lentiviral transduction method to gene modify cord blood CD8+ T cells for cancer therapy applications. Mol Ther Methods Clin Dev. 2021 Mar 23;21:357-368 PMID: 33898633; PMCID: PMC8056177).

However, in the case of viral vectors according to the present invention, where EF-1 alpha (Human elongation factor-1 alpha) promoters allow to achieve robust, constitutive, long-term expression of genes of interest, obtained results unexpectedly showed that the use of much higher MOI values (50-100) led to achieve surprisingly high transduction efficiency. What is important, significantly higher MOI parameters than those recommended in the literature for T cells transduction were used. The usage of MOI 75, 100 for anti-GPRC5D lentiviruses and MOI 100, 125 for anti-BCMA lentiviruses compared to the MOI 5 or 10 know from the art (https://www.ncbi.nlm.nih.gov/pmc/articles/PMC6895454/) significantly increased the efficiency of T lymphocyte transduction, which was manifested by a high percentage of GFP+ cells in relation to control cells. Importantly and surprisingly, a significant increase in the MOI parameter and obtaining a much higher transduction efficiency and percentage of GFP positive cells did not negatively affect the viability of the modified cells. The viability and functionality of the engineered cells was similar to the untreated control cells. The dual CARs, vectors and used lentiviruses system of the present invention ensured surprisingly high efficiency although it is widely believed that high MOI negatively affects the survival and functionality of the modified cells (https://journals.aai.org/jimmunol/article/195/5/2493/105012/Exclusive-Transduction-of-Human-CD4-T-Cells-upon).

Table 4 Results of modifications of T lymphocyte transduction with BCMA virus.

| BCMA transduction efficiency after 14 days of starting culture | GFP positive population (%) |
| --- | --- |
| MOI 5 | 2.33% |
| MOI 10 | 8.18% |
| MOI 100 | 68.23% |
| MOI 125 | 79.25% |
| MOI 150 | 68.8% |

Table 5 Results of modifications of T lymphocyte transduction with GPRC5D virus.

| GPRC5D transduction efficiency after 14 days of starting culture | GFP positive population (%) |
| --- | --- |
| MOI 5 | 1.29% |
| MOI 10 | 6.32% |
| MOI 50 | 12.24% |
| MOI 75 | 16.9% |
| MOI 100 | 18.04% |

Table 6 Results of modifications of T lymphocyte transduction with GFP virus.

| GFP transduction efficiency after 14 days of starting culture | GFP positive population (%) |
| --- | --- |
| MOI 5 | 0.83% |
| MOI 10 | 3.47% |
| MOI 20 | 2.56% |
| MOI 40 | 1.76% |
| MOI 60 | 1.6% |

(continued)

| GFP transduction efficiency after 14 days of starting culture | GFP positive population (%) |
|---|---|
| MOI 150 | 15.77% |
| MOI 175 | 10.07% |
| MOI 200 | 16.4% |

Table 7 Results of modifications of T lymphocyte transduction with both BCMA and GPRC5D virus.

| Dual CAR-T transduction efficiency after 14 days of starting culture (both virus transduction at the same time) | GFP positive population (%) |
|---|---|
| MOI 40:40 | 48.56% |
| MOI 50:50 | 71.3% |

Taking into account the above results of the efficacy experiments, the condition of MOI 100 for BCMA, MOI 75 for GPRC5D and MOI 150 for GFP was selected. The obtained results were considered in relation to the immunophenotype of cells and their viability. Hence, the most effective results of transduction efficiency and cell viability were selected.

## EXAMPLE 2

### GENERATING OF ANTI-BCMA AND ANTI-GPRC5D LENTIVIRUS PARTICLES

### T CELL TRANSDUCTION WITH ANTI-BCMA AND ANTI-GPRC5D LENTIVIRUS PARTICLES

Activation of CD4+, CD8+ lymphocytes is required for efficient transduction with a lentiviral vector generated as described in **EXAMPLE 1.** T Cell TransAct, human (Miltenyi Biotec, catalog number: 130-128-758) was used for this purpose. The reagent consists of a colloidal polymeric nanomatrix conjugated to humanized CD3 and CD28 agonists, providing primary and costimulatory signals for efficient T cell activation and expansion while maintaining high viability. The most appropriate incubation and cell activation time was determined by flow cytometry. The time from activation to transduction with viral vectors was set at 24 hours. To strengthen and increase the efficiency of transduction, the Vectofusin-1 reagent (Miltenyi Biotec, catalog number: 130-111-163) is added together with the viral vectors. It is a fully synthetic, non-toxic cationic amphipathic peptide with the ability to enhance viral transduction, allowing higher levels of transduction with small amounts of retroviral vector. It facilitates the entry of several pseudotypes of retroviruses into target cells.

### Mono anti-BCMA

In this variant, CD4+ and CD8+ lymphocytes were only transduced with the anti-BCMA viral vector. It was found that the most efficient case of this vector is the use of MOI 100. For efficient transduction, on day 0, cells were seeded in a suitable culture vessel at a ratio of $1 \times 10^6$ cells/ml TexMACS medium and activated with the T reagent Cell TransAct at 10 $\mu$l/ml medium. On day 1, the cells were transduced with the anti-BCMA lentivirus in the appropriate volume in relation to the initial number of cells to finally obtain MOI 100 with the addition of Vectofusin-1 reagent (10 $\mu$l/ml). It has been found that transduction shows better efficiency when spinoculation is used for transduction. For this, cells with virus particles and a transduction enhancer were centrifuged for 1 hour at 32°C at 400 RCF. The cells were then placed in an incubator at 37°C, 5% $CO_2$ for 18 hours. After this time, medium used for transduction was replaced with TexMACS culture medium supplemented with interleukin 7 (IL-7) and interleukin 15 (IL-15) at a concentration of 155 UI/ml (for IL-7) and 290 UI/ml (for IL-15). In the following days, the medium was changed every 48 hours and, additionally, on days 9 and 11, their immunophenotype and the fluorescence level were checked using flow cytometry. The culture was carried out for 14 days, on 14 days the immunophenotype and fluorescence level were checked again, and then the cells are frozen in FreezeMe One, Cryopreservation Medium with Fetal Bovine Serum (FBS) (Capricorn Scientific, catalog number: FM1-F) in concentration 10 x $10^6$/ml and cryopreserved in liquid nitrogen.

### Mono anti-GPRC5D

CD4+, CD8+ lymphocytes were transduced exclusively with the anti-GPRC5D lentiviral particles. It was found that the most efficient case of this vector is the use of MOI 75. For efficient transduction, on day 0, cells were seeded in an appropriate culture vessel at a ratio of $1 \times 10^6$ cells/ml TexMACS medium and activated with T reagent Cell TransAct at 10 $\mu$l/ml medium. On day 1, the cells were transduced with the anti-GPRC5D lentivirus in the appropriate volume in relation to the initial number of cells to finally obtain MOI 75 with the addition of Vectofusin-1 reagent (10 $\mu$l/ml). The cells were then centrifuged for 1 hour at 32°C at 400 RCF. The cells were then placed in an incubator at 37°C, 5% $CO_2$ for 18 hours. After this time, the medium used for transduction was replaced with TexMACS culture medium supplemented with interleukin 7 (IL-7) and interleukin 15 (IL-15) at a concentration of 155 UI/ml (for IL-7) and 290 UI/ml (for IL-15). In the following days, the medium was changed every 48 hours and, additionally, on days 9 and 11, their immunophenotype and the level of fluorescence were checked using flow cytometry. The culture was carried out for 14 days, on 14 days the immunophenotype and fluorescence level were checked again, and then the cells are frozen in FreezeMe One, Cryopreservation Medium with Fetal Bovine Serum (FBS) in concentration 10 x $10^6$/ml and cryopreserved in liquid nitrogen.

## EXAMPLE 3

### GENERATION OF DUAL ANTI-BCMA, ANTI-GPRCSD CD4+ AND CD8+ LYMPHOCYTES

### DUAL ANTI-BCMA, ANTI-GPRC5D

CD4+ and CD8+ lymphocytes were transduced with both anti-BCMA and anti-GPRC5D lentivirus particles generated as described in **EXAMPLE 1.**

It was determined that the most efficient case of this vector is the use of an MOI (multiplicity of infection) of 50 for the anti-BCMA viral particles and an MOI of 50 for the anti-GPRC5D viral particles. For efficient transduction on day 0, cells were plated in a suitable culture vessel at $1 \times 10^6$ cells/1ml TexMACS medium and activated with T Cell TransAct at 10 $\mu$l/ml medium. On day 1, the cells were transduced in the appropriate volume in relation to the initial number of cells to finally obtain MOI 50 and 50 with the addition of Vectofusin-1 reagent (10 $\mu$l/ml). The cells were then centrifuged for 1 hour at 32°C at 400 RCF. The cells were then placed in an incubator at 37°C, 5% $CO_2$ for 18 hours. After this time, the medium used for transduction were replaced with TexMACS medium supplemented with interleukin 7 (IL-7) and interleukin 15 (IL-15) at a concentration of 155 UI/ml (for IL-7) and 290 UI/ml (for IL-15). In the following days, the medium was changed every 48 hours and, additionally, on days 9 and 11, their immunophenotype and the level of GFP fluorescence were checked using flow cytometry. The culture was carried out for 14 days, on 14 days the immunophenotype and fluorescence level were checked again, and then the cells were frozen in FreezeMe One, Cryopreservation Medium with Fetal Bovine Serum (FBS) in concentration 10 x $10^6$/ml and cryopreserved in liquid nitrogen.

## EXAMPLE 4

### DOUBLE TRANSDUCTION

### ANTI-BCMA MOI 100 + 2ND DAY ANTI-GPRCSD MOI 75

CD4+ and CD8+ lymphocytes were transduced with both anti-BCMA and anti-GPRC5D lentivirus particles generated as described in **EXAMPLE 1,** but on different days. For efficient transduction on day 0, cells were plated in a suitable culture vessel at $1 \times 10^6$ cells/1ml TexMACS medium and activated with T Cell TransAct at 10 $\mu$l/ml medium. In this case, on day 1, the cells were transduced with the anti-BCMA lentivirus in the appropriate volume in relation to the initial number of cells to finally obtain MOI 100 with the addition of Vectofusin-1 reagent (10 $\mu$l/ml). The cells were then centrifuged for 1 hour at 32°C at 400 RCF. The cells were placed in an incubator at 37°C, 5% CO2 for 18 hours. After this time, the cells were counted, the medium was replaced and transduction is carried out again, this time with the anti-GPRC5D virus in the appropriate volume in relation to the initial number of cells to finally obtain MOI 75 with the addition of Vectofusin-1 reagent (10 $\mu$l/ml). The cells were then centrifuged for 1 hour at 32°C at 400 RCF. The cells were then placed in an incubator at 37°C, 5% $CO_2$ for 18 hours. After this time, the cells were replaced with TexMACS medium supplemented with interleukin 7 (IL-7) and interleukin 15 (IL-15) at a concentration of 155 UI/ml (for IL-7) and 290 UI/ml (for IL-15). In the following days, the medium was changed every 48 hours and, additionally, on days 9 and 11, their immunophenotype and the level of fluorescence were checked using flow cytometry. The culture is carried out for 14 days, on 14 days the immunophenotype and fluorescence level were checked again, and then the cells are frozen in FreezeMe One, Cryopreservation Medium with Fetal Bovine Serum (FBS) in concentration 10 x $10^6$/ml and cryopreserved in liquid nitrogen.

## EXAMPLE 5

## DOUBLE TRANSDUCTION

**ANTI-BCMA MOI 50 + 2ND DAY ANTI-GPRCSD MOI 50**

CD4+ and CD8+ lymphocytes are transduced with both anti-BCMA and anti-GPRC5D lentivirus particles generated as described in **EXAMPLE 1,** but on different days. For efficient transduction on day 0, cells were plated in a suitable culture vessel at $1 \times 10^6$ cells/1ml TexMACS medium and activated with T Cell TransAct at 10 $\mu$l/ml medium. In this case, on day 1, the cells were transduced with the anti-BCMA viral particles in the appropriate volume in relation to the initial number of cells to finally obtain MOI 50 with the addition of Vectofusin-1 reagent (10 $\mu$l/ml). The cells were then centrifuged for 1 hour at 32°C at 400 RCF. The cells were then placed in an incubator at 37°C, 5% $CO_2$ for 18 hours. After this time, the cells were counted, the medium was replaced and transduction was carried out again, this time with the anti-GPRC5D virus in the appropriate volume in relation to the initial number of cells to finally obtain MOI 50 with the addition of Vectofusin-1 reagent (10 $\mu$l/ml). The cells are then centrifuged for 1 hour at 32°C at 400 x g. The cells are then placed in an incubator at 37°C, 5% $CO_2$ for 18 hours. After this time, the cells were replaced with TexMACS medium supplemented with interleukin 7 (IL-7) and interleukin 15 (IL-15) at a concentration of 155 UI/ml (for IL-7) and 290 UI/ml (for IL-15). In the following days, the medium was changed every 48 hours and, additionally, on days 9 and 11, their immunophenotype and the level of fluorescence were checked using flow cytometry. The culture was carried out for 14 days, on 14 days the immunophenotype and fluorescence level were checked again, and then the cells are frozen in FreezeMe One, Cryopreservation Medium with Fetal Bovine Serum (FBS) in concentration 10 x $10^6$/ml and cryopreserved in liquid nitrogen.

## EXAMPLE 6

## DOUBLE TRANSDUCTION

**ANTI-BCMA MOI 100 + 3RD DAY ANTI-GPRCSD MOI 75**

CD4+ and CD8+ lymphocytes were transduced with both anti-BCMA and anti-GPRC5D viral particles generated as described in **EXAMPLE 1,** but on different days. For efficient transduction on day 0, cells were plated in a suitable culture vessel at $1 \times 10^6$ cells/1ml TexMACS medium and activated with T Cell TransAct at 10$\mu$l/ml medium. In this case, on day 1, the cells were transduced with the anti-BCMA viral particles in the appropriate volume in relation to the initial number of cells to finally obtain MOI 100 with the addition of Vectofusin-1 reagent (10 $\mu$l/ml). The cells were then centrifuged for 1 hour at 32°C at 400 RCF. The cells were then placed in an incubator at 37°C, 5% $CO_2$ for 18 hours. After this time, the medium used for transduction were replaced with TexMACS culture medium supplemented with interleukin 7 (IL-7) and interleukin 15 (IL-15) at a concentration of 155 UI/ml (for IL-7) and 290 UI/ml (for IL-15). After 24 hours from the change of the medium, the cells were counted, the medium was replaced and transduced again, this time with the anti-GPRC5D virus in the appropriate volume in relation to the initial number of cells to finally obtain MOI 75 with the addition of Vectofusin-1 reagent (10 $\mu$l/ml). The cells were then centrifuged for 1 hour at 32°C at 400 RCF. The cells were then placed in an incubator at 37°C, 5% $CO_2$ for 18 hours. After this time, the cells were replaced with TexMACS medium supplemented with interleukin 7 (IL-7) and interleukin 15 (IL-15) at a concentration of 155 UI/ml (for IL-7) and 290 UI/ml (for IL-15). In the following days, the medium was changed every 48 hours and, additionally, on days 9 and 11, their immunophenotype and the level of fluorescence are checked using flow cytometry. The culture was carried out for 14 days, on 14 days the immunophenotype and fluorescence level were checked again, and then the cells were frozen in FreezeMe One, Cryopreservation Medium with Fetal Bovine Serum (FBS) in concentration 10 $\times$ $10^6$/ml and cryopreserved in liquid nitrogen.

## EXAMPLE 7

## DOUBLE TRANSDUCTION

**ANTI-BCMA MOI 50 + 3RD DAY ANTI-GPRCSD MOI 50**

CD4+ and CD8+ lymphocytes were transduced with both anti-BCMA and anti-GPRC5D lentivirus particles generated as described in **EXAMPLE 1,** but on different days. For efficient transduction on day 0, cells were plated in a suitable culture vessel at $1 \times 10^6$ cells/1ml TexMACS medium and activated with T Cell TransAct at 10 $\mu$l/ml medium. In this case, on day 1, the cells were transduced with the anti-BCMA viral particles in the appropriate volume in relation to the initial number of cells to finally obtain MOI 50 with the addition of Vectofusin-1 reagent (10 $\mu$l/ml). The cells were then centrifuged for 1 hour at 32°C at 400 RCF. The cells were then placed in an incubator at 37°C, 5% $CO_2$ for 18 hours. After this time, the cells were replaced with TexMACS medium supplemented with interleukin 7 (IL-7) and interleukin 15 (IL-15) at a concentration of 155 UI/ml (for IL-7) and 290 UI/ml (for IL-15). After 24 hours from the change of the medium, the cells were counted, the medium was replaced and transduced again, this time with the anti-GPRC5D virus in the appropriate volume in relation to the initial number of cells to finally obtain MOI 50 with the addition of Vectofusin-1 reagent (10 μl/ml). The cells were then centrifuged for 1 hour at 32°C at 400 RCF. The cells were then placed in an incubator at 37°C, 5% $CO_2$ for 18 hours. After this time, the cells were replaced with TexMACS medium supplemented with interleukin 7 (IL-7) and interleukin 15 (IL-15) at a concentration of 155 UI/ml (for IL-7) and 290 UI/ml (for IL-15). In the following days, the medium was changed every 48 hours and, additionally, on days 9 and 11, their immunophenotype and the level of fluorescence were checked using flow cytometry. The culture was carried out for 14 days, on 14 days the immunophenotype and fluorescence level were checked again, and then the cells were frozen in FreezeMe One, Cryopreservation Medium with Fetal Bovine Serum (FBS) in concentration 10 x $10^6$/ml and cryopreserved in liquid nitrogen.

## EXAMPLE 8

## RESULTS OBTAINED FROM EXAMPLES 3-7

### DAY 0 - beginning of cell culture and T cells activation

Commercial cell lines of CD4+ and CD8+ T cells were used for the experiment. The cells were thawed and cultured in a ratio 1:2 of CD8+ to CD4+ cells, imitating the normal CD4+ to CD8+ ratio which is greater than 1.0, with CD4 lymphocytes ranging from 500 to 1200/mm$^3$ and CD8 lymphocytes ranging from 150 to 1000/mm $^3$ (Garrido-Rodriguez V, Herrero-Fernández I, Castro MJ, Castillo A, Rosado-Sánchez I, Galvá MI, Ramos R, Olivas-Martinez I, Bulnes-Ramos A, Cañizares J, Leal M, Pacheco YM. Immunological features beyond CD4/CD8 ratio values in older individuals. Aging (Albany NY). 2021 May 26; 13(10): 13443-13459 Epub 2021 May 26. PMID: 34038386; PMCID: PMC8202849).

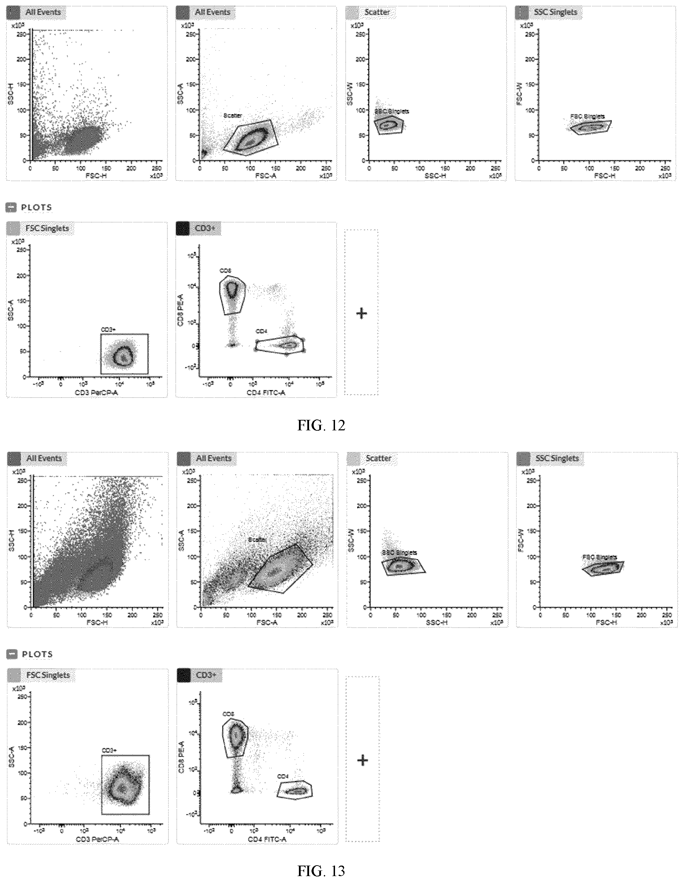

Immunophenotyping was performed using the BD Tritest™ CD4 FITC/CD8 PE/CD3 PerCP to confirm the purity and ratio of CD4+ and CD8+ cells of the co-culture. The percentage of CD4+ cells obtained was 61.52% and the percentage of CD8+ cells was 34.96% (see Fig.12).

Cells were seeded in 24-well plates at $1 \times 10^6$ cells/well at a ratio of $1 \times 10^6$/ml in TexMACS medium with TransAct (10 μl/ml).

### DAY 1

All cells were counted and transduced with anti-BCMA virus in the appropriate MOI. Conditions 100-1 and 175-1 were additionally transduced with anti-GPRC5D virus and addiction of Vectofusin -1 (10μl/ml) at the same time.

- (175-1) - BCMA virus MOI 100 + GPRC5D virus MOI 75 + Vectofusin-1 (at the same time)
- (100-1) - BCMA virus MOI 50 + GPRC5D virus MOI 50 + Vectofusin-1 (at the same time)
- (175-2) - BCMA virus MOI 100 + Vectofusin-1 (GPRC5D transduction on day 2nd)
- (100-2) - BCMA virus MOI 50 + Vectofusin-1 (GPRC5D transduction on day 2nd)
- (175-3) - BCMA virus MOI 100 + Vectofusin-1 (GPRC5D transduction on day 3rd)
- (100-3) - BCMA virus MOI 50 + Vectofusin-1 (GPRC5D transduction on day 3rd) The plates were centrifuged for 1h, 32°C, 400 RCF and placed in a shaking incubator.

### DAY 2

Cells from the plate were transferred to 50 ml TPP culture falcons and counted. Then cells were centrifuged at 300 RCF for 5 min, culture medium with transduction mixture was aspirated, add the required amount of TexMACS medium with IL-7 (3.1 μl/ml) and IL-15 (5.8 μl/ml) except for the condition (175-2) and (100-2). Cells from condition: 175-2 (anti-BCMA MOI 100, Anti-GPRC5D MOI 75 - 2nd day) and from condition 100-2 (anti-BCMA MOI 50, anti-GPRC5D MOI 50 - 2nd day) were transduced with anti-GPRC5D MOI 75 and 50 virus, supplemented with Vectofusin-1 (10 μl/ml) in medium (TexMACS) without interleukins, transferred for 2 wells in a 6-well plate, centrifuge the plate for 1h, 32°C, 400 RCF and cultured with shaking.

Table 8 Cell amount and viability.

| Experiment symbol | Description | Total cell number | Cell viability | Amount of medium and interleukins |
|---|---|---|---|---|
| 175-1 | Anti-BCMA MOI 100, Anti-GPRC5D MOI 75 - 1st day | $0.514 \times 10^6 \times 1$ ml $= 0.51 \times 10^6$ | 83.4% | **1 ml;** 3.1 μl IL-7; 5.8 μl IL-15 |
| 100-1 | Anti-BCMA MOI 50, Anti-GPRC5D MOI 50 - 1st day | $0.507 \times 10^6 \times 1$ ml $= 0.51 \times 10^6$ | 91.1% | **1 ml;** 3.1 μl IL-7; 5.8 μl IL-15 |
| 175-2 | Anti-BCMA MOI 100, Anti-GPRC5D MOI 75 - 2nd dav | $0.698 \times 10^6 \times 1$ ml $= 0.7 \times 10^6$ | 91.3% | **0.7 ml;** |
| 100-2 | Anti-BCMA MOI 50, Anti-GPRC5D MOI 50 - 2nd day | $0.808 \times 10^6 \times 1$ ml $= 0.8 \times 10^6$ | 92.6% | **0.8 ml;** |
| 175-3 | Anti-BCMA MOI 100, Anti-GPRC5D MOI 75 - 3rd day | $0.646 \times 10^6 \times 1$ ml $= 0.65 \times 10^6$ | 93.3% | **1 ml;** 3.1 μl IL-7; 5.8 μl IL-15 |
| 100-3 | Anti-BCMA MOI 50, Anti-GPRC5D MOI 50 - 3rd day | $0.726 \times 10^6 \times 1$ ml $= 0.73 \times 10^6$ | 94.1% | **1 ml;** 3.1 μl IL-7; 5.8 μl IL-15 |
| Control | Negative Control | $0.65 \times 10^6 \times 5$ ml $= 3.25 \times 10^6$ | 94.2% | **3.2 ml;** 9.9 μl IL-7; 18.6 μl IL-15 |

**DAY 3**

The transduced cells from the plate the day before were transferred to 50 ml TPP culture falcons. Cells were counted in each condition. The cells were centrifuged at 300 RCF for 5 min, the culture medium with the transduction mixture was aspirated, the required amount of TexMACS medium with IL-7 (3.1 μl/ml) and IL-15 (5.8 μl/ml) was added. Cells from Condition 175-3 (anti-BCMA MOI 100, anti-GPRC5D MOI 75 -3rd day) and 100-3 (anti-BCMA MOI 50, anti-GPRC5D MOI 50 - 3rd day) transduced with anti-GPRC5D virus supplemented with Vectofusin-1 (10 μl/ml) in medium (TexMACS) without interleukins and transfered to 2 wells in a 6-well plate. Centrifuged the plate for 1h at 32°C, 400 RCF and placed in an incubator with shaker.

Table 9 Cell amount and viability.

| Experiment symbol | Description | Total cell number | Cell viability | Amount of medium and interleukins |
|---|---|---|---|---|
| 175-2 | Anti-BCMA MOI 100, Anti-GPRC5D MOI 75 - 2nd day | $1.51 \times 10^6 \times 0.8$ ml $= 1.2 \times 10^6$ | 95.1% | **1.5 ml;** 4.7 μl IL-7; 8.7 μl IL-15 |
| 100-2 | Anti-BCMA MOI 50, Anti-GPRC5D MOI 50 - 2nd dav | $1.16 \times 10^6 \times 0.9$ ml $= 1.1 \times 10^6$ | 94.2% | **1.5 ml;** 4.7 μl IL-7; 8.7 μl IL-15 |
| 175-3 | Anti-BCMA MOI 100, Anti-GPRC5D MOI 75 - 3rd dav | $1.37 \times 10^6 \times 1$ ml $= 1.4 \times 10^6$ | 96.8% | **1.4 ml;** |
| 100-3 | Anti-BCMA MOI 50, Anti-GPRC5D MOI 50 - 3rd dav | $1.63 \times 10^6 \times 1$ ml $= 1,.6 \times 10^6$ | 94.2% | **1.6 ml;** |

**DAY 4**

The transduced cells from the plate the day before were transferred to 50 ml TPP culture falcons. Cells were counted in each condition. The cells were centrifuged at 300 RCF for 5 min, the culture medium with the transduction mixture was aspirated, the required amount of TexMACS medium with IL-7 (3.1 µl/ml) and IL-15 (5.8 µl/ml) was added. Cell was placed in an incubator with shaker.

Table 10 Cell amount and viability.

| Experiment symbol | Description | Total cell number | Cell viability | Amount of medium and interleukins |
|---|---|---|---|---|
| 175-1 | Anti-BCMA MOI 100, Anti-GPRC5D MOI 75 - 1st dav | $3.25 \times 10^6 \times 1$ ml = $3.3 \times 10^6$ | 94.6% | **3.5 ml;** 10.9 µl IL-7; 20.3 µl IL-15 |
| 100-1 | Anti-BCMA MOI 50, Anti-GPRC5D MOI 50 - 1st dav | $3.28 \times 10^6 \times 1$ ml = $3.3 \times 10^6$ | 95.4% | **3.5 ml;** 10.9 µl IL-7; 20.3 µl IL-15 |
| 175-3 | Anti-BCMA MOI 100, Anti-GPRC5D MOI 75 - 3rd day | $1.91 \times 10^6 \times 1.6$ ml = $3.06 \times 10^6$ | 94.3% | **3.5 ml;** 10.9 µl IL-7; 20.3 µl IL-15 |
| 100-3 | Anti-BCMA MOI 50, Anti-GPRC5D MOI 50 - 3rd dav | $1.93 \times 10^6 \times 1.8$ ml = $3.5 \times 10^6$ | 95.2% | **3.5 ml;** 10.9 µl IL-7; 20.3 µl IL-15 |
| Control | Negative Control | $3.61 \times 10^6 \times 3.2$ ml = $11.6 \times 10^6$ | 93.7% | **12 ml;** 37.2 µl IL-7; 69.6 µl IL-15 |

**DAY 5**

Cells were counted in each condition. The cells were centrifuged at 300 RCF for 5 min, the culture medium with the transduction mixture was aspirated, the required amount of TexMACS medium with IL-7 (3.1 µl/ml) and IL-15 (5.8 µl/ml) was added. Cell was placed in an incubator with shaker.

Table 11 Cell amount and viability.

| Experiment symbol | Description | Total cell number | Cell viability | Amount of medium and interleukins |
|---|---|---|---|---|
| 175-2 | Anti-BCMA MOI 100, Anti-GPRC5D MOI 75 - 2nd dav | $3.075 \times 10^6 \times 1.5$ ml = $4.6125 \times 10^6$ | 94.85% | **4.5 ml;** 13.85 µl IL-7; 26.1 µl IL-15 |
| 100-2 | Anti-BCMA MOI 50, Anti-GPRC5D MOI 50 - 2nd dav | $3 \times 10^6 \times 1.5$ ml = $4.5 \times 10^6$ | 94.1% | **4.5 ml;** 13.85 µl IL-7; 26.1 µl IL- 15 |

**DAY 6**

Cells were counted in each condition. The cells were centrifuged at 300 RCF for 5 min, the culture medium with the transduction mixture was aspirated, the required amount of TexMACS medium with IL-7 (3.1 µl/ml) and IL-15 (5.8 µl/ml) was added. Cell was placed in an incubator with shaker.

Table 12 Cell amount and viability.

| Experiment symbol | Description | Total cell number | Cell viability | Amount of medium and interleukins |
|---|---|---|---|---|
| 175-1 | Anti-BCMA MOI 100, Anti-GPRC5D MOI 75 - 1st dav | $2.88 \times 10^6 \times 3.5$ ml = $10.1 \times 10^6$ | 95.5% | **10.5 ml;** 32.6 $\mu$l IL-7; 60.9 $\mu$l IL-15 |
| 100-1 | Anti-BCMA MOI 50, Anti-GPRC5D MOI 50 - 1st dav | $2.73 \times 10^6 \times 3.5$ ml = $9.5 \times 10^6$ | 96.1% | **9.5 ml;** 29.5 $\mu$l IL-7; 55.1 $\mu$l IL-15 |
| 175-3 | Anti-BCMA MOI 100, Anti-GPRC5D MOI 75 - 3rd dav | $3.7 \times 10^6 \times 3.5$ ml = $12.9 \times 10^6$ | 96.3% | **13 ml;** 40.3 $\mu$l IL-7; 75.4 $\mu$l IL-15 |
| 100-3 | Anti-BCMA MOI 50, Anti-GPRC5D MOI 50 - 3rd dav | $3.08 \times 10^6 \times 3.5$ ml = $10.8 \times 10^6$ | 95.3% | **11 ml;** 34.1 $\mu$l IL-7; 63.8 $\mu$l IL-15 |
| Control | Negative Control | $3.75 \times 10^6 \times 12$ ml = $45 \times 10^6$ | 96.6% | **45 ml;** 139.5 $\mu$l IL-7; 261 $\mu$l IL-15 |

**DAY 7**

Cells were counted in each condition. The cells were centrifuged at 300 RCF for 5 min, the culture medium with the transduction mixture was aspirated, the required amount of TexMACS medium with IL-7 (3.1 $\mu$l/ml) and IL-15 (5.8 $\mu$l/ml) was added. Cell was placed in an incubator with shaker.

Table 13 Cell amount and viability with % of GPF transduction efficiency.

| Experiment symbol | Description | Total cell number | Cell viability | Amount of medium and interleukins | % GFP positive cells |
|---|---|---|---|---|---|
| 175-2 | Anti-BCMA MOI 100, Anti-GPRC5D MOI 75 - 2nd day | $2.05 \times 10^6 \times 4.5$ ml = $9.23 \times 10^6$ | 96.5% | **9.5 ml;** 29.5 $\mu$l IL-7; 55.1 $\mu$l IL-15 **9.5 ml - 0.5 ml = 9 ml** | 42.62% |
| 100-2 | Anti-BCMA MOI 50, Anti-GPRC5D MOI 50 - 2nd day | $2.05 \times 10^6 \times 4.5$ ml = $9.23 \times 10^6$ | 96.2% | **9.5 ml;** 29.5 $\mu$l IL-7; 55.1 $\mu$l IL-15 **9.5 ml - 0.5 ml = 9 ml** | 45.02% |

**DAY 8**

Cells were counted in each condition. Immunophenotyping for the number of GFP positive cells was performer. The cells were centrifuged at 300 RCF for 5 min, the culture medium with the transduction mixture was aspirated, the required amount of TexMACS medium with IL-7 (3.1 $\mu$l/ml) and IL-15 (5.8 $\mu$l/ml) was added. Cell was placed in an incubator with shaker.

Table 14 Cell amount and viability with % of GPF transduction efficiency.

| Experiment symbol | Description | Total cell number | Cell viability | Amount of medium and interleukins | % GFP positive cells |
|---|---|---|---|---|---|
| 175-1 | Anti-BCMA MOI 100, Anti-GPRC5D MOI 75 - 1st day | $2.92 \times 10^6 \times 10.5$ ml = $30.6 \times 10^6$- 1 = $249.6 \times 10^6$ | 90.8% | **30 ml;** 93 $\mu$l IL-7; 174 $\mu$l IL-15 | 44.85% |
| 100-1 | Anti-BCMA MOI 50, Anti-GPRC5D MOI 50 - 1st day | $2.64 \times 10^6 \times 9.5$ ml = $25.1 \times 10^6$- 1 = $24.1 \times 10^6$ | 92.3% | **24 ml;** 74.4 $\mu$l IL-7; 139.2 $\mu$l IL-15 | 46.58% |

(continued)

| Experiment symbol | Description | Total cell number | Cell viability | Amount of medium and interleukins | % GFP positive cells |
|---|---|---|---|---|---|
| 175-3 | Anti-BCMA MOI 100, Anti-GPRC5D MOI 75 - 3rd day | $2.1 \times 10^6 \times 13$ ml = 27.3 x $10^6$ - 1 = 26.3 x $10^6$ | 90.2% | **26 ml;** 80.6 $\mu$l IL-7; 151 $\mu$l IL-15 | 48.36% |
| 100-3 | Anti-BCMA MOI 50, Anti-GPRC5D MOI 50 - 3rd day | $2.1 \times 10^6 \times 11$ ml = 23.1 $\times 10^6$ - 1 = 22.1 x $10^6$ | 91.8% | **22 ml;** 68.2 $\mu$l IL-7; 127.6 $\mu$l IL-15 | 44.27% |
| Control | Negative control | 1.62 x $10^6$ x 12.5 ml = 20.7 x $10^6$ - 1 = 19.7 $\times$ $10^6$ | 94% | **20 ml;** 62 $\mu$l IL-7; 116 $\mu$l IL-15 | 0% |

**DAY 9**

Cells were counted in each condition. The cells were centrifuged at 300 RCF for 5 min, the culture medium with the transduction mixture was aspirated, the required amount of TexMACS medium with IL-7 (3.1 $\mu$l/ml) and IL-15 (5.8 $\mu$l/ml) was added. Cell was placed in an incubator with shaker.

Table 15 Cell amount and viability.

| Experiment symbol | Description | Total cell number | Cell viability | Amount of medium and interleukins |
|---|---|---|---|---|
| 175-2 | Anti-BCMA MOI 100, Anti-GPRC5D MOI 75 - 2nd day | 2.49 x $10^6$ x 9 ml = 22.4 x $10^6$ | 94.1% | **22.5 ml;** 69.8 $\mu$l IL-7; 131 $\mu$l IL-15 |
| 100-2 | Anti-BCMA MOI 50, Anti-GPRC5D MOI 50 - 2nd day | 2.66 x $10^6$ x 9 ml = 23.9 x $10^6$ | 96.1% | **24 ml;** 74.4 $\mu$l IL-7; 139.2 $\mu$l IL-15 |

**DAY 10**

Cells were counted in each condition. The cells were centrifuged at 300 RCF for 5 min, the culture medium with the transduction mixture was aspirated, the required amount of TexMACS medium with IL-7 (3.1 $\mu$l/ml) and IL-15 (5.8 $\mu$l/ml) was added. Cell was placed in an incubator with shaker.

Table 16 Cell amount and viability with % of GPF transduction efficiency.

| Experiment symbol | Description | Total cell number | Cell viability | Amount of medium and interleukins (20 x $10^6$ cells was seed) | % GFP positive cells |
|---|---|---|---|---|---|
| 175-1 | Anti-BCMA MOI 100, Anti-GPRC5D MOI 75 - 1st dav | 3.59 x $10^6$ x 30 ml = 107.7 x $10^6$ | 95.3% | **20 ml;** 62 $\mu$l IL-7; 116 $\mu$l IL-15 | 64.33% |
| 100-1 | Anti-BCMA MOI 50, Anti-GPRC5D MOI 50 - 1st day | 3.01 x $10^6$ x 24 ml = 72.24 x $10^6$ | 96.3% | **20 ml;** 62 $\mu$l IL-7; 116 $\mu$l IL-15 | 60.56% |
| 175-3 | Anti-BCMA MOI 100, Anti-GPRC5D MOI 75 - 3rd day | 3.21 x $10^6$ x 26 ml = 83.5 x $10^6$ | 94.2% | **20 ml;** 62 $\mu$l IL-7; 116 $\mu$l IL-15 | 77.98% |

(continued)

| Experiment symbol | Description | Total cell number | Cell viability | Amount of medium and interleukins (20 x 10$^6$ cells was seed) | % GFP positive cells |
|---|---|---|---|---|---|
| 100-3 | Anti-BCMA MOI 50, Anti-GPRC5D MOI 50 - 3rd day | 2.9 x 10$^6$ x 22 ml = 63.8 x 10$^6$ | 96.5% | **20 ml;** 62 μl IL-7; 116 μl IL-15 | 74.12% |
| Control | Negative Control | 2.63 x 10$^6$ x 20 ml = 52.5 x 10$^6$ | 96.1% | **20 ml;** 62 μl IL-7; 116 μl IL-15 | 0% |

**DAY 11**

Cells were counted in each condition. Immunophenotyping for the number of GFP positive cells was performed 20 million cells were collected for further culture. The cells were centrifuged at 300 RCF for 5 min, the culture medium with the transduction mixture was aspirated, the required amount of TexMACS medium with IL-7 (3.1 μl/ml) and IL-15 (5.8 μl/ml) was added. Cell was placed in an incubator with shaker.

Table 17 Cell amount and viability with % of GPF transduction efficiency.

| Experiment symbol | Description | Total cell number | Cell viability | Amount of medium and interleukins | % GFP positive cells |
|---|---|---|---|---|---|
| 175-2 | Anti-BCMA MOI 100, Anti-GPRC5D MOI 75 - 2nd day | 2.595 x 10$^6$ x 22.5 ml = 57.09 x 10$^6$ | 97.5% | **20 ml;** 62 μl IL-7; 116 μl IL-15 | 68.83% |
| 100-2 | Anti-BCMA MOI 50, Anti-GPRC5D MOI 50 - 2nd day | 2.04 x 10$^6$ x 24 ml = 48.96 x 10$^6$ | 96.55% | **20 ml;** 62 μl IL-7; 116 μl IL-15 | 72.68% |

**DAY 12**

Cells were counted in each condition. The cells were centrifuged at 300 RCF for 5 min, the culture medium with the transduction mixture was aspirated, the required amount of TexMACS medium with IL-7 (3.1 μl/ml) and IL-15 (5.8 μl/ml) was added. Cell was placed in an incubator with shaker.

Table 18 Cell amount and viability.

| Experiment symbol | Description | Total cell number | Cell viability | Amount of medium and interleukins |
|---|---|---|---|---|
| 175-1 | BCMA MOI 100, GPRC5D MOI 75 - 1st day | 2.235 x 10$^6$ x 18 ml = 42 x 10$^6$ | 95.55% | **42 ml;** 130.2 μl IL-7; 243.6 μl IL-15 |
| 100-1 | BCMA MOI 50, GPRC5D MOI 50 - 1st day | 2.76 x10$^6$ x 18 ml = 49.68 x 10$^6$ | 96.15% | **49.5 ml;** 153.45μl IL-7; 287.1μl IL-15 |
| 175-3 | BCMA MOI 100, GPRC5D MOI 75 - 3rd day | 2.6 x 10$^6$ x 18 ml = 46.8 x 10$^6$ | 96.65% | **47 ml;** 14517 μl IL-7; 272.6 μl IL-15 |
| 100-3 | BCMA MOI 50, GPRC5D MOI 50 - 3rd day | 2.48 x 10$^6$ x 18 ml = 44.64 x 10$^6$ | 96.85% | **44,5 ml;** 137.95 μl IL-7; 258.1 μl IL-15 |

(continued)

| Experiment symbol | Description | Total cell number | Cell viability | Amount of medium and interleukins |
|---|---|---|---|---|
| Control | Negative Control | $2x\ 10^6\ x\ 18\ ml = 36\ x\ 10^6$ | 96.1% | **36 ml;** 111.6 $\mu$l IL-7; 208.8 $\mu$l IL-15 |

**DAY 13**

Cells were counted in each condition. The cells were centrifuged at 300 rpm for 5 min, the culture medium with the transduction mixture was aspirated, the required amount of TexMACS medium with IL-7 (3.1 $\mu$l/ml) and IL-15 (5.8 $\mu$l/ml) was added. Cell was placed in an incubator with shaker.

Table 19 Cell amount and viability.

| Experiment symbol | Description | Total cell number | Cell viability | Amount of medium and interleukins |
|---|---|---|---|---|
| 175-2 | BCMA MOI 100, GPRC5D MOI 75 - 2nd day | $3.1\ x\ 10^6\ x\ 20\ ml = 62\ x\ 10^6$ | 95.2% | **62 ml;** 192.2 $\mu$l IL-7; 359.6 $\mu$l IL-15 |
| 100-2 | BCMA MOI 50, GPRC5D MOI 50 - 2nd day | $3.58\ x\ 10^6\ x\ 20\ ml = 71.6\ x\ 10^6$ | 97.5% | **72 ml;** 223.2 $\mu$l IL-7; 417.6 $\mu$l IL-15 |

**DAY 14**

Cells were counted in each condition. The $10 \times 10^6$ cells were centrifuged at 300 RCF for 5 min, the culture medium with the transduction mixture was aspirated, the required amount of TexMACS medium with IL-7 (3.1 $\mu$l/ml) and IL-15 (5.8 $\mu$l/ml) was added. Cell was placed in an incubator with shaker. The remaining cells were centrifuged at 300 RCF for 5 min, aspirate the culture medium with the transduction mixture, add the required amount of freezing medium. Placed in a low temperature freezer. Transferred to liquid nitrogen dewar the next day.

Table 20 Cell amount and viability with % of GPF transduction efficiency.

| Experiment symbol | Description | Total cell number | Cell viability | Frozen cells | Amount of medium and interleukins |
|---|---|---|---|---|---|
| 175-1 | BCMA MOI 100, GPRC5D MOI 75 - 1st day | $2.37 \times 10^6 \times 42\ ml = 99.5 \times 10^6 - 10 \times 10^6$ | 97.5% | 8 vials $\times$ 11.2 $\times$ $10^6$ | **10 ml;** 31 $\mu$l IL-7; 58 $\mu$l IL-15 |
| 100-1 | BCMA MOI 50, GPRC5D MOI 50 - 1st day | $1.75 \times 10^6 \times 49\ ml = 85.5 \times 10^6 - 10 \times 10^6$ | 96.1% | 7 vials $\times$ 10.8 $\times$ $10^6$ | **10 ml;** 31 $\mu$l IL-7; 58 $\mu$l IL-15 |
| 175-2 | BCMAMOI 100, GPRC5D MOI 75 - 2nd day | $1.31 \times 10^6 \times 60\ ml = 78.3 \times 10^6 - 10 \times 10^6$ | 98.3% | 6 vials $\times$ 11.4 $\times$ $10^6$ | **10 ml;** 31 $\mu$l IL-7; 58 $\mu$l IL-15 |
| 100-2 | BCMA MOI 50, GPRC5D MOI 50 - 2nd day | $1.24 \times 10^6 \times 70ml = 86.8 \times 10^6 - 10 \times 10^6$ | 98.5% | 7 vials $\times$ 10.9 $\times$ $10^6$ | **10 ml;** 31 $\mu$l IL-7; 58 $\mu$l IL-15 |
| 175-3 | BCMAMOI 100, GPRC5D MOI 75 - 3rd day | $2.25 \times 10^6 \times 47\ ml = 105.8 \times 10^6 - 10 \times 10^6$ | 95.8% | 9 vials $\times$ 10.6 $\times$ $10^6$ | **10 ml;** 31 $\mu$l IL-7; 58 $\mu$l IL-15 |
| 100-3 | BCMA MOI 50, GPRC5D MOI 50 - 3rd day | $2.32 \times 10^6 \times 44\ ml = 101.8 \times 10^6 - 10 \times 10^6$ | 96.3% | 9 vials $\times$ 10.2 $\times$ $10^6$ | **10 ml;** 31 $\mu$l IL-7; 58 $\mu$l IL-15 |

(continued)

| Experiment symbol | Description | Total cell number | Cell viability | Frozen cells | Amount of medium and interleukins |
|---|---|---|---|---|---|
| Control | Negative control | $1.42 \times 10^6 \times 36$ ml = $50.9 \times 10^6$ - $10 \times 10^6$ | 95.9% | 4 vials $\times$ $10.2 \times 10^6$ | **10 ml;** 31 µl IL-7; 58 µl IL-15 |

**DAY 15**

Immunophenotyping was performed. Figure 13 presents immunophenotyping of negative control. It represents the percentage of CD4 and CD8 lymphocyte subpopulations not transduced with a viral vector grown under the same conditions as transduced cells.

Table 21. Percentage of CD4+ and CD8+ cells

| | CD4+ | CD8+ |
|---|---|---|
| Negative Control | 27.29% | 58.70% |

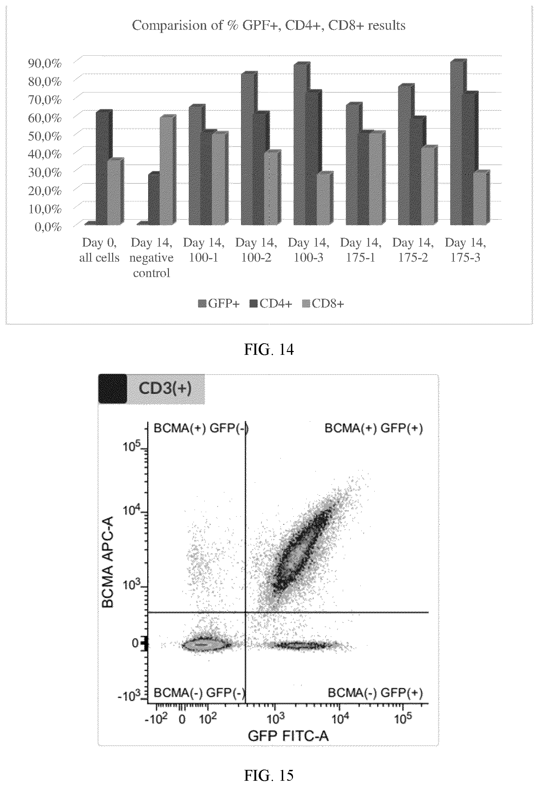

On Figure 14 comparison of the efficiency of the co-transduction process depending on the time of the second anti-GPRC5D transduction can be seen. Within each population, a CD4+ and CD8+ population was immunophenotyped, with **an average of 87.45% of central memory cells for CD4+ and 80.38% for CD8+.** The transduction procedure proceeded according to the previously described schedule:

- (175-1) - BCMA virus MOI 100 + GPRC5D virus MOI 75 + Vectofusin-1 (at the same time)
- (100-1) - BCMA virus MOI 50 + GPRC5D virus MOI 50 + Vectofusin-1 (at the same time)
- (175-2) - BCMA virus MOI 100 + Vectofusin-1 (GPRC5D transduction on day 2nd)
- (100-2) - BCMA virus MOI 50 + Vectofusin-1 (GPRC5D transduction on day 2nd)
- (175-3) - BCMA virus MOI 100 + Vectofusin-1 (GPRC5D transduction on day 3rd)
- (100-3) - BCMA virus MOI 50 + Vectofusin-1 (GPRC5D transduction on day 3rd)

Both CD4 and CD8 T cells in the CAR T cell product contribute to efficacy of the therapy in haematological malignancy but CD8 CAR T cells have more immediate cytotoxic capacity, for this reason, even a slightly higher percentage of these cells seems to be a valuable starting point for therapy.

**EXAMPLE 9**

**TRANSDUCTION IN A CLOSED SYSTEM**

The CliniMACS Prodigy® Instrument (Miltenyi Biotec) with Tubing Set 520 was used for closed cell culture transduction. The protocol established on the device was modified. After installing the required reagents and equipment, a bag with CD4+, CD8+ cell culture was connected to the device in the Reapplication Bag. After selection, ~ $6.2 \times 10^7$ cells were used to initiate cell culture. Cells were cultured using TexMACS® media supplemented with 155 IU/ml IL-7 and 290 IU/ml IL-15 (Miltenyi Biotec #170-076-111 and #170-076-114, respectively). Cells were activated using TransACT GMP Grade (Miltenyi Biotec, Cat. N. 170-076-156) and transduced 24h later using anti-GPRC5D and anti-BCMA lentivirus at MOI = 50 each followed by spinoculation for 120 min in 33°C. The cell culture wash was programmed 48 h after transduction. The cells were then maintained in culture with increasing shaking until the desired cell number was reached. Cells were finally eluted with the PBS/EDTA with human albumin to a final concentration of 0.5%, aliquoted and cryopreserved ~ $10 \times 10^6$ cells in 1 ml Freeze Me One freezing medium in liquid nitrogen.

Table 22 The parameters of CliniMACS Prodigy transduction procedure.

| Day | Activity (Parameter) | Volume | Sample |
|---|---|---|---|
| 0 | Connection, CD4+, CD8+ separation, activation via TransAct | | 1x |
| 1 | Feed (port: 3, vol (+): 30) | 100 | 1x |
| 1 | Volume reduction (port: 3, vol = 50) | 50 | |

(continued)

| Day | Activity (Parameter) | Volume | Sample |
|---|---|---|---|
| 1 | Transduction (reagent vol.: 10 ml) - virus + vectofusine | 80 | |
| 1 | Spin (120 min, 33°C) | 80 | |
| 3 | Medium bag exchange | 80 | |
| 3 | Culture wash (cycles: 1) | 200 | |
| 3 | Volume reduction ( port: 3, vol = 100) | 100 | |
| 3 | Activate shaker (shaker type 2) | 100 | |
| 4 | Sample | | 1x |
| 5 | Media exchange (port: 3, vol (-/+): 25/25) | 100 | |
| 7 | Activate shaker (shaker type 3) | 100 | |
| 7 | Media exchange (port: 3, vol (-/+): 25/25) | 100 | 1x |
| 9 | Media exchange (port: 3, vol (-/+): 25/25) | 100 | 1x |
| 11 | Media exchange (port: 3, vol (-/+): 25/25) | 100 | |
| 11 | Feed (port: 3, vol (+): 80) | 180 | |
| 13 | Media exchange (port: 3, vol (-/+): 100/170) | 250 | |
| 14 | End of culture | 250 | 1x |

On day 14, the culture reached $6.8 \times 10^8$ cells/100 ml, 47.35% GFP+. Importantly, leaving a small part of the cells in further culture in an open system where cell density parameters are precisely controlled, maintaining the ratio of $1 \times 10^6$ cells per ml of medium with the appropriate concentration of interleukins, the percentage of GFP positive cells increased to 64.25% after an additional 7 days of cell culture.

## CELL SORTING

Sorting of BCMA positive cells was performed to determine the sequence of the resulting CAR-T cells and to confirm the presence of both introduced genes. For this purpose, a cell sorter (BD FACS Melody) was used. The dual CAR-T cells collected were sorted using a protein labelled with Allophycocyanin (APC) and anti-CD3 antibody. The population was determined by separating the cells into GFP positive and BCMA positive (Fig. 15).

## CONTROL DUAL CAR-T MYCOPLASMA DETECTION TEST

The presence of *Mycoplasma* in the dual CAR-T cells was excluded by using the PanReac, AppliChem, ITW Reagents, Real time/qPCR test kit for Mycoplasma detection, A9019 (data not shown).

## EXAMPLE 10

## COMPARISON OF % CAR-T CELLS BETWEEN SMALL-SCALE (MANUAL CULTURE) AND CLINIMACS PRODIGY MANUFACTURING SYSTEM

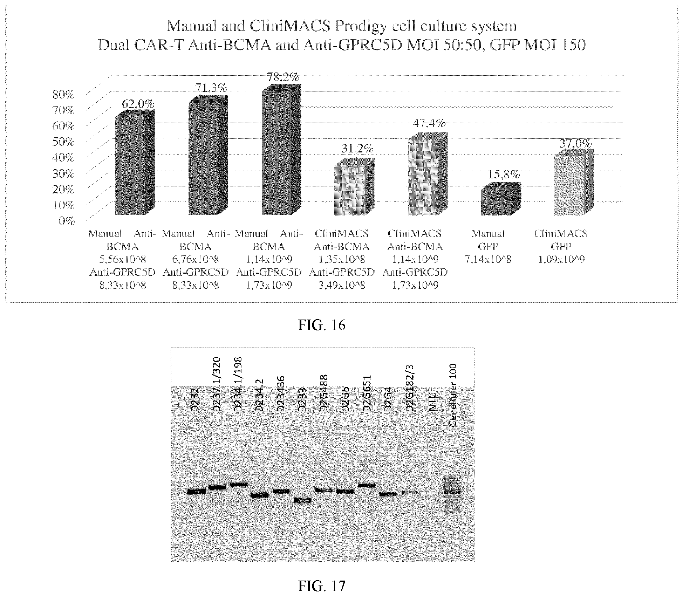

The comparison of the obtained products, namely dual CAR-T and GFP CAR-T, was performed accordingly and presented on Fig. 16.

## DETECTION OF DUAL CAR-T CELLS

### Isolation of DNA

CAR-T cell from cell culture or CliniMACS Prodigy® system (Milteny Biotec) were harvested at $3.0 \times 10^6$ cells, centrifuged for 2 minutes at 1000 RCF. Then DNA was isolated using the GeneMATRIX Tissue DNA Purification Kit [E3550, EURx] and the whole procedure was carried out according to the manufacturer's instructions.

**DNA quantitative assessment**

In the next step, the concentration and purity of the isolated DNA were assessed by measuring the absorbance using the NanoDrop One spectrophotometer (Thermo Fisher Scientific). Isolates were also analysed on a 1.0% agarose gel, stained with Midori Green Advance DNA Stain (MG04, Nippon Genetics), as recommended by the manufacturer. Perfect Plus 1 kb DNA Ladder [E3131, EURx] was used for DNA length estimation at 500 ng per well. Visualization of electropherograms was performed using the GelDoc Go Imaging System (Bio-Rad).

**Storing of DNA isolates**

DNA samples were stored at -20 °C until further analyse.

**_In silico_ analysis of anti-BCMA and anti-GPRC5D sequences and primers design**

To confirm the presence of the anti-BCMA and/or anti-GPRC5D transgenes in the transduced T lymphocytes, the gDNA was sequenced in the appropriate DNA region using specific primers. However, due to the length of the scFv regions of anti-BCMA and anti-GPRC5D plasmids (732 and 750 bp, respectively), it was necessary to design sets of primers whose amplification products would cover the entire region scFv and flanking sequences. The sequences of the primers, their location relative to the plasmids pCDCAR1-LX-R100421-1C-1 and pCDCAR1-LX-R100421-1C-2 and the length of the amplification product obtained using them are presented in the Table 25. The primers were designed using the Primer BLAST® bioinformatics tools available on the website https://www.ncbi.nlm.nih.gov/tools/primer-blast/.

Table 25 Characteristics of primers used for sequencing of anti-BCMA and anti-GPRC5D transgene

| Target gene | Primers | Sequence (5'→3') | Location of the primers on the plasmid template | Product lenght [bp] |
|---|---|---|---|---|
| Anti-BCMA | B2_F | GGGGAGGGGTTTTATGCGAT | 3169-3188 | 435 |
| | B2_R | GAATGTATTTCCACCGCCGC | 3584-3603 | |
| | B3_F | CCCCGGAGGAGGAAGCTTTA | 4524-4543 | 246 |
| | B3_R | AGGCCCTCTTGGGGATTCTT | 4750-4769 | |
| | B4.2_F | CCTGAGTACACATTCGGCCA | 4114-4133 | 343 |
| | B4.2_R | CGTAGGGCTGGTAGTGCTTC | 4437-4456 | |
| | B436_F | CATTTCTGGGTCCCGTCCTC | 4296-4315 | 436 |
| | B436_R | CTACATTTGGGCCCCTCTGG | 4712-4731 | |
| | B4.1_F | GTACTACTGCGCCAAGGTGA | 3705-3724 | 610 |
| | B198_R | CAGAGGGGCCCAAATGTAGA | 4295-4314 | |
| | B7.1_F | GCCAGCGGATTCACTTTCAG | 3496-3515 | 525 |
| | B320_R | GCTGAACCTGTCAGGGATGC | 4001-4020 | |
| Anti-GPRC5D | G4_F | ATAGCGTGAAGGGCAGGTTC | 4004-4023 | 429 |
| | G4_R | TGTACTCGCCATTGGGATCG | 4413-4432 | |
| | G5_F | CCCCGTGCTGGTTATCTACG | 3552-3571 | 471 |
| | G5_R | AACCTGCCCTTCACGCTATC | 4003-4022 | |
| | G488_F | CACTGAGTGGGTGGAGACTG | 3201-3220 | 488 |
| | G488_R | TGCAGTAGTAATCGGCCTCG | 3669-3688 | |
| | G651_F | CTGAGGACGAGGCCGATTAC | 3662-3681 | 651 |
| | G651_R | AATCGCAGGCGAAGTCAAGA | 4293-4312 | |
| | G182_F | TCTTGACTTCGCCTGCGATT | 4293-4312 | 457 |
| | G3_R | CATTTCTGGGTCCCGTCCTC | 4730-4749 | |

**Synthesis, reconstituting and diluting primers**

Primers were synthetized at Genomed S.A. and shipped in a lyophilized state. To create a stock of primers, the primers have been reconstituted in sterile nuclease-free water.

**Modification of PCR conditions**

Modificiaton of PCR conditions was performed to obtain high specificity and efficiency of the amplification reaction. All PCR reactions were performed using the AmpliTaq Gold™ 360 Master Mix kit (4398881, Applied Biosystems™) in a volume of 10 µl. The first step was the selection of the appropriate primer annealing temperature, carried out separately for each pair of primers with the appropriate template (pCDCAR1-LX-R100421-1C-1 or pCDCAR1-LX-R100421-1C-2). Subsequently, the most efficient concentration of primers and the amount of template DNA were selected.

**Evaluation of the length, concentration and purity of the amplification products**

Each of the amplification products was subjected to electrophoretic analysis using a 1.2% agarose gel stained with Midori Green Advance DNA Stain (MG04, Nippon Genetics), as recommended by the manufacturer. GeneRuler 100 DNA Ladder (SM0241, Thermo Scientific™) was used at 500 ng per well to assess the length of the DNA fragments (Fig. 17). Visualization of electropherograms was performed using the GelDoc Go Imaging System (Bio-Rad), and precise DNA fragment length analysis and measuring the intensity of the bands on electropherogram was performed using Image Lab Software 6.1 (Bio-Rad). Fig. 17 presents electropherogram of gDNA amplification products from Dual CAR-T cells with anti-BCMA and anti-GPRC5D sequence-specific primers.

**Sequencing analysis of anti-BCMA and anti-GPRC5D**

The targeted regions of anti-BCMA and anti-GPRC5D were amplified from genomic DNA using the AmpliTaq Gold™ 360 Master Mix kit (4398881, Applied Biosystems™) in a volume of 20 µl, containing also 240 nM primers solution and 20 ng gDNA. The PCR conditions were as follows: 1 cycle at 95°C for 10 min. followed by 35 cycles at 95°C for 30 s, 59°C for 30 s, and 72°C for 45 s, and finally 1 cycle at 72°C for 7 min. PCR was performed using the T-100 Thermal Cycler (Bio-Rad). After confirming the presence and expected length of the amplification products by electrophoretic analysis, they were then purified using ExoSAP-IT™ Express (Applied Biosystems). 10 µl of a post-PCR reaction product was mixed with 4 µl of ExoSAP-IT™ Express reagent. The reaction volume was mixed thoroughly by gentle vortexing and quick spined. Subsequently, the reaction mixture was incubated in a thermal cycler (T100, Bio-Rad) - incubation at 37°C for 4 minutes and then, 80°C for 1 minute to inactivate ExoSAP-IT™ Express reagent. The samples were hold at 4°C pending further analysis. The next stage of the analysis was to carry out the Sanger sequencing reactions using the BigDye™ Terminator v3.1 Cycle Sequencing Kit (Applied Biosystems) with the use of purified PCR products. After amplification, DNA fragments were purified using BigDye XTerminator™. Both of the above reactions were carried out according to the manufacturer's instructions. The last stage of the sequencing analysis of the samples was capillary electrophoresis using the SeqStudio Genetic Analyzer (Applied Biosystems).

***In silico* analysis of sequencing results**

The raw result of PCR product sequences was then analysed *in silico* using the SnapGene program ver. 6.2.1 (Dotmatics) and other applications listed below. In the first stage of analysis via the CAP3 Sequence Assembly Program (Huang, X. and Madan, A. (1999) CAP3: A DNA sequence assembly program. Genome Res., 9, 868-877.) based on single reads, contigs were created. The common region, repeated in both reads (consensus) was the starting sequence for further *in silico* reconstruction, from all prepared contigs, of the full sequence of the analysed region of the anti-BCMA and anti-GPRC5D transgenes. Sequence assembly enabled aligning and merging fragments from a longer DNA sequence in order to reconstruct the original sequence. The complete anti-BCMA and anti-GPRC5D sequences were then compared with the corresponding reference sequences present in the plasmids (pCDCAR1-LX-R100421-1C-1 or pCDCAR1-LX-R100421-1C-2). For this purpose, the tools available in the SnapGene program and with Nucleotide BLAST® (NCBI) were used.

**Confirmation of the presence of the transgene using sequence analysis**

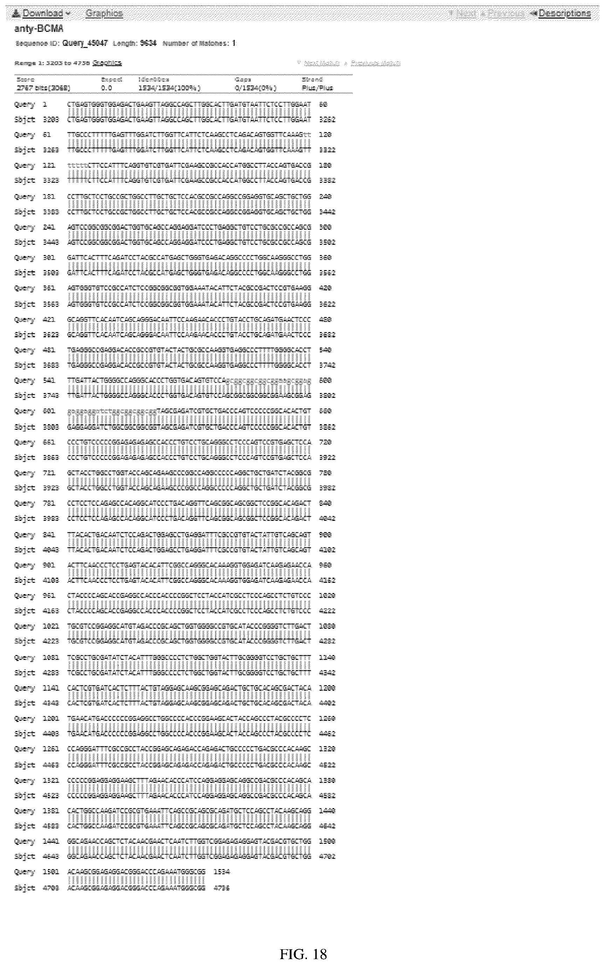

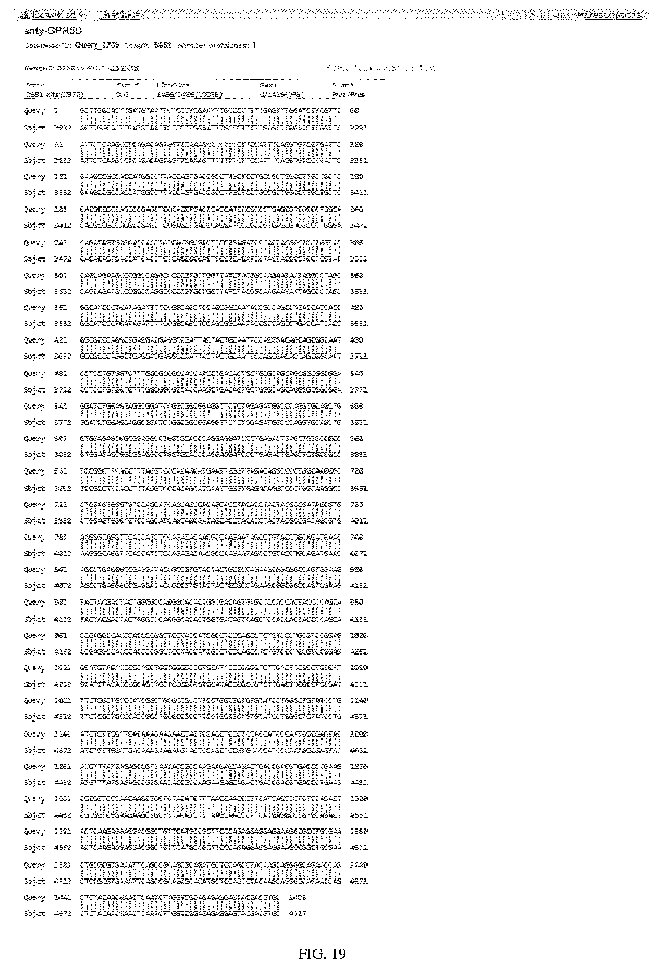

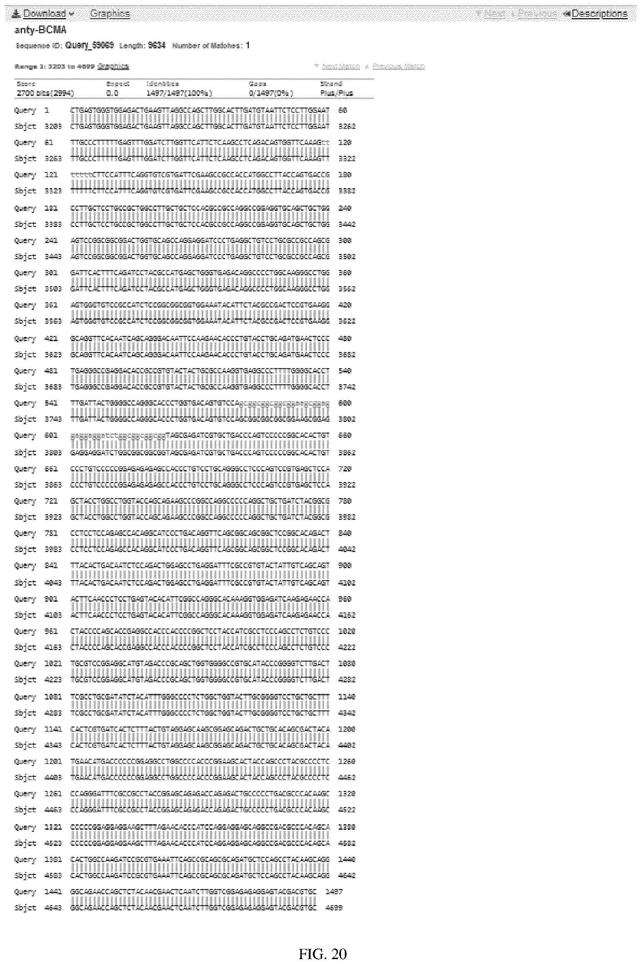

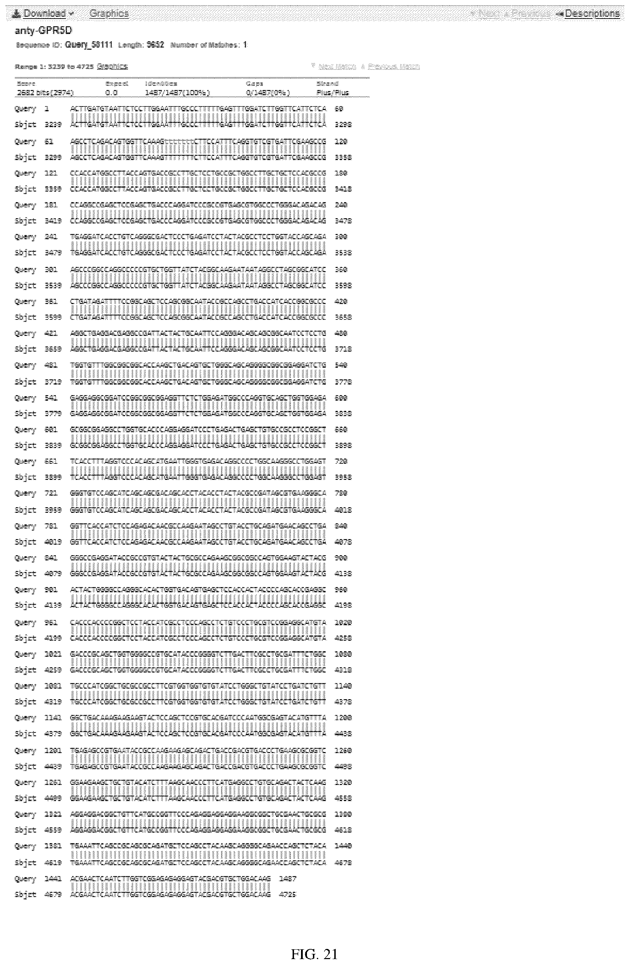

Thanks to the sequencing analysis, it was proved that transgenic anti-BCMA and/or anti-GPRC5D sequences (depending on the type of experiment performed) are present in the genomic DNA from transduced lymphocytes. The length of the full assembled consensus sequences was 1534-1539 bp for anti-BCMA and 1486-1491 bp for anti-GPRC5D. Each time they showed 100% homology with the reference sequences (Fig. 18, 20 and 19, 21, respectively), thus confirming the achievement of transgenic populations of T lymphocytes. In particular, Fig. 18 presents the result of the anti-BCMA sequence align with the reference sequence (pCDCAR1-LX-R100421-1C-1). Genomic DNA was isolated from T lymphocytes modified in a one-step transduction in CliniMACS Prodigy® system (Miltenyi Biotec), Fig. 19 shows the result of the anti-GPRC5D sequence align with the reference sequence (pCDCAR1-LX-R100421-1C-2). Genomic DNA was isolated from T lymphocytes modified in a one-step transduction in CliniMACS Prodigy® system (Miltenyi Biotec), Fig. 20 presents the result of the anti-BCMA sequence align with the reference sequence (pCDCAR1-LX-R100421-1C-1). Genomic DNA was isolated from T lymphocytes modified in a two-step transduction, and Fig. 21 presents the result of the anti-GPRC5D sequence align with the reference sequence (pCDCAR1-LX-R100421-1C-2). Genomic DNA was isolated from T lymphocytes modified in a two-step transduction.

## Detection of the presence of dual CAR-T cells - genetic level

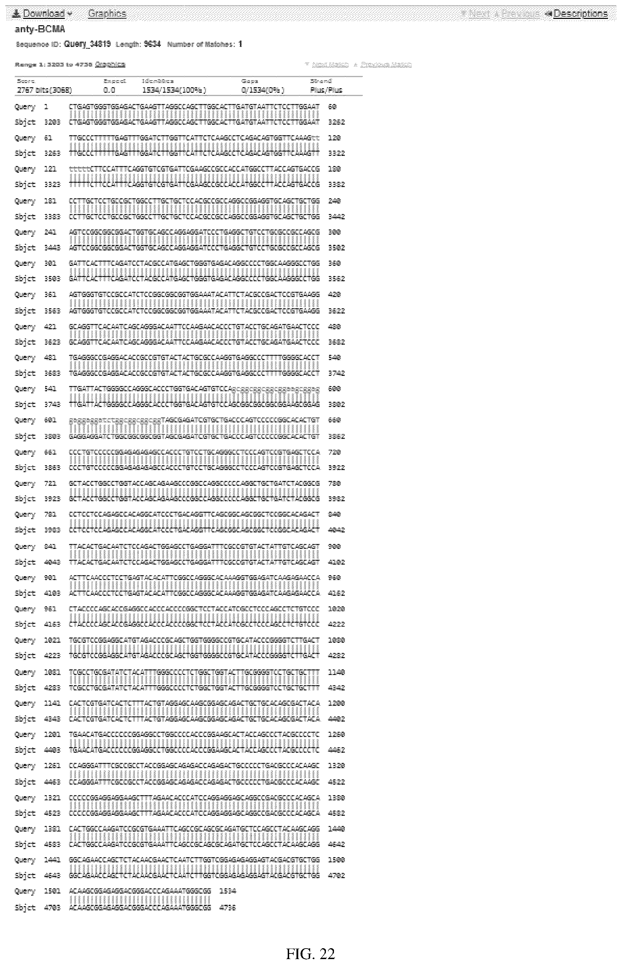

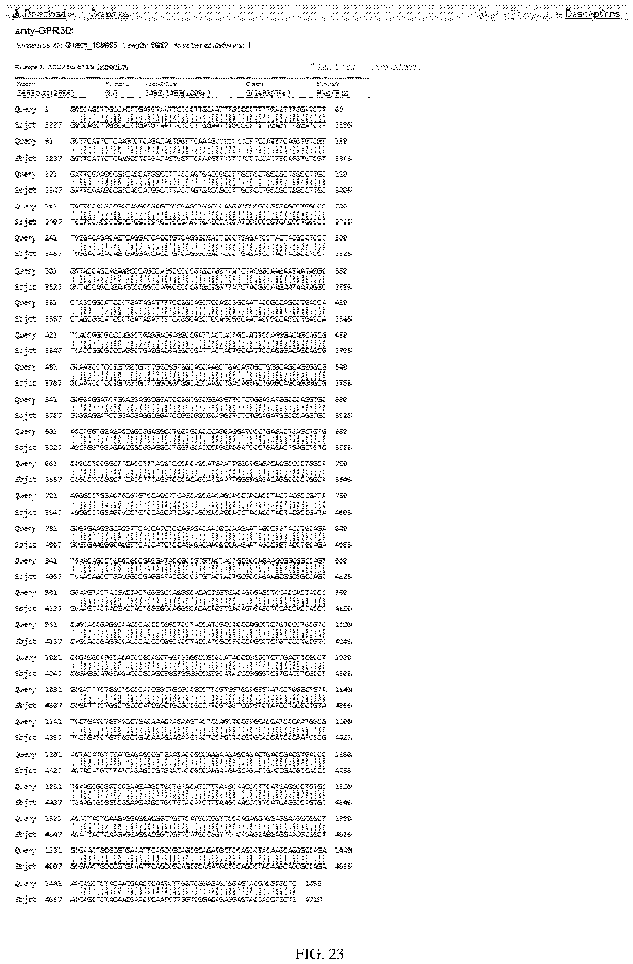

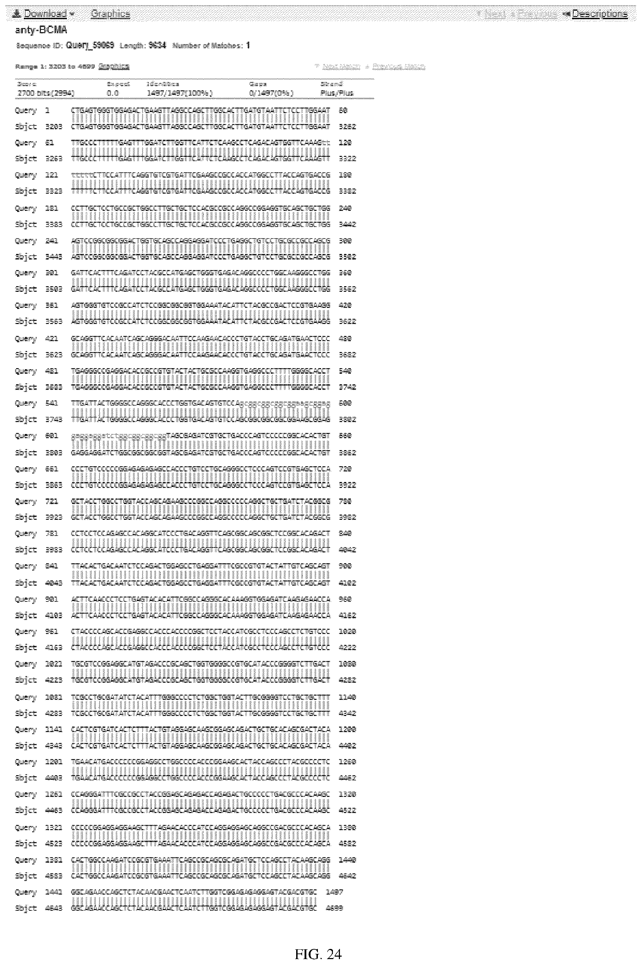

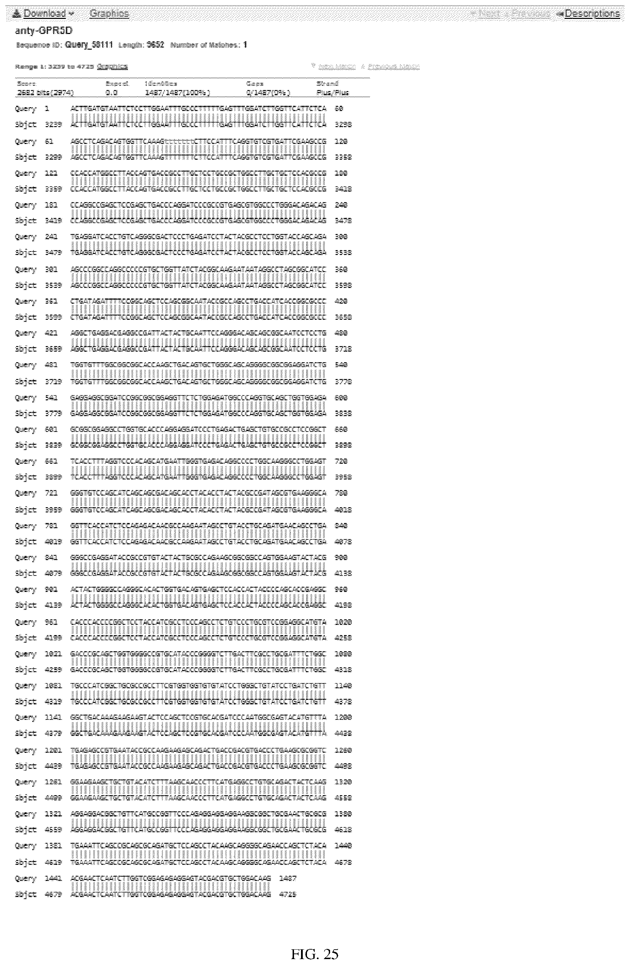

The T cell culture, subjected to the previously described lentiviral transduction procedures was then evaluated for the effectiveness of obtaining dual CAR cells, showing the presence of two transgenes - anti-BCMA and anti-GPRC5D. For this purpose, a population of anti-BCMA-positive cells was isolated from the culture of transduced T cells using the flow cytometer BD FACSMelody™ Cell Sorter (BD Biosciences), using the appropriate labelled antibody (as described above). Then, genomic DNA was isolated from the sorted population of cells, which was amplified with the above-described primers and subjected to the previously described sequencing procedure of specific anti-BCMA and anti-GPRC5D regions. The performed sequence analysis confirmed the presence of all analysed sequences in the cell's genome, encoding both anti-BCMA and anti-GPRC5D antigens (Fig. 22, 24 and 23, 25, respectively), thereby confirming the receipt of double-modified T cells. In particular, Fig. 22 shows the result of the anti-BCMA sequence align with the reference sequence (pCDCAR1-LX-R100421-1C-1). Genomic DNA was isolated from T cells population, expressing the anti-BCMA antigen (one-step transduction of T cells), Fig. 23 presents the result of the anti-GPRC5D sequence align with the reference sequence (pCDCAR1-LX-R100421-1C-2). Genomic DNA was isolated from T cells population, expressing the anti-BCMA antigen (one-step transduction of T cells), Fig. 24. shows the result of the anti-BCMA sequence align with the reference sequence (pCDCAR1-LX-R100421-1C-1). Genomic DNA was isolated from T cells population, expressing the anti-BCMA antigen (two-step transduction of T cells), Fig. 25 presents the result of the anti-GPRC5D sequence align with the reference sequence (pCDCAR1-LX-R100421-1C-2). Genomic DNA was isolated from T cells population, expressing the anti-BCMA antigen (two-step transduction of T cells).

## EXAMPLE 11

## CONFIRMATION OF GENETIC INFORMATION EXPRESSION

In order to confirm the expression of the genetic information contained within the transgenes introduced into T lymphocytes by means of transduction with lentiviral vectors, the expression level of the relevant genes was modified and analysed using the Real Time PCR technique.

In the first stage of the experiment, the single plex qPCR technique was used, and in the next stage, the simultaneous determination of the level of anti-BCMA and anti-GPRC5D expression was performed in a multiplex PCR reaction with appropriately labelled, specific probes. Comparative results of both reactions are presented below.

## Isolation of RNA

RNA from T cell samples for qPCR analysis was isolated with the use of NucleoSpin® RNA Virus (740956.50, Macherey-Nagel). $3.0 \times 10^6$ cells were centrifuged for 2 minutes at 1000 RCF. The supernatant was discarded and the pellet was suspended in 150 $\mu$l of PBS buffer and thoroughly vortexed for 20 sec. The subsequent steps of the procedure were performed in accordance with the kit manufacturer's instructions.

## RNA quantitative assessment

For concentration measurements, UV spectroscopy was applied (NanoDrop One, Thermo Fisher Scientific). RNA isolates were then analysed on a 1.0% agarose gel, stained with Midori Green Advance DNA Stain (MG04, Nippon Genetics), according to the manufacturer's recommendations. Perfect Plus 1 kb DNA Ladder [E3131, EURx] was used for DNA length estimation at 500 ng per well. Visualization of electropherograms was performed using the GelDoc Go Imaging System (Bio-Rad).

**Storing of RNA isolates**

RNA samples were stored at -80 °C until further analyse.

**Primers design**

The primers for qPCR targeting the anti-BCMA and anti-GPRC5D gene were designed based on the scFv region of each, using the known sequence using Primer BLAST®. Primers with the following sequences were designed and used in the experiment: B157_F ACATTCTACGCCGACTCCGT, B157_R CCCAGTAATCAAAGGTGCCC for anti-BCMA detection and G138_F GCGGCATCCCTGATAGAT, G138_R CAAACACCACAGGAGGATTG for anti-GPRC5D copy number estimation. All the primers used were synthesized by Genomed S.A., delivered as a lyophilizate, and then reconstituted and appropriately diluted using sterile, nuclease-free water. To quantify the levels of Dual CAR-T in cell culture, real-time qPCR was optimized using the sequence of EF1-$\alpha$ as a control for transduced cell numbers. For this purpose, universal primers with the following sequences were used: EF1_F CAAGCCTCAGACAGTGGTTC and EF1_R CAAGGCGGTCACTGGTAAGG. These resulted in an amplification product of 91 bp.

**Preparation of DNA standards, creating the standard curves**

A standard curve in which a gene of interest was present at $10^1$-$10^9$ copies (10-fold dilution series) was prepared. The template for the reaction was suitably diluted plasmid DNA (pCDCAR1-LX-R100421-1C-1 and pCDCAR1-LX-R100421-1C-2), which was amplified by real-time PCR (CFX96 Dx, Bio-Rad) with primers B157, G138 and EF1, separately, using the reagents described below. All reactions were performed in triplicate.

**Reverse transcription**

RNA samples were diluted to a final concentration of 200 ng/$\mu$l in nuclease-free H$_2$O. The final volume per qPCR reaction (smART First Strand cDNA Synthesis Kit, E0804-01, EurX) was 20 $\mu$l containing 1x cDNA buffer, 2.5 $\mu$M oligo-dT primers, 500 $\mu$M dNTPS, 10 mM DTT, 1.25 U/$\mu$l RNase inhibitor, 10 U/$\mu$l of smART reverse transcriptase and 400 ng RNA. Non-template control (NTC) was included within all experiments. Samples were incubated as follows: 45 min. at 50°C and 85°C for 5 min. to terminate the reaction.

**Single plex Real-Time PCR**

The real-time PCR reactions, for each of the primer pairs used, were optimized in terms of the annealing temperature of the primers and their concentration, as well as by selecting the optimal concentration of cDNA. On the basis of the developed reaction conditions, amplification was carried out on the plasmid DNA template (appropriate to the primer pair used) in the range of $10^1$- $10^9$ copies per reaction in order to create standard curves. Then, reactions were performed using cDNA as a template. Estimation of the Dual CAR T cells was performed using RNA isolated from the population of T lymphocytes transduced with two lentiviral vectors (anti-BCMA and anti-GPRC5D), additionally sorted on the basis of the surface presence of the anti-BCMA molecule, the presence of which indicated successful transduction with the anti-BCMA vector. Reactions were carried out using the GoTaq® qPCR Master Mix, 2X (A600A, Promega), 240 $\mu$M primers and 2 $\mu$l cDNA diluted 1:4. The cycling parameters were as follows: 2 min. at 95°C and 40 cycles: 15 sec. at 95°C and 1 min. at 59°C.

Based on the results of the cytometric analysis and the quantitative PCR reaction for T lymphocytes subjected to the lentiviral transduction process, the percentage share of individual cell populations was estimated. **Within the population of GFP-positive cells, surprisingly 92.49% were shown to be Dual CAR-T, 6.02% mono CAR-T BCMA and 1.49% mono CAR-T GPRC5D.**

**Multiplex Real-Time PCR**

To perform simultaneous detection of both transgenes (anti-BCMA and anti-GPRC5D) during a single reaction, thus maintaining identical amplification conditions for both sequences, the multiplex real-time PCR technique was determined. Specific probes were therefore designed that would hybridize to the cDNA within the amplified region using the previously described primer pairs - B157 and G138. The probe was designed using PrimerQuest Tool (Integrated DNA Technologies, https://www.idtdna.com/pages/tools/primerquest). The sequence of the primers and their respective probes is shown in Table 26.

Probe B 157_sonda was synthetized at Genomed S.A. and B 138_sonda by Metabion. Both were delivered in a lyophilized state. To create a stock solution, the probes have been reconstituted in sterile nuclease-free water.

Table 26 Characteristics of primers and probes used for Dual CAR T detection in the multiplex real-time PCR

| Primers | Sequence | Length [bp] | Product length [bp] |
|---|---|---|---|
| B157_F | ACATTCTACGCCGACTCCGT | 20 | 157 |
| B157_R | CCCAGTAATCAAAGGTGCCC | 20 | |
| G138_F | GCGGCATCCCTGATAGAT | 18 | 138 |
| G138_R | CAAACACCACAGGAGGATTG | 20 | |

| Probes | Sequence | Length [bp] | |
|---|---|---|---|
| B157_sonda | FAM-TTGTCCCTGCTGATTGTGAACCTGC-TAMRA | 25 | - |
| G138_sonda | ROX-ACGAGGCCGATTACTACTGCAATTCC-TAMRA | 26 | - |

The possibility of mutual secondary structure formation between primers and/or probes was also assessed with the use of Oligo Analyzer (Integrated DNA Technologies, https://www.idtdna.com/calc/analyzer). The Maximum Delta G value refers to the free energy of the oligo sequence binding to its perfect complement. The corresponding results are shown in Table 27.

Table 27 Evaluation of the possibility of creating secondary structures between primers and/or probes.

| Maximum Delta G [kcal/mol] | | | | | | |
|---|---|---|---|---|---|---|
| | B157_F | B157_R | B157 sonda | G138_F | G138_R | G138 sonda |
| B157_F | -4,95 | - | - | - | - | - |
| B157_R | -3,42 | -3,14 | - | - | - | - |
| B157 sonda | -3,52 | -6,95 | -3,14 | - | - | - |
| G138_F | -9,82 | -8,16 | -5,09 | -3,61 | - | - |
| G138_R | -6,24 | -6,37 | -6,62 | -6,62 | -3,9 | - |
| G138 sonda | -6,21 | -8,9 | -5,37 | -9,82 | -5,37 | -9,28 |

Several aspects of the multiplex TaqMan Real-Time PCR were optimized to achieve low threshold cycle (Ct) values and high fluorescent signals, including primer concentrations, primers and probe ratios, annealing temperature and DNA concentration. Real-time PCR was performed using an CFX96 Dx Real-Time PCR Detection System (Bio-Rad). Multiplex PCR was performed using PCR Mix Probe (2008-200P, A&A Biotechnology) kit, according to the manufacturer's instructions (100 nM of each primer pair). The amplified template was cDNA, synthesized as described above. The TaqMan Real-Time PCR conditions were as follows: 1 cycle at 95°C for 2 min. followed by 40 cycles at 95°C for 15 and 58°C for 1 min.

**EXAMPLE 12**

**ESTABLISHMENT OF THE *IN VITRO* EFFICACY OF THE ANTI-BCMA ANTI-GPRC5D CAR-T CELLS**

**MULTIPLE MYELOMA CELL LINE WITH MONO CAR-T AND DUAL CAR-T COCULTURE**

In order to establish the *in vitro* efficacy of the anti-BCMA anti-GPRC5D CAR-T cells an experiment was performed in which generated CAR-T cells were cultured together with the H929 multiple myeloma cell line. The assumption of the experiment was to check the cellular response of CAR-T lymphocytes to H929 myeloma cells (controls were non-transduced lymphocytes and lymphocytes cloned with a GFP-only vector). Parameters such as % inhibition, cytotoxicity index were determined and the release profile of cytokines such as INF-y, TNFα, IL-10, IL-5, IL-4, IL-2 by cells was determined.

The used NCI-H929 tumor cell line was commercially purchased from ATCC (ref. number ATCC CRL-9068). The cells show a suspension type of growth, their doubling time is 70-80 hours, the diameter of the cells is about 18 μm. Optimal conditions for the growth of H929 cells are the use of culture medium RPMI-1640 (ATCC cat. number: 30-2001) with the addition of 20% fetal bovine serum (Biological Industries), 2-mercaptoethanol (Gibco cat. number: 21985023) at a concentration of 0.05 mM and interleukin 6 (Miltenyi Biotec, cat. number: 130-093-929). Cell culture was carried out without the addition of any antibiotics.

## I. Preparation of RPMI-1640 culture medium with 20% serum and 2-mercaptoethanol

1. From the bottle containing 500 ml of RPMI-1640 medium, 100 ml of medium were removed.
2. 100 ml of FBS were added to the bottle containing the medium.
3. The complex medium were filtered using a 0.22 μm filter and a syringe into a sterile falcon, labeled and stored at 4°C.
4. The dilution of 2-mercaptoethanol were prepared from initial concentration 55 mM to final concentration 0.05 mM (50 μM).

## II. H929 cells culture supplements

- fetal bovine serum concentration: 20%
- IL-6 concentration: 50 ng/ml
- 2-mercaptoethanol concentration: 0.05 mM

## III. Cell thawing protocol

1. The cryotube from liquid nitrogen vapor was thawing in a water bath (37°C) (approx. 1-2 min) and added to 9 ml of the medium heated to 37°C.
2. Cells were centrifuged at 300 RCF for 5 min.
3. The supernatant were discarded with a serological pipette.
4. Cells were suspended in 1 ml of medium.
5. 20 μl of the obtained suspension were counted with Trypan Blue 1:1 using the DeNovix Cell Drop device. Three measurements were taken.
6. The cells were cultured in a TPP cell culture or in a T25 culture vessel for the suspension cells culture at a density of $0.6 \times 10^6$ cells per 1 ml in the incubator with the orbital shaking, speed 80 RPM.

## IV. Medium exchange protocol

1. Every 48 hours cells were analyzed using the FLoid™ Cell microscope and count using the DeNovix device.
2. The cells were centrifuged at 300 RCF for 5 min.
3. The supernatant were removed with a serological pipette without disturbing the pellet.
4. The appropriate amount of medium and interleukins were added to cells.
5. The cell culture were placed in the incubator with the shaker, speed 80 RPM.

## V. Cell freezing protocol

1. The number of cells were counted using the DeNovix device. 20 μl of the homogeneous cell suspension mixed with 20 μl of Trypan Blue, three measurements were taken.
2. The cells were centrifuged at 300 RCF for 5 min and the supernatant were removed with a serological pipette.
3. The appropriate amount of Freeze Me One freezing medium were added to cells ($2-5 \times 10^6$ cells mixed with 1 ml of medium) in the cryotube.

4. The cryotubes were transferred to a freezing container filled with isopropanol (replace isopropanol every 5th freezing) and placed for 5h at -80°C. After this time, the cells were transferred to the liquid nitrogen vapor (-196°C).

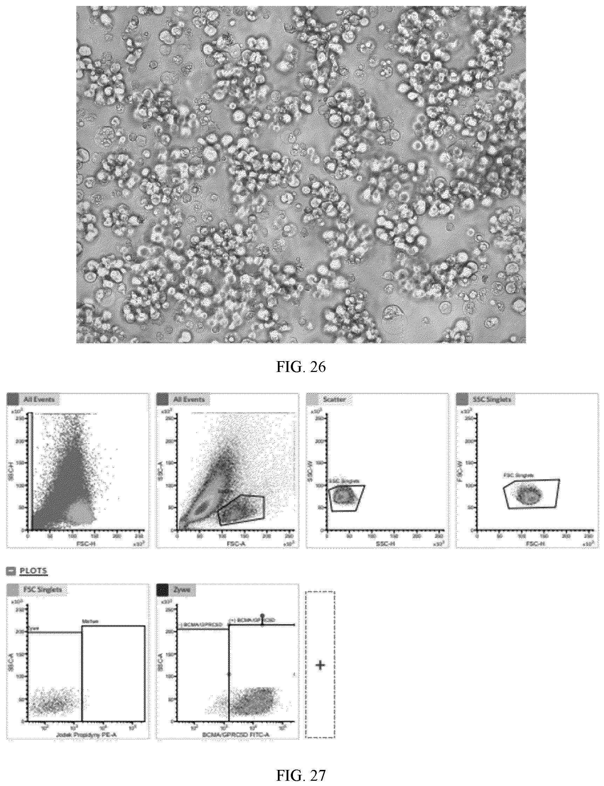

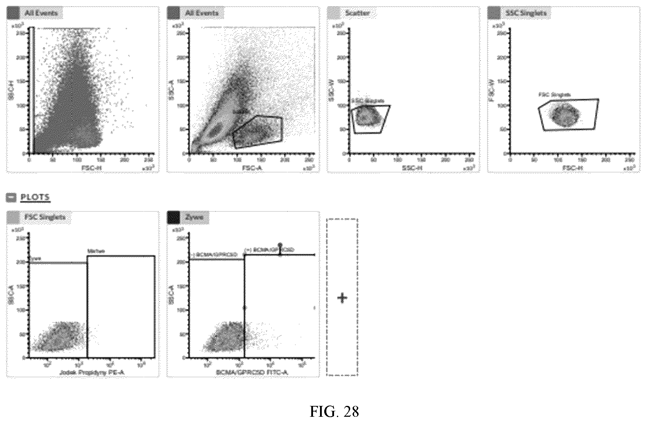

The H929 cell culture is presented on Fig. 26. Additionally, the immunophenotype of H929 cells was determined by flow cytometry to determine the level of expression of BCMA and GPRC5D antigens on the cell's surfaces (Figures 27-28). The presence of the BCMA antigen were detected on more than 97% of the cells and the GPRC5D antigen were detected on more than 3% of the cells.

The study of the cytokine release profile in the co-culture of T lymphocytes (CAR-T) and the H929 myeloma line was performed on the basis of the CBA test (Cytometric Bead Array). Image of CBA result (BD FACS Melody, Horus Software).

## EXAMPLE 13

### DUAL CAR-T AND GFP CELLS WITH MULTIPLE MYELOMA CELLS CO-CULTURE

**Establishment of the therapeutic efficacy of the dual CAR-T system with respect to the control GFP T-cells**

Medium: RPMI 1640 + 20% FBS without cytokines

Conditions with all parameters were described below (the given numbers correspond to the respective panels in Tables 29-30):

1. Non-transduced T cells $1 \times 10^6$ cells/ml (negative control)
2. Dual CAR-T $1 \times 10^6$ cells/ml (54% GFP+)
3. Mono Anti-BCMA CAR-T $1 \times 10^6$ cells/ml (78.5% GFP+)
4. Non-transduced T cells $1 \times 10^6$ cells/ml (negative control)
5. GFP CAR-T $1 \times 10^6$ cells/ml (35% GFP+)
6. H929 $1 \times 10^6$ cells/ml

Table 28. Conditions of CAR-T cells and H929 co-culture

| 1. Dual CAR-T + H929 | | | | |
|---|---|---|---|---|
| Lp. | Effector cells | Target cells | Volume | The number of repetitions |
| 7. | $1 \times 10^6$ cells Dual CAR-T 1852 $\mu$l = $1 \times 10^6$ cells | $2 \times 10^6$ | 3.8 ml | Phenotype after 24 and 48 h. |
| 8. | $1 \times 10^6$ cells Dual CAR-T 1852 $\mu$l = $1 \times 10^6$ cells | $5 \times 10^6$ | 6.8 ml | Phenotype after 24 and 48 h. |
| 9. | $1 \times 10^6$ cells CAR-T DUAL 1852 $\mu$l = $1 \times 10^6$ cells | $10 \times 10^6$ | 11.8 ml | Phenotype after 24 and 48 h. |
| 2. Anti-BCMA mono CAR-T + H929 | | | | |
| Lp. | Effector cells | Target cells | Volume | The number of repetitions |
| 10. | $1 \times 10^6$ Mono CAR-T Anti-BCMA 1274 $\mu$l = $1 \times 10^6$ cells | $2 \times 10^6$ | 3.2 ml | Phenotype after 24 and 48 h |
| 11. | $1 \times 10^6$ Mono CAR-T Anti-BCMA 1274 $\mu$l = $1 \times 10^6$ cells | $5 \times 10^6$ | 6.2 ml | Phenotype after 24 and 48 h |
| 12. | $1 \times 10^6$ Mono CAR-T Anti-BCMA 1274 $\mu$l = $1 \times 10^6$ cells | $10 \times 10^6$ | 11.2 ml | Phenotype after 24 and 48 h |

(continued)

| Lp. | Effector cells | Target cells | Volume | The number of repetitions |
|---|---|---|---|---|
| 13. | $1 \times 10^6$ non transduced cells 1000 $\mu l = 1 \times 10^6$ cells | $2 \times 10^6$ | 3 ml | Phenotype after 24 and 48 h |
| 14. | $1 \times 10^6$ non transduced cells 1000 $\mu l = 1 \times 10^6$ cells | $5 \times 10^6$ | 6 ml | Phenotype after 24 and 48 h |
| 15. | $1 \times 10^6$ non transduced cells 1000 $\mu l = 1 \times 10^6$ cells | $10 \times 10^6$ | 11 ml | Phenotype after 24 and 48 h |

| 3. GFP T- cells + H929 | | | | |
|---|---|---|---|---|
| **Lp.** | **Effector cells** | **Target cells** | **Volume** | **The number of repetitions** |
| 16. | $1 \times 10^6$ cells GFP T-cells 2857 $\mu l = 1 \times 10^6$ cells | $2 \times 10^6$ | 4.8 ml | Phenotype after 24 and 48 h |
| 17. | $1 \times 10^6$ cells GFP T-cells 2857 $\mu l = 1 \times 10^6$ cells | $5 \times 10^6$ | 7.8 ml | Phenotype after 24 and 48 h |
| 18. | $1 \times 10^6$ cells GFP T-cells 2857 $\mu l = 1 \times 10^6$ cells | $10 \times 10^6$ | 12.8 ml | Phenotype after 24 and 48 h |

| **Lp.** | **Effector cells** | **Target cells** | **Volume** | **The number of repetitions** |
|---|---|---|---|---|
| 19. | $1 \times 10^6$ non transduced cells 1000 $\mu l = 1 \times 10^6$ cells | $2 \times 10^6$ | 3 ml | Phenotype after 24 and 48 h |
| 20. | $1 \times 10^6$ non transduced cells 1000 $\mu l = 1 \times 10^6$ cells | $5 \times 10^6$ | 6 ml | Phenotype after 24 and 48 h |
| 21. | $1 \times 10^6$ non transduced cells 1000 $\mu l = 1 \times 10^6$ cells | $10 \times 10^6$ | 11 ml | Phenotype after 24 and 48 h |

Then the following parameters were determined.

The inhibition percentage (% inhibitions) was determined by the following formula:

$$100 - \left( \frac{\text{Target exp.}}{\text{Target alone}} + 100 \right)$$

It was checked after 24 and 48 hours and indicates the percentage of cancer cell eliminated by CAR-T Cells directed against specific proteins found on the surface of cancer cells.

The elimination index was determined by the following formula:

$$1 - \left( \frac{\text{Residual Target Cells} + \text{CAR} - \text{T}}{\text{Residual Target Cells} + \text{Control (Untransduced T Cells)}} \right)$$

and was analysed after 48 hours.

The cytotoxicity index was determined by the following formula:

$$\frac{\text{Tumor Cell Number CAR} - \text{T treated}}{\text{Tumor Cell Number no CAR} - \text{T treated}}$$

and was checked after 24 and 48 hours.

**COCULTURE AFTER 24 HOURS**

**Establishment of the efficacy of the dual CAR-T system against multiple myeloma cells *in vitro* (mono anti-BCMA cells and GFP T-cells were used as additional controls)**

Medium: RPMI 1640 + 20% FBS
Cells were pipetted. $2 \times 20$ µl were taken and counted.

Table 29. Average cell number and viability (deNovix), PD-1 analysed in 6 samples.

| Condition | Number of cells | Viability [%] | PD-1 |
|---|---|---|---|
| 1. | $0.88 \times 10^6 \times 3$ ml = $2.64 \times 10^6$ | 81.6 % | - |
| 2. | $0.844 \times 10^6 \times 2.5$ ml = $2.1 \times 10^6$ | 82.9 % | - |
| 3. | $0.885 \times 10^6 \times 4$ ml = $3.54 \times 10^6$ | 82.8 % | - |
| 4. | $0.89 \times 10^6 \times 4$ ml = $3.54 \times 10^6$ | 90.4 % | - |
| 5. | $0.151 \times 10^6 \times 1$ ml = $0.151 \times 10^6$ | 80.7 % | - |
| 6. | $1.695 \times 10^6 \times 5$ ml = $8.48 \times 10^6$ | 84% | - |
| 7. | $0.88 \times 10^6 \times 3.8$ ml = $3.34 \times 10^6$ | 76.6 % | 23.63% |
| 8. | $0.66 \times 10^6 \times 6.8$ ml = $4.45 \times 10^6$ | 79.8 % | 39.53% |
| 9. | $1.07 \times 10^6 \times 11.8$ ml = $12.63 \times 10^6$ | 75.7 % | - |
| 10. | $0.597 \times 10^6 \times 3.2$ ml = $1.9 \times 10^6$ | 60.9 % | 19.85% |
| 11. | $1.06 \times 10^6 \times 6.2$ ml = $6.57 \times 10^6$ | 80.3 % | 19.28% |
| 12. | $0.83 \times 10^6 \times 11.2$ ml = $9.33 \times 10^6$ | 77.9% | - |
| 13. | $1.3 \times 10^6 \times 3$ ml = $3.9 \times 10^6$ | 81.2 % | - |
| 14. | $1.134 \times 10^6 \times 6$ ml = $6.8 \times 10^6$ | 79.95 % | - |
| 15. | $1.22 \times 10^6 \times 11$ ml = $13.4 \times 10^6$ | 83.9 % | - |
| 16. | $1.03 \times 10^6 \times 4.8$ ml = $4.9 \times 10^6$ | 79.3 % | 2.41% |
| 17. | $0.29 \times 10^6 \times 7.8$ ml = $2.26 \times 10^6$ | 74.6 % | 3.31% |
| 18. | $0.886 \times 10^6 \times 12.8$ ml = $11.35 \times 10^6$ | 75.5 % | - |
| 19. | $0.943 \times 10^6 \times 3$ ml = $2.83 \times 10^6$ | 82.8 % | - |
| 20. | $1.38 \times 10^6 \times 6$ ml = $8.25 \times 10^6$ | 81.5 % | - |
| 21. | $0.189 \times 10^6 \times 11$ ml = $2.08 \times 10^6$ | 59.1 % | - |

$1.5 \times 10^6$ or $1 \times 10^6$ cells were harvested for phenotypic analysis.
The cells were centrifuged 7 min. 250 RCF.

**COCULTURE AFTER 48 HOURS**

**Establishment of the efficacy of the dual CAR-T system against multiple myeloma cells *in vitro* after 48 hours**

Medium: RPMI 1640 + 20% FBS
Cells were pipetted. $2 \times 20$ µl were taken and counted.

Table 30. Average cell number and viability (deNovix), PD-1 analysed in 8.

| Condition | Number of cells | Viability [%] | PD-1 |
|---|---|---|---|
| 1. | $0.825 \cdot 10^6 \times 1.8ml = 1.49 \times 10^6$ | 86.1 % | - |
| 2. | $0.664 \times 10^6 \times 1.3ml = 0.86 \times 10^6$ | 80.8 % | 11.97% |
| 3. | $0.92 \times 10^6 \times 2.3ml = 2.12 \times 10^6$ | 86.2 % | 7.03% |
| 4. | $0.87 \times 10^6 \times 2.3ml = 2 \times 10^6$ | 90.1 % | - |
| 5. | $0.04 \times 10^6 \times 0.5ml = 0.02 \times 10^6$ | 55.1 % | - |
| 6. | $1.94 \times 10^6 \times 4.1ml = 7.95 \times 10^6$ | 84.9 % | - |
| 7. | $0.348 \times 10^6 \times 2.6ml = 0.997 \times 10^6$ | 51.8 % | 13.51% |
| 8. | $0.748 \times 10^6 \times 4.5ml = 3.37 \times 10^6$ | 63.6 % | 16.25% |
| 9. | $0.91 \times 10^6 \times 10.3ml = 9.4 \times 10^6$ | 71.2 % | - |
| 10. | $0.43 \times 10^6 \times 1.5ml = 0.65 \times 10^6$ | 48.4 % | 15.51% |
| 11. | $0.744 \times 10^6 \times 4.7ml = 3.49 \times 10^6$ | 59.9 % | 21.6% |
| 12. | $0.98 \times 10^6 \times 9.3ml = 9.1 \times 10^6$ | 71.1 % | - |
| 13. | $1.77 \times 10^6 \times 2.2ml = 3.88 \times 10^6$ | 82.1 % | - |
| 14. | $1.19 \times 10^6 \times 4.6ml = 5.47 \times 10^6$ | 76.3 % | - |
| 15. | $1.42 \times 10^6 \times 9.7ml = 13.73 \times 10^6$ | 76.8 % | - |
| 16. | $1.06 \times 10^6 \times 3.8ml = 4 \times 10^6$ | 73.6 % | 5.22% |
| 17. | $0.345 \times 10^6 \times 4.3ml = 1.48 \times 10^6$ | 68.3 % | 5.25% |
| 18. | $0.868 \times 10^6 \times 11.4ml = 9.89 \times 10^6$ | 66.7 % | - |
| 19. | $1.48 \times 10^6 \times 1.9ml = 2.8 \times 10^6$ | 81.8 % | - |
| 20. | $1.49 \times 10^6 \times 4.9ml = 7.3 \times 10^6$ | 76.1 % | - |
| 21. | $1.02 \times 10^6 \times 7.4ml = 7.57 \times 10^6$ | 69.6 % | - |

$1.5 \times 10^6$ or $1 \times 10^6$ cells were harvested for phenotypic analysis.

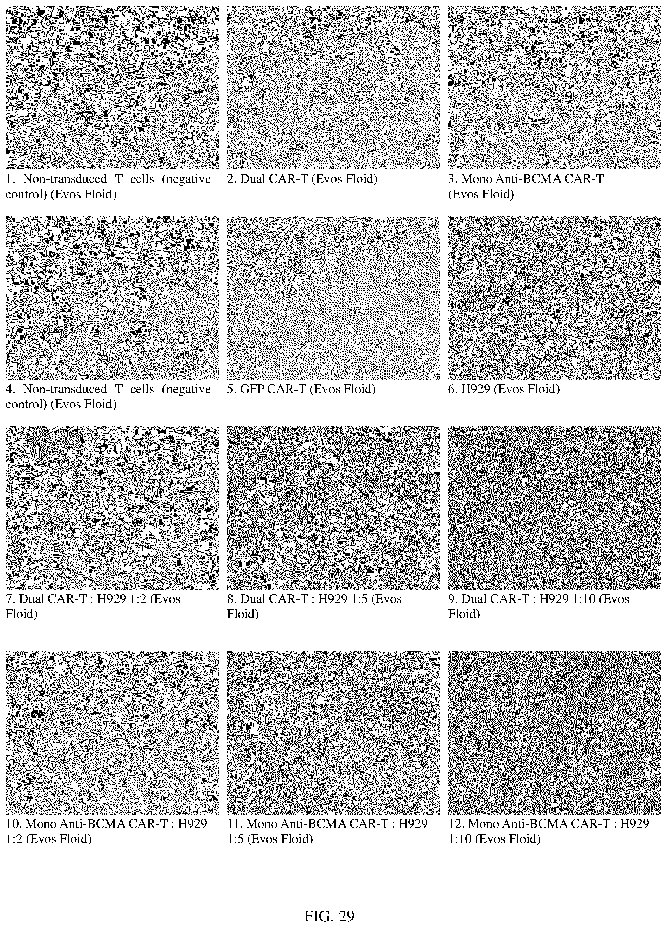

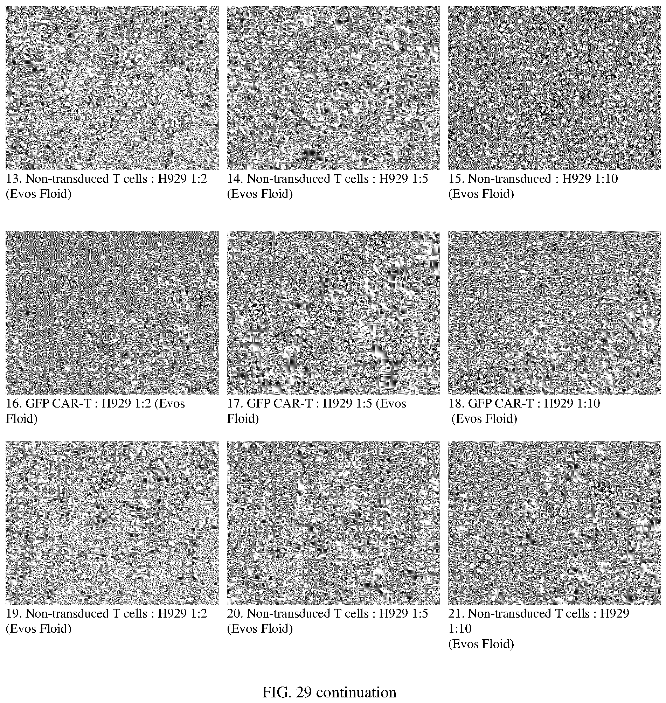

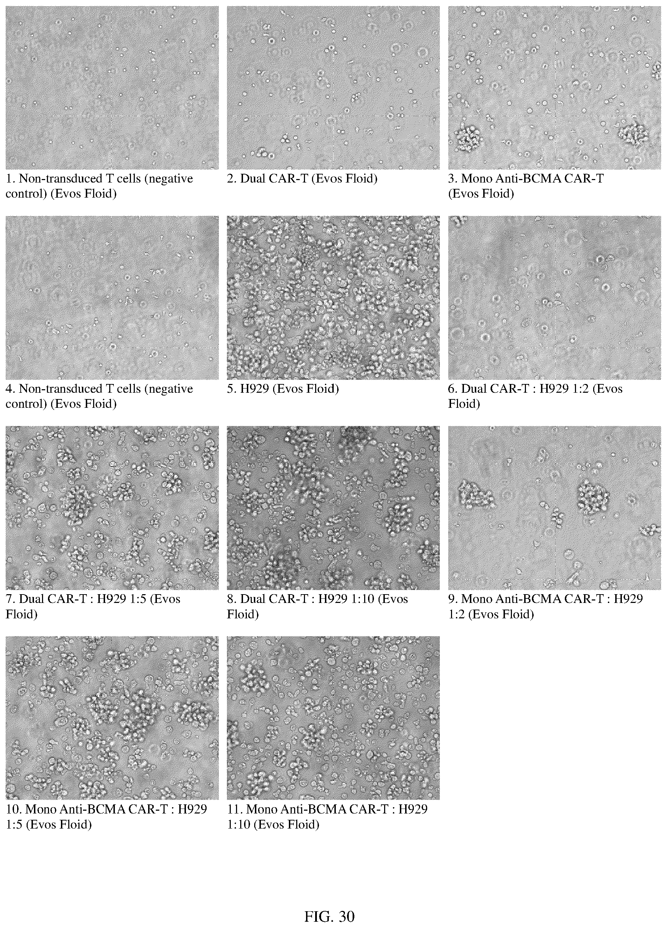

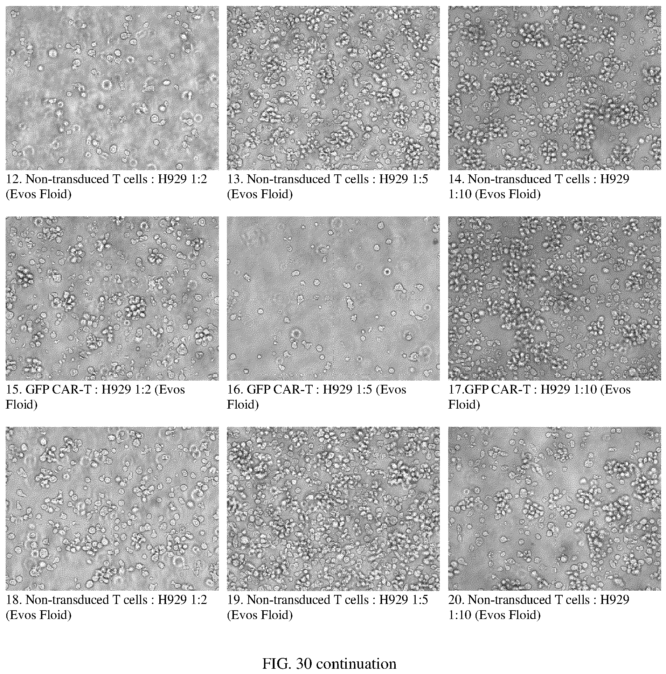

The cells were centrifuged 7 min. 250 RCF. Cells in co-culture and all control cells (1-21) after 24 hours of culture are presented on Figure 29-30.

Table 31. Inhibition, elimination index and cytotoxicity index of Dual CAR-T, Mono Anti-BCMA CAR-T and GFP CART-T cells in co-culture with Multiple Myeloma H929 cells compared to control cells (tables below).

| Dual CAR-T | | | |
|---|---|---|---|
| % inhibition | | | |
| H929 | | 24h | 48h |
| Effector cells | Target cells | % inhibition | % inhibition |
| 1 mln dual CAR-T | 2mln | 73.83 | 94.12 |
| 1 mln dual CAR-T | 5mln | 49.60 | 76.14 |
| 1 mln dual CAR-T | 10mln | 28.28 | 46.39 |
| | | | |
| H929 | Elimination index | | |
| Effector cells | Target cells | 24h | 48h |
| 1 mln dual CAR-T | 2mln | 63.05% | 93.67% |
| 1 mln dual CAR-T | 5mln | 28.83% | 73.95% |

(continued)

| H929 | Elimination index | | |
|---|---|---|---|
| Effector cells | Target cells | 24h | 48h |
| 1 mln dual CAR-T | 10mln | -1.28% | 41.47% |
| | | | |
| Cytotoxicity index | | | |
| H929 | | 24h | 48h |
| Effector cells | Target cells | Cytotoxicity index | Cytotoxicity index |
| 1 mln dual CAR-T | 2mln | 26.17% | 5.80% |
| 1 mln dual CAR-T | 5mln | 50.40% | 23.86% |
| 1 mln dual CAR-T | 10mln | 71.72% | 53.61% |
| | | | |
| Mono Anti-BCMA CAR-T | | | |
| % inhibition | | | |
| H929 | | 24h | 48h |
| Effector cells | Target cells | % inhibitions | % inhibitions |
| 1 mln Anti-BCMACAR-T | 2mln | 74.02 | 93.96 |
| 1 mln Anti-BCMACAR-T | 5mln | 41.91 | 73.27 |
| 1 mln Anti-BCMACAR-T | 10mln | 38.85 | 51.45 |
| H929 | Elimination index | | |
| Effector cells | Target cells | 24h | 48h |
| 1 mln Anti-BCMACAR-T | 2mln | 63.32% | 93.41% |
| 1 mln Anti-BCMACAR-T | 5mln | 17.97% | 70.81% |
| 1 mln Anti-BCMACAR-T | 10mln | 13.65% | 47.00% |
| | | | |
| Cytotoxicity index | | | |
| H929 | | 24h | 48h |
| Effector cells | Target cells | Cytotoxicity index | Cytotoxicity index |
| 1 mln Anti-BCMACAR-T | 2mln | 25.98% | 6.04% |
| 1 mln Anti-BCMACAR-T | 5mln | 58.09% | 26.73% |
| 1 mln Anti-BCMACAR-T | 10mln | 61.15% | 48.55% |
| | | | |
| Control | | | |
| % inhibition | | | |
| H929 | | 24h | 48h |
| Effector cells | Target cells | % inhibitions | % inhibitions |
| 1 mln non transduced control | 2mln | 29.18 | 8.41 |
| 1 mln non transduced control | 5mln | 22.42 | 25.28 |
| 1 mln non transduced control | 10mln | 28.06 | 21.72 |
| | | | |

(continued)

| H929 | Elimination index | | |
|---|---|---|---|
| Effector cells | Target cells | 24h | 48h |
| 1 mln non transduced control | 2mln | 0.00% | 0.00% |
| 1 mln non transduced control | 5mln | -9.55% | 18.42% |
| 1 mln non transduced control | 10mln | -1.59% | 14.53% |
| | | | |
| Cytotoxicity index | | | |
| H929 | | 24h | 48h |
| Effector cells | Target cells | Cytotoxicity index | Cytotoxicity index |
| 1 mln non transduced control | 2mln | 70.82% | 91.59% |
| 1 mln non transduced control | 5mln | 77.58% | 74.72% |
| 1 mln non transduced control | 10mln | 71.94% | 78.28% |
| GFP T-cells | | | |
| % inhibition | | | |
| H929 | | 24h | 48h |
| Effector cells | Target cells | % inhibitions | % inhibitions |
| 1 mln GFPT-cells | 2mln | 64.39 | 64.42 |
| 1 mln GFPT-cells | 5mln | 49.48 | 58.45 |
| 1 mln GFPT-cells | 10mln | 46.54 | 53.16 |
| | | | |
| H929 | Elimination | index | |
| Effector cells | Target cells | 24h | 48h |
| 1 mln GFPT-cells | 2mln | 41.96% | 51.93% |
| 1 mln GFPT-cells | 5mln | 17.66% | 44.11% |
| 1 mln GFPT-cells | 10mln | 12.86% | 36.99% |
| | | | |
| Cytotoxicity index | | | |
| H929 | | 24h | 48h |
| Effector cells | Target cells | Cytotoxicity index | Cytotoxicity index |
| 1 mln GFPT-cells | 2mln | 35.61% | 35.73% |
| 1 mln GFPT-cells | 5mln | 50.52% | 41.55% |
| 1 mln GFPT-cells | 10mln | 53.46% | 46.84% |
| | | | |
| Control | | | |
| % inhibition | | | |
| H929 | | 24h | 48h |
| Effector cells | Target cells | % inhibitions | % inhibitions |
| 1 mln non transduced control | 2mln | 38.64 | 25.66 |
| 1 mln non transduced control | 5mln | 23.71 | 26.06 |

(continued)

| Control | | | |
|---|---|---|---|
| % inhibition | | | |
| H929 | | 24h | 48h |
| 1 mln non transduced control | 10mln | 31.67 | 42.87 |
| | | | |
| H929 | Elimination index | | |
| Effector cells | Target cells | 24h | 48h |
| 1 mln non transduced control | 2mln | 0.00% | 0.00% |
| 1 mln non transduced control | 5mln | -24.34% | 0.54% |
| 1 mln non transduced control | 10mln | -11.36% | 23.15% |
| | | | |
| Cytotoxicity index | | | |
| H929 | | 24h | 48h |
| Effector cells | Target cells | Cytotoxicity index | Cytotoxicity index |
| 1 mln non transduced control | 2mln | 61.36% | 74.34% |
| 1 mln non transduced control | 5mln | 76.29% | 73.94% |
| 1 mln non transduced control | 10mln | 68.33% | 57.13% |

To summaries, the experimental results presented above unequivocally indicate the effectiveness of CAR-T cells *in vitro.*

The analyzed parameters of inhibition percentage, elimination index showed a significant increase in the case of CAR-T cell co-cultures (mono anti-BCMA, dual CAR-T) and the H929 myeloma line in relation to the co-culture containing control cells (non-transduced). It is worth noting that the studied parameters of inhibition and elimination of H929 cells by said CAR-T cells significantly increased with the time of coculture digestion. In the case of non-transduced (control) cells, this trend was not observed. Transduced GFP cells also inhibited the proliferation and elimination of H929 cells, but this value did not increase over time. The tests carried out clearly indicate the superiority of said CAR-T cells in inhibiting the proliferation and elimination of H929 myeloma cells and in the cytotoxic effect on cancer.

Analyzing the level of expression of the PD-1 (maximum level on dual CAR-T 1:5, see Table 28-30) antigen on the cell surface, which is an indicator of the appearance of exhausted cells (i.e., those cells that despite the specific connection with the cancer cell, lose their cytotoxic properties towards cancer cells), it was noticed that it appeared on the surface of both mono anti-BCMA CAR-T and dual CAR-T cells. However, there was a significant decrease in the expression of PD-1 on dual CAR-T cells compared to mono anti-BCMA CAR-T on the second day of the experiment (after 48h, see Table 30), which, without being bound by any theory, decreases exhaustion and thus increases efficiency of dual CAR-T. Moreover, increased dual CAR-T efficiency, effectiveness, and reduction of antigen escape were furthermore obtained by introducing additional anti-GPRC5D surface receptor and specific domains in third-generation CARs according to the present invention, namely in dual CAR-T system comprising the first and the second CAR encoded by a nucleic acid sequence having the following structure - anti-GPRC5D scFv (ET150-8) h (ICOS-4-1BB-CD3ζ) and anti-BCMA scFv (25D2) h (CD28-OX40-CD3ζ).

In particular, the PD-1 surface antigen assay was performed on mono (BCMA) CAR-T and dual (BCMA, GPRC5D) CAR-T cells exposed to the multiple myeloma cell line (H929). During the co-culture, a small percentage of CAR-T cells expressing the PD-1 antigen on their surface was observed, which proves that these cells have not lost their functionality in relation to the multiple myeloma cancer cells (H929) directed against them. Surprisingly, in the case of the dual CAR-T test condition, the percentage of cells expressing PD-1 was lower than in the mono CAR-T co-culture condition at 48h of the experiment. Obtained data suggest that the object of the present invention will obviate the need for use additional preparations in therapy aimed at blocking PD-1, which will significantly reduce the cost of therapy and improve the patient's comfort in the treatment process. The application of two receptors (BCMA and GPRC5D) into the CAR-T system additionally reduces the chance of antigenic escape. Moreover, the use of cells with both of said receptors with low expression of the PD-1 cell depletion provides more effective and faster complete healing of the patient and remission of the disease.

**EXAMPLE 14**

**CYTOKINES PROFILE**

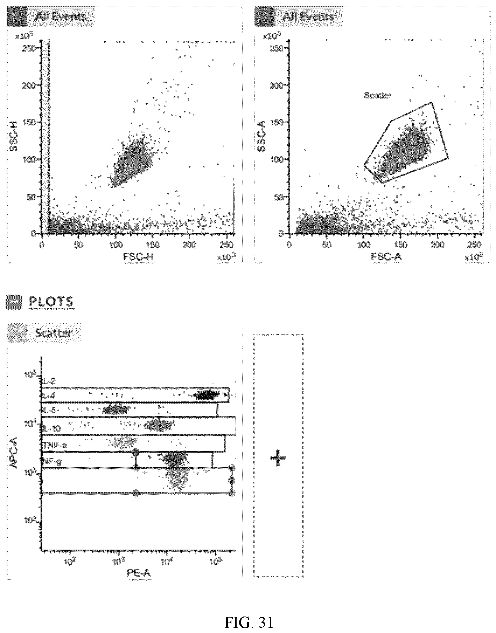

The profile of cytokines released by the cells present in the co-culture was analyzed after 24 h and 48 h (see Figure 31). In this experiment, the cytokine release profile was assessed for non-transduced T cells, CAR-T cells (50.28 % transduced) and H929 cells (negative controls).
The following test samples were tested in co-culture variants:

- non-transduced T cells + H929 myeloma cells in a 1:1 ratio
- CAR-T cells (50.28% transduced) + H929 myeloma cells in a 1:1 ratio
- CAR-T cells (50.28% transduced) + H929 myeloma cells in a 1:2 ratio
- CAR-T cells (50.28% transduced) + H929 myeloma cells in a 1:5 ratio

CBA test results were analyzed using CBA Analysis Software Version: 1.1.14

Table 32. Results for cytokine release CBA tests for INF-γ, TNF-α, IL-10, IL-5, IL-4 and IL-2 after 24h and 48h of co-culture with Dual CAR-T cells.

| Concentration [pg/ml] | INF-γ [pg/ml] | | TNF [pg/ml] | | IL-10 [pg/ml] | | IL-5 [pg/ml] | | IL-4 [pg/ml] | | IL-2 [pg/ml] | |
|---|---|---|---|---|---|---|---|---|---|---|---|---|
| | 24h | 48h | 24h | 48h | 24h | 48h | 24h | 48h | 24h | 48h | 24h | 48h |
| Non-transduced T cells | 16.9 | 12.7 | 1.3 | 3.2 | 2.4 | 2.4 | 5.6 | 5.2 | 0.0 | 1.2 | 0.0 | 0.0 |
| Dual CAR-T (50.28% transduced) | 319.2 | 329.8 | 16.0 | 20.8 | 37.9 | 26.5 | 49.3 | 51.2 | 0.0 | 1.8 | 0.0 | 0.0 |
| H929 | 0.0 | 1.7 | 0.0 | 2.0 | 30.7 | 62.9 | 0.0 | 1.1 | 0.0 | 1.8 | 0.0 | 1.4 |
| Non-transduced T cells + H929 1:1 | 3.9 | 3.0 | 1.6 | 2.2 | 25.5 | 58.0 | 1.4 | 1.3 | 0.0 | 0.0 | 0.0 | 1.2 |
| CAR-T cells (50.28% transduced) + H929 1:1 | 4015.9 | 10573.3 | 6127.6 | 3257.4 | 284.6 | 186.7 | 275.4 | 716.8 | 340.9 | 63.7 | 10659.4 | 10490.3 |
| CAR-T cells (50.28% transduced) + H929 1:2 | 4262.3 | 17797.0 | 10881.5 | 9775.7 | 298.9 | 204.9 | 181.7 | 629.5 | 626.4 | 200.7 | 12620.4 | 24358.2 |
| CAR-T cells (50.28% transduced) + H929 1:5 | 3408.6 | 10288.6 | 5200.3 | 6449.4 | 171.9 | 172.0 | 39.7 | 112.9 | 476.8 | 313.8 | 11131.0 | 23177.3 |

In another experiment, the level of cytokine release was tested in the case of T cells that were transfused with a vector containing only GFP (34% transducted). The profile of cytokines released by the cells present in the co-culture was analyzed after 24 h and 48 h. In this experiment, the cytokine release profile was assessed for non-transduced T cells, transduced GFP+ T cells and H929 cells (negative controls).

The following test samples were tested in co-culture variants:

- non-transducted T cells + H929 myeloma cells in a 1:1 ratio
- Transducted GFP + T cells (32% transducted) + H929 myeloma cells in a 1:1 ratio
- Transducted GFP + T cells (32% transducted) + H929 myeloma cells in a 1:2 ratio
- Transducted GFP + T cells (32% transducted) + H929 myeloma cells in a 1:5 ratio

Table 33. Results for cytokine release CBA tests for INF-$\gamma$, TNF-$\alpha$, IL-10, IL-5, IL-4 and IL-2 after 24h and 48h of co-culture with transduced GFP+ T cells.

Table 33. Results for cytokine release CBA tests for INF-γ, TNF-α, IL-10, IL-5, IL-4 and IL-2 after 24h and 48h of co-culture with transduced GFP+ T cells.

| Concentration [pg/ml] | INF-γ [pg/ml] | | TNF [pg/ml] | | IL-10 [pg/ml] | | IL-5 [pg/ml] | | IL-4 [pg/ml] | | IL-2 [pg/ml] | |
|---|---|---|---|---|---|---|---|---|---|---|---|---|
| | 24h | 48h | 24h | 48h | 24h | 48h | 24h | 48h | 24h | 48h | 24h | 48h |
| Non-transduced T cells | 4.65 | 4.74 | 2.17 | 2.20 | 2.08 | 1.49 | 1.44 | 1.46 | 1.20 | 1.65 | 1.46 | 1.36 |
| Transduced GFP(+) cells (32% transduced) | 26.94 | 22.66 | 3.05 | 2.97 | 2.81 | 2.34 | 15.36 | 13.94 | 1.79 | 2.77 | 1.51 | 2.02 |
| H929 | 1.57 | 1.54 | 2.52 | 1.94 | 14.91 | 27.19 | 1.47 | 1.23 | 1.98 | 2.12 | 1.94 | 1.57 |
| non-transduced T cells + H929 1:1 ratio | 20.29 | 13.34 | 2.83 | 2.76 | 7.22 | 9.14 | 1.78 | 1.50 | 1.66 | 2.01 | 2.43 | 1.53 |
| Transduced GFP(+) cells (32% transduced) + H929 1:1 | 22.63 | 13.60 | 1.85 | 2.77 | 5.15 | 5.84 | 13.31 | 10.52 | 0.00 | 1.51 | 1.15 | 1.39 |
| Transduced GFP(+) cells (32% transduced) + H929 1:2 | 54.59 | 13.13 | 2.65 | 2.27 | 7.72 | 12.42 | 30.92 | 8.93 | 0.00 | 1.88 | 0.00 | 1.59 |
| Transduced GFP(+) cells (32% transduced) + H929 1:5 | 55.65 | 7.11 | 2.80 | 4.01 | 10.85 | 16.51 | 35.25 | 5.71 | 0.00 | 3.13 | 0.00 | 2.66 |

In order to confirm the efficiency and demonstrate the surprising efficiency of dual CAR-T cells, an additional experiment was performed with co-culture of H929 cells with mono anti-BCMA CAR-T. This allowed for a comparative analysis of the effectiveness of dual CAR-T versus mono CAR-T. The profile of cytokines released by the cells present in the co-culture was analyzed after 24 h and 48 h. In this experiment, the cytokine release profile was assessed for transduced mono CAR-T cells (60 % transducted) and H929 cells (negative controls).

The following test samples were tested in co-culture variants:

- Transducted mono CAR-T cells (60 % transducted) + H929 myeloma cells in a 1:1 ratio
- Transducted mono CAR-T cells (60 % transducted) + H929 myeloma cells in a 1:2 ratio
- Transducted mono CAR-T cells (60 % transducted) + H929 myeloma cells in a 1:5 ratio

Table 34. Results for cytokine release CBA tests for INF-$\gamma$, TNF-$\alpha$, IL-10, IL-5, IL-4 and IL-2 after 24h and 48h of co-culture with transducted Mono Anti-BCMA CAR T cells.

Comparison of pro-inflammatory and anti-inflammatory cytokine secretion (concentration [pg/ml]) Mono Anti-

Table 34. Results for cytokine release CBA tests for INF-γ, TNF-α, IL-10, IL-5, IL-4 and IL-2 after 24h and 48h of co-culture with transducted Mono Anti-BCMA CAR T cells.

| Concentration [pg/ml] | INF-γ [pg/ml] | | TNF [pg/ml] | | IL-10 [pg/ml] | | IL-5 [pg/ml] | | IL-4 [pg/ml] | | IL-2 [pg/ml] | |
|---|---|---|---|---|---|---|---|---|---|---|---|---|
| | 24h | 48h | 24h | 48h | 24h | 48h | 24h | 48h | 24h | 48h | 24h | 48h |
| Transducted mono CAR-T cells (60% transducted) H929 cells | 96.39 0.00 | 95.42 0.00 | 128.49 0.00 | 215.89 0.00 | 0.00 74.19 | 0.00 365.56 | 227.30 0.00 | 292.63 0.00 | 0.00 1.64 | 1.55 0.00 | 0.00 0.00 | 2.80 0.00 |
| Transducted mono CAR-T cells (60% transducted) + H929 1:1 | 1549.92 | 2025.32 | 6635.59 | 7861.18 | 31.58 | 29.85 | 989.98 | 3836.91 | 204.58 | 175.08 | 7697.08 | 9717.08 |
| Transducted mono CAR-T cells (60% transducted) + H929 1:2 | 1713.35 | 2864.29 | 4765.24 | 6165.12 | 72.84 | 146.74 | 530.55 | 1467.08 | 171.68 | 158.29 | 8213.51 | 12099.01 |
| Transducted mono CAR-T cells (60% transducted) + H929 1:5 | 759.80 | 1799.64 | 1609.07 | 1799.64 | 77.38 | 228.49 | 182.38 | 562.91 | 82.28 | 93.37 | 4185.02 | 7313.55 |

EP 4 520 346 A1

62

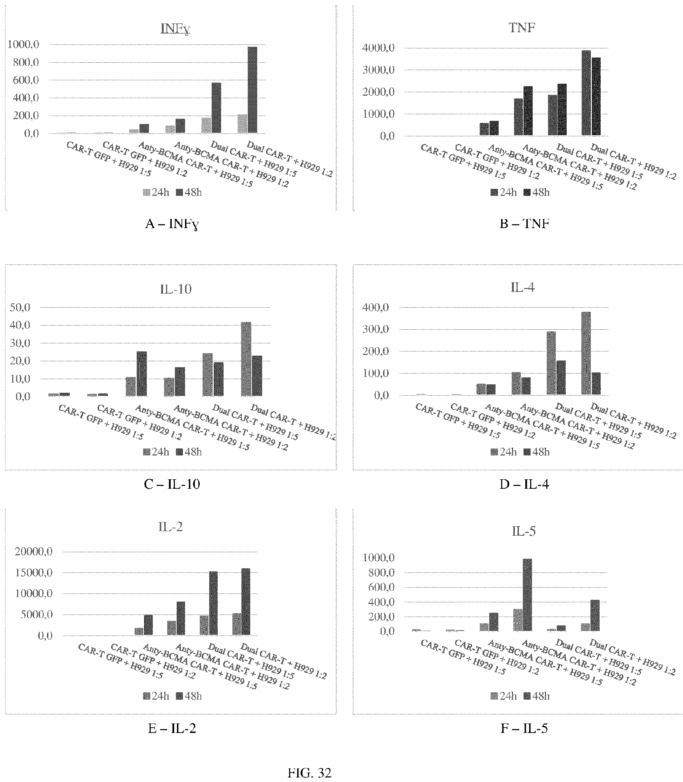

BCMA CAR-T and Dual CAR-T in relation to control GFP+ CAR-T in co-culture with B lymphocytes isolated from malignant exudate of a 62-year-old patient with myeloma plasma cell-line H929, NCI-H929, CRL-9068™ was presented on Figure 32.

Analyzing the profile of released cytokines in co-culture of CAR-T cells (mono anti-BCMA) a significant increase in INF-$\gamma$, TNF and IL-2 was noticed. However, a stronger cytokine storm was observed in the case of dual CAR-T cells, where the level of cytokine release is almost <u>twice as high</u> in the case of IL-2 and definitely noticeable in the case of INF-$\gamma$ and TNF. A greater amount of cytokine release in response to the appearance of tumor cells (multiple myeloma of the H929 cell line) indicates a more rapid cellular response, which have a positive impact in further clinical trials. In the case of the analysis of the cytokine release profile of non-transduced cells and cells transduced with the GFP lentivirus, a significant increase in the level of cytokine release was not observed, which proves the lack of cellular response to the myeloma line. Cell release of IL-10, IL5 and IL-4 was not significantly increased relative to control cells.

In summary, the presented results clearly demonstrate the *in vitro* unexpected efficacy of dual CAR-T cells against the multiple myeloma cells. The level of cytokine release by the cells closely correlates with the values of percent inhibition, elimination index and cytotoxicity for all tested samples.

## Claims

1. A T cell that co-expresses at least a first chimeric antigen receptor (CAR) and second CAR at the cell surface, each CAR comprising:

   (i) an extracellular antigen-binding domain;
   (ii) a spacer
   (iii) a linking/trans-membrane domain; and
   (iv) an endodomain

   wherein the antigen binding domains of the first and second CAR bind to different antigens, and wherein the first CAR is directed against the group C group 5 member D orphan G protein-coupled receptor and the second CAR is directed against B cell maturation antigen.

2. A set of at least two nucleic acid sequences comprising nucleic acid sequences encoding the first and the second CAR as defined in claim 1.

3. The set according to claim 2,

   wherein the first CAR encoding sequence comprises a sequence set forth in SEQ NO ID: 2, or a variant thereof having at least 80% sequence identity, and the second CAR encoding sequence comprises a sequence set forth in SEQ NO ID: 4, or a variant thereof having at least 80% sequence identity,
   and wherein the said first and second CAR encoding sequences, when expressed in a T cell, present the said non-identical first and second CAR independently co-expressed on a T cell surface.

4. A system of two vectors, comprising a first vector and a second vector, wherein the first vector comprises a nucleic acid sequence encoding the first CAR as defined in claim 1, wherein the said first vector has the following structure

   anti-GPRC5D scFv (ET150-8) h (ICOS-4-1BB-CD3$\zeta$), in which
   anti-GPRC5D scFv is the nucleic acid sequence encoding the antigen binding domain of the first CAR,
   ICOS is the nucleic acid sequence encoding the transmembrane domain of the first CAR, and 4-1BB-CD3$\zeta$ is the nucleic acid sequence encoding the endodomain of the first CAR,
   and the second vector comprises a nucleic acid sequence encoding the second CAR as defined in claim 1, wherein the second vector has the following structure
   anti-BCMA scFv (25D2) h (CD28-OX40-CD3$\zeta$), in which
   anti-BCMA scFv is the nucleic acid sequence encoding the antigen binding domain of the second CAR,
   CD28 is the nucleic acid sequence encoding the transmembrane domain of the second CAR, and
   OX40-CD3$\zeta$ is the nucleic acid sequence encoding the endodomain of the second CAR.

5. The system according to claim 4, wherein the first vector comprises the first nucleic acid sequence comprising a sequence as set forth in SEQ NO ID: 1 or a variant thereof having at least 80% sequence identity, and the second vector comprises the second nucleic acid sequence comprising a sequence as set forth in SEQ NO ID: 3 or a variant thereof having at least 80% sequence identity.

6. A kit which comprises

    (i) a first nucleic acid sequence encoding a first CAR as defined in claim 1, and
    (ii) a second nucleic acid sequence encoding a second CAR as defined in claim 1,

    and wherein the kit further comprises instructions for use.

7. The kit according to claim 6, wherein the said kit comprises: the first nucleic acid sequence the second nucleic acid sequence as defined in claims 2 or 3 or comprises a first vector and a second vector as defined in claim 4 or 5.

8. A method for generating a T cell according to claim 1, which comprises a step of introducing into a T cell *ex vivo* a set of nucleic acid sequences as defined in claim 2 or 3.

9. The method according to claim 8, wherein the step of introducing the set of nucleic acid sequences into a T cell ex vivo is carried out via a process of viral transduction, preferably using lentivirus particles and a system of vectors as defined in claim 4 or 5.

10. The method according to claim 8 or 9, wherein said method comprises additional steps of cell activation, specific interleukin stimulations, and/or cell expansion following before the step as specified in claim 8 is carried out.

11. A composition comprising a population of human T cells comprising a plurality of the T cells according to claim 1.

12. An immunotherapeutic composition comprising an antitumor effective amount of the composition of claims 11.

13. The composition according to claim 12 for use in treating and/or preventing a disease, preferably cancer.

14. A system of at least two CARs for expression on a T cell surface, comprising a first CAR and a second CAR, wherein each CAR comprises:

    (i) an extracellular antigen-binding domain;
    (ii) a spacer
    (iii) a linking/trans-membrane domain; and
    (iv) an endodomain

    and wherein the antigen binding domains of the first and second CARs bind to different antigens, and wherein the first CAR is directed against the group C group 5 member D orphan G protein-coupled receptor and the other second CAR is directed against B cell maturation antigen.

15. The system according to claim 14, wherein the first CAR is encoded by a nucleic acid sequence comprising a sequence as set forth in SEQ NO ID: 2, or a variant thereof having at least 80% sequence identity, and the second CAR is encoded by a second nucleic acid sequence comprising a sequence as set forth in SEQ NO ID: 4, or a variant thereof having at least 80% sequence identity,

    and said the first and second nucleic acid sequences, when expressed in a T cell, encode non-identical said the first and second CARs independently co-expressed on a T cell surface,
    or
    wherein the first CAR encoding sequence is a part of a first vector having the following structure
    anti-GPRC5D scFv (ET150-8) h (ICOS-4-1BB-CD3ζ), in which
    anti-GPRC5D scFv is the nucleic acid sequence encoding the antigen binding domain of the first CAR,
    ICOS is the nucleic acid sequence encoding the transmembrane domain of the first CAR, and 4-1BB-CD3ζ is the nucleic acid sequence encoding the endodomain of the first CAR,
    and the second CAR encoding sequence is a part of a second vector having the following structure
    anti-BCMA scFv (25D2) h (CD28-OX40-CD3ζ), in which
    anti-BCMA scFv is the nucleic acid sequence encoding the antigen binding domain of the second CAR,
    CD28 is the nucleic acid sequence encoding the transmembrane domain of the second CAR, and
    OX40-CD3ζ is the nucleic acid sequence encoding the endodomain of the second CAR.

FIG. 1

FIG. 2

FIG. 3

FIG. 4

Lane 1: Plasmid

Lane 2: Plasmid Digested with XhoI-XbaI

Lane M: DNA Marker

FIG. 5

FIG. 6

Lane 1: Plasmid

Lane 2: Plasmid Digested with XhoI-XbaI

Lane M: DNA Marker

FIG. 7

EP 4 520 346 A1

FIG. 8

FIG. 9

## Standard curve - qPCR

$y = -3{,}253x + 34{,}997$
$R^2 = 0{,}9999$

Cq vs Log DNA copies/mL

| DNA Copies/mL | log DNA copies/mL | Cq |
|---|---|---|
| 5000 | 3,698970004 | 23,03 |
| 50000 | 4,698970004 | 19,66 |
| 500000 | 5,698970004 | 16,4 |
| 5000000 | 6,698970004 | 13,21 |
| 50000000 | 7,698970004 | 9,99 |

| PCR effidency E | E=10^(-1/-3,253) |
|---|---|
| | 2,029591082 |

| % PCE efficiency | %E=(E-1)*100 |
|---|---|
| | 102,9591082 |
| | 102,96% |

| | after exponentiation | copies/ml | copies/ml |
|---|---|---|---|
| BCMA | 1065024 | 532511958 | 5,33E+08 |
| BCMA x0.1 | 283469 | 141734437 | 1,42E+08 |
| BCMA x0.01 | 30276 | 15137856 | 1,51E+07 |
| K | 1 | 326 | 3,26E+02 |

FIG. 10

## Standard curve - qPCR

| DNA Copies/mL | log DNA copies/mL | Cq |
|---|---|---|
| 5000 | 3,698970004 | 23,03 |
| 50000 | 4,698970004 | 19,66 |
| 500000 | 5,698970004 | 16,4 |
| 5000000 | 6,698970004 | 13,21 |
| 50000000 | 7,698970004 | 9,99 |

| | | |
|---|---|---|
| PCR efficiency E | E=10^(-1/-3,253) | |
| | 2,029591082 | |

| | | |
|---|---|---|
| % PCE efficiency | %E=(E-1)*100 | |
| | 102,9591082 | |
| | 102,96% | |

| | after exponentiation | copies/ml | copies/ml |
|---|---|---|---|
| GPRC5D | 1930112 | 965056063 | 9,65E+08 |
| GPRC5D x0.1 | 445910 | 222954814 | 2,23E+08 |
| GPRC5D x0.01 | 47625 | 23812547 | 2,38E+07 |
| K | 1 | 326 | 3,26E+02 |

FIG. 11

FIG. 12

FIG. 13

FIG. 14

FIG. 15

Manual and CliniMACS Prodigy cell culture system
Dual CAR-T Anti-BCMA and Anti-GPRC5D MOI 50:50, GFP MOI 150

FIG. 16

FIG. 17

anty-BCMA

Sequence ID: Query_45047  Length: 9634  Number of Matches: 1

Range 1: 3203 to 4736 Graphics

| Score | Expect | Identities | Gaps | Strand |
|---|---|---|---|---|
| 2767 bits(3068) | 0.0 | 1534/1534(100%) | 0/1534(0%) | Plus/Plus |

FIG. 18

▼ Download ⌄   Graphics                                 ▼ Next ▲ Previous ◀Descriptions

anty-GPR5D

Sequence ID: Query_1789  Length: 9652  Number of Matches: 1

Range 1: 3232 to 4717 Graphics                        ▼ Next Match ▲ Previous Match

| Score | Expect | Identities | Gaps | Strand |
|---|---|---|---|---|
| 2651 bits(2972) | 0.0 | 1486/1486(100%) | 0/1486(0%) | Plus/Plus |

```
Query  1     GCTTGGCACTTGATGTAATTCTCCTTGGAATTTGCCCTTTTTGAGTTTGGATCTTGGTTC  60
             ||||||||||||||||||||||||||||||||||||||||||||||||||||||||||||
Sbjct  3232  GCTTGGCACTTGATGTAATTCTCCTTGGAATTTGCCCTTTTTGAGTTTGGATCTTGGTTC  3291

Query  61    ATTCTCAAGCCTCAGACAGTGGTTCAAAG--------CTTCCATTTCAGGTGTCGTGATTC  120
             |||||||||||||||||||||||||||||        |||||||||||||||||||||||
Sbjct  3292  ATTCTCAAGCCTCAGACAGTGGTTCAAAGTTTTTTCTTCCATTTCAGGTGTCGTGATTC  3351

Query  121   GAAGCCGCCACCATGGCCTTACCAGTGACCGCCTTGCTCCTGCCGCTGGCCTTGCTGCTC  180
             ||||||||||||||||||||||||||||||||||||||||||||||||||||||||||||
Sbjct  3352  GAAGCCGCCACCATGGCCTTACCAGTGACCGCCTTGCTCCTGCCGCTGGCCTTGCTGCTC  3411

Query  181   CACGCCGCCAGGCCGAGCTCCGAGCTGACCCAGGATCCCGCCGTGAGCGTGGCCCTGGGA  240
             ||||||||||||||||||||||||||||||||||||||||||||||||||||||||||||
Sbjct  3412  CACGCCGCCAGGCCGAGCTCCGAGCTGACCCAGGATCCCGCCGTGAGCGTGGCCCTGGGA  3471

Query  241   CAGACAGTGAGGATCACCTGTCAGGGCGACTCCCTGAGATCCTACTACGCCTCCTGGTAC  300
             ||||||||||||||||||||||||||||||||||||||||||||||||||||||||||||
Sbjct  3472  CAGACAGTGAGGATCACCTGTCAGGGCGACTCCCTGAGATCCTACTACGCCTCCTGGTAC  3531

Query  301   CAGCAGAAGCCCGGCCAGGCCCCCGTGCTGGTTATCTACGGCAAGAATAATAGGCCTAGC  360
             ||||||||||||||||||||||||||||||||||||||||||||||||||||||||||||
Sbjct  3532  CAGCAGAAGCCCGGCCAGGCCCCCGTGCTGGTTATCTACGGCAAGAATAATAGGCCTAGC  3591

Query  361   GGCATCCCTGATAGATTTTCCGGCAGCTCCAGCGGCAATACCGCCAGCCTGACCATCACC  420
             ||||||||||||||||||||||||||||||||||||||||||||||||||||||||||||
Sbjct  3592  GGCATCCCTGATAGATTTTCCGGCAGCTCCAGCGGCAATACCGCCAGCCTGACCATCACC  3651

Query  421   GGCGCCCAGGCTGAGGACGAGGCCGATTACTACTGCAATTCCAGGGACAGCAGCGGCAAT  480
             ||||||||||||||||||||||||||||||||||||||||||||||||||||||||||||
Sbjct  3652  GGCGCCCAGGCTGAGGACGAGGCCGATTACTACTGCAATTCCAGGGACAGCAGCGGCAAT  3711

Query  481   CCTCCTGTGGTGTTTGGCGGCGGCACCAAGCTGACAGTGCTGGGCAGCAGGGCGGCGGA  540
             ||||||||||||||||||||||||||||||||||||||||||||||||||||||||||||
Sbjct  3712  CCTCCTGTGGTGTTTGGCGGCGGCACCAAGCTGACAGTGCTGGGCAGCAGGGCGGCGGA  3771

Query  541   GGATCTGGAGGAGGCGGATCCGGCGGCGGAGGTTCTCTGGAGATGGCCCAGGTGCAGCTG  600
             ||||||||||||||||||||||||||||||||||||||||||||||||||||||||||||
Sbjct  3772  GGATCTGGAGGAGGCGGATCCGGCGGCGGAGGTTCTCTGGAGATGGCCCAGGTGCAGCTG  3831

Query  601   GTGGAGAGCGGCGGAGGCCTGGTGCACCCAGGAGGATCCCTGAGACTGAGCTGTGCCGCC  660
             ||||||||||||||||||||||||||||||||||||||||||||||||||||||||||||
Sbjct  3832  GTGGAGAGCGGCGGAGGCCTGGTGCACCCAGGAGGATCCCTGAGACTGAGCTGTGCCGCC  3891

Query  661   TCCGGCTTCACCTTTAGGTCCCACAGCATGAATTGGGTGAGACAGGCCCCTGGCAAGGGC  720
             ||||||||||||||||||||||||||||||||||||||||||||||||||||||||||||
Sbjct  3892  TCCGGCTTCACCTTTAGGTCCCACAGCATGAATTGGGTGAGACAGGCCCCTGGCAAGGGC  3951

Query  721   CTGGAGTGGGTGTCCAGCATCAGCAGCGACAGCACCTACACCTACTACGCCGATAGCGTG  780
             ||||||||||||||||||||||||||||||||||||||||||||||||||||||||||||
Sbjct  3952  CTGGAGTGGGTGTCCAGCATCAGCAGCGACAGCACCTACACCTACTACGCCGATAGCGTG  4011

Query  781   AAGGGCAGGTTCACCATCTCCAGAGACAACGCCAAGAATAGCCTGTACCTGCAGATGAAC  840
             ||||||||||||||||||||||||||||||||||||||||||||||||||||||||||||
Sbjct  4012  AAGGGCAGGTTCACCATCTCCAGAGACAACGCCAAGAATAGCCTGTACCTGCAGATGAAC  4071

Query  841   AGCCTGAGGGCCGAGGATACCGGCGTGTACTACTGCGCCAGAAGCGGCGGCCAGTGGAAG  900
             ||||||||||||||||||||||||||||||||||||||||||||||||||||||||||||
Sbjct  4072  AGCCTGAGGGCCGAGGATACCGGCGTGTACTACTGCGCCAGAAGCGGCGGCCAGTGGAAG  4131

Query  901   TACTACGACTACTGGGGCCAGGGCACACTGGTGACAGTGAGCTCCACCACTACCCCAGCA  960
             ||||||||||||||||||||||||||||||||||||||||||||||||||||||||||||
Sbjct  4132  TACTACGACTACTGGGGCCAGGGCACACTGGTGACAGTGAGCTCCACCACTACCCCAGCA  4191

Query  961   CCGAGGCCACCCACCCGGCTCCTACCATCGCCTCCCAGCCTCTGTCCCTGCGTCCGGAG  1020
             ||||||||||||||||||||||||||||||||||||||||||||||||||||||||||||
Sbjct  4192  CCGAGGCCACCCACCCGGCTCCTACCATCGCCTCCCAGCCTCTGTCCCTGCGTCCGGAG  4251

Query  1021  GCATGTAGACCCGCAGCTGGTGGGGCCGTGCATACCCGGGGTCTTGACTTCGCCTGCGAT  1080
             ||||||||||||||||||||||||||||||||||||||||||||||||||||||||||||
Sbjct  4252  GCATGTAGACCCGCAGCTGGTGGGGCCGTGCATACCCGGGGTCTTGACTTCGCCTGCGAT  4311

Query  1081  TTCTGGCTGCCCATCGGCTGCGCCGCCTTCGTGGTGGTGTGTATCCTGGGCTGTATCCTG  1140
             ||||||||||||||||||||||||||||||||||||||||||||||||||||||||||||
Sbjct  4312  TTCTGGCTGCCCATCGGCTGCGCCGCCTTCGTGGTGGTGTGTATCCTGGGCTGTATCCTG  4371

Query  1141  ATCTGTTGGCTGACAAAGAAGAAGTACTCCAGCTCCGTGCACGATCCCAATGGCGAGTAC  1200
             ||||||||||||||||||||||||||||||||||||||||||||||||||||||||||||
Sbjct  4372  ATCTGTTGGCTGACAAAGAAGAAGTACTCCAGCTCCGTGCACGATCCCAATGGCGAGTAC  4431

Query  1201  ATGTTTATGAGAGCCGTGAATACCGCCAAGAAGAGCAGACTGACCGACGTGACCCTGAAG  1260
             ||||||||||||||||||||||||||||||||||||||||||||||||||||||||||||
Sbjct  4432  ATGTTTATGAGAGCCGTGAATACCGCCAAGAAGAGCAGACTGACCGACGTGACCCTGAAG  4491

Query  1261  CGCGGTCGGAAGAAGCTGCTGTACATCTTTAAGCAACCCTTCATGAGGCCTGTGCAGACT  1320
             ||||||||||||||||||||||||||||||||||||||||||||||||||||||||||||
Sbjct  4492  CGCGGTCGGAAGAAGCTGCTGTACATCTTTAAGCAACCCTTCATGAGGCCTGTGCAGACT  4551

Query  1321  ACTCAAGAGGAGGACGGCTGTTCATGCCGGTTCCCAGAGGAGGAGGAGGGCGGCTGCGAA  1380
             ||||||||||||||||||||||||||||||||||||||||||||||||||||||||||||
Sbjct  4552  ACTCAAGAGGAGGACGGCTGTTCATGCCGGTTCCCAGAGGAGGAGGAGGGCGGCTGCGAA  4611

Query  1381  CTGCGCGTGAAATTCAGCCGCAGCGCAGATGCTCCAGCCTACAAGCAGGGGCAGAACCAG  1440
             ||||||||||||||||||||||||||||||||||||||||||||||||||||||||||||
Sbjct  4612  CTGCGCGTGAAATTCAGCCGCAGCGCAGATGCTCCAGCCTACAAGCAGGGGCAGAACCAG  4671

Query  1441  CTCTACAACGAACTCAATCTTGGTCGGAGAGGAGGAGTACGACGTGC  1486
             |||||||||||||||||||||||||||||||||||||||||||||||
Sbjct  4672  CTCTACAACGAACTCAATCTTGGTCGGAGAGGAGGAGTACGACGTGC  4717
```

FIG. 19

FIG. 20

anty-GPR5D

Sequence ID: Query_58111 Length: 9652 Number of Matches: 1

Range 1: 3239 to 4725 Graphics

| Score | Expect | Identities | Gaps | Strand |
|---|---|---|---|---|
| 2682 bits(2974) | 0.0 | 1487/1487(100%) | 0/1487(0%) | Plus/Plus |

FIG. 21

FIG. 22

FIG. 23

FIG. 24

FIG. 25

FIG. 26

FIG. 27

FIG. 28

1. Non-transduced T cells (negative control) (Evos Floid)

2. Dual CAR-T (Evos Floid)

3. Mono Anti-BCMA CAR-T (Evos Floid)

4. Non-transduced T cells (negative control) (Evos Floid)

5. GFP CAR-T (Evos Floid)

6. H929 (Evos Floid)

7. Dual CAR-T : H929 1:2 (Evos Floid)

8. Dual CAR-T : H929 1:5 (Evos Floid)

9. Dual CAR-T : H929 1:10 (Evos Floid)

10. Mono Anti-BCMA CAR-T : H929 1:2 (Evos Floid)

11. Mono Anti-BCMA CAR-T : H929 1:5 (Evos Floid)

12. Mono Anti-BCMA CAR-T : H929 1:10 (Evos Floid)

FIG. 29

13. Non-transduced T cells : H929 1:2 (Evos Floid)

14. Non-transduced T cells : H929 1:5 (Evos Floid)

15. Non-transduced : H929 1:10 (Evos Floid)

16. GFP CAR-T : H929 1:2 (Evos Floid)

17. GFP CAR-T : H929 1:5 (Evos Floid)

18. GFP CAR-T : H929 1:10 (Evos Floid)

19. Non-transduced T cells : H929 1:2 (Evos Floid)

20. Non-transduced T cells : H929 1:5 (Evos Floid)

21. Non-transduced T cells : H929 1:10 (Evos Floid)

FIG. 29 continuation

1. Non-transduced T cells (negative control) (Evos Floid)

2. Dual CAR-T (Evos Floid)

3. Mono Anti-BCMA CAR-T (Evos Floid)

4. Non-transduced T cells (negative control) (Evos Floid)

5. H929 (Evos Floid)

6. Dual CAR-T : H929 1:2 (Evos Floid)

7. Dual CAR-T : H929 1:5 (Evos Floid)

8. Dual CAR-T : H929 1:10 (Evos Floid)

9. Mono Anti-BCMA CAR-T : H929 1:2 (Evos Floid)

10. Mono Anti-BCMA CAR-T : H929 1:5 (Evos Floid)

11. Mono Anti-BCMA CAR-T : H929 1:10 (Evos Floid)

FIG. 30

12. Non-transduced T cells : H929 1:2 (Evos Floid)

13. Non-transduced T cells : H929 1:5 (Evos Floid)

14. Non-transduced T cells : H929 1:10 (Evos Floid)

15. GFP CAR-T : H929 1:2 (Evos Floid)

16. GFP CAR-T : H929 1:5 (Evos Floid)

17.GFP CAR-T : H929 1:10 (Evos Floid)

18. Non-transduced T cells : H929 1:2 (Evos Floid)

19. Non-transduced T cells : H929 1:5 (Evos Floid)

20. Non-transduced T cells : H929 1:10 (Evos Floid)

FIG. 30 continuation

FIG. 31

A – INFγ

B – TNF

C – IL-10

D – IL-4

E – IL-2

F – IL-5

FIG. 32

Europäisches
Patentamt

European
Patent Office

Office européen
des brevets

# EUROPEAN SEARCH REPORT

Application Number

EP 23 46 1647

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2020/092854 A2 (JUNO THERAPEUTICS INC [US] ET AL.) 7 May 2020 (2020-05-07) | 1,2,6-14 | INV. A61K39/00 C07K14/705 C07K14/725 |
| Y | * the whole document * | 3-5,15 | |
| X | FERN?NDEZ DE LARREA CARLOS ET AL: "Defining an Optimal Dual-Targeted CAR T-cell Therapy Approach Simultaneously Targeting BCMA and GPRC5D to Prevent BCMA Escape-Driven Relapse in Multiple Myeloma", BLOOD CANCER DISCOVERY, vol. 1, no. 2, 6 July 2020 (2020-07-06), pages 146-154, XP093102949, ISSN: 2643-3230, DOI: 10.1158/2643-3230.BCD-20-0020 Retrieved from the Internet: URL:https://aacrjournals.org/bloodcancerdiscov/article-pdf/1/2/146/3109369/146.pdf> | 1,2,6-14 | |
| Y | * the whole document * | 3-5,15 | |
| Y | WO 2016/090312 A1 (SLOAN KETTERING INST CANCER [US]; EUREKA THERAPEUTICS INC [US]) 9 June 2016 (2016-06-09) * the whole document * | 3-5,15 | TECHNICAL FIELDS SEARCHED (IPC) A61K C07K |
| Y | WO 2018/133877 A1 (CARSGEN THERAPEUTICS CO LTD [CN]) 26 July 2018 (2018-07-26) * the whole document * | 3-5,15 | |

The present search report has been drawn up for all claims

| Place of search | Date of completion of the search | Examiner |
|---|---|---|
| The Hague | 7 March 2024 | Manu, Dominique |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

................................................................

& : member of the same patent family, corresponding
    document

EPO FORM 1503 03.82 (P04C01)

## EP 4 520 346 A1

### ANNEX TO THE EUROPEAN SEARCH REPORT
### ON EUROPEAN PATENT APPLICATION NO.

EP 23 46 1647

07-03-2024

| Patent document cited in search report | Publication date | Patent family member(s) | Publication date |
|---|---|---|---|
| WO 2020092854 A2 | 07-05-2020 | AU 2019374103 A1 | 20-05-2021 |
| | | CA 3116413 A1 | 07-05-2020 |
| | | CL 2021001153 A1 | 07-01-2022 |
| | | CN 113614108 A | 05-11-2021 |
| | | CO 2021007254 A2 | 30-08-2021 |
| | | EP 3873937 A2 | 08-09-2021 |
| | | IL 282666 A | 30-06-2021 |
| | | JP 2022506598 A | 17-01-2022 |
| | | KR 20210102888 A | 20-08-2021 |
| | | MA 54079 A | 08-09-2021 |
| | | PE 20211058 A1 | 07-06-2021 |
| | | SG 11202103873V A | 28-05-2021 |
| | | TW 202021981 A | 16-06-2020 |
| | | US 2021393689 A1 | 23-12-2021 |
| | | WO 2020092854 A2 | 07-05-2020 |
| WO 2016090312 A1 | 09-06-2016 | AU 2015357518 A1 | 29-06-2017 |
| | | AU 2022200052 A1 | 03-02-2022 |
| | | BR 112017011920 A2 | 27-02-2018 |
| | | CA 2969864 A1 | 09-06-2016 |
| | | CN 107428829 A | 01-12-2017 |
| | | CN 113683711 A | 23-11-2021 |
| | | CN 113968912 A | 25-01-2022 |
| | | CY 1125008 T1 | 22-07-2022 |
| | | DK 3227339 T3 | 10-01-2022 |
| | | EP 3227339 A1 | 11-10-2017 |
| | | EP 4015534 A1 | 22-06-2022 |
| | | ES 2899779 T3 | 14-03-2022 |
| | | HR P20211978 T1 | 01-04-2022 |
| | | HU E057777 T2 | 28-06-2022 |
| | | JP 6919091 B2 | 18-08-2021 |
| | | JP 2017537629 A | 21-12-2017 |
| | | JP 2021040652 A | 18-03-2021 |
| | | JP 2023091037 A | 29-06-2023 |
| | | KR 20170109537 A | 29-09-2017 |
| | | LT 3227339 T | 10-01-2022 |
| | | MY 192065 A | 25-07-2022 |
| | | NZ 732574 A | 24-11-2023 |
| | | PH 12017501044 A1 | 05-03-2018 |
| | | PL 3227339 T3 | 21-02-2022 |
| | | PT 3227339 T | 14-01-2022 |
| | | RS 62870 B1 | 28-02-2022 |
| | | RU 2017123551 A | 14-01-2019 |
| | | SA 517381665 B1 | 24-01-2022 |
| | | SG 10202007326Q A | 28-08-2020 |
| | | SG 11201704547R A | 28-07-2017 |

## ANNEX TO THE EUROPEAN SEARCH REPORT
## ON EUROPEAN PATENT APPLICATION NO.

EP 23 46 1647

This annex lists the patent family members relating to the patent documents cited in  the above-mentioned European search report.
The members are as contained in the European Patent Office EDP file on
The European Patent Office is in no way liable for these particulars which are merely given for the purpose of information.

07-03-2024

| Patent document cited in search report | Publication date | Patent family member(s) | | Publication date |
|---|---|---|---|---|
| | | SI | 3227339 T1 | 28-02-2022 |
| | | US | 2018118803 A1 | 03-05-2018 |
| | | US | 2020270326 A1 | 27-08-2020 |
| | | US | 2020270327 A1 | 27-08-2020 |
| | | US | 2020270328 A1 | 27-08-2020 |
| | | WO | 2016090312 A1 | 09-06-2016 |
| WO 2018133877 A1 | 26-07-2018 | AU | 2018209012 A1 | 12-09-2019 |
| | | BR | 112019014986 A2 | 28-04-2020 |
| | | CA | 3050835 A1 | 26-07-2018 |
| | | CL | 2019002036 A1 | 04-10-2019 |
| | | CN | 108341872 A | 31-07-2018 |
| | | CN | 111574628 A | 25-08-2020 |
| | | EP | 3572427 A1 | 27-11-2019 |
| | | IL | 268206 A | 26-09-2019 |
| | | JP | 7280828 B2 | 24-05-2023 |
| | | JP | 2020506688 A | 05-03-2020 |
| | | KR | 20190130562 A | 22-11-2019 |
| | | NZ | 756635 A | 27-10-2023 |
| | | RU | 2019123366 A | 24-02-2021 |
| | | SG | 11201906780S A | 27-08-2019 |
| | | US | 2019359726 A1 | 28-11-2019 |
| | | WO | 2018133877 A1 | 26-07-2018 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018200582 A1
- WO 2015142675 A2
- WO 2017172981 A2
- US 11208455 B2
- EP 3071223 B1

**Non-patent literature cited in the description**

- **BHATT P ; KLOOCK C ; COMENZO R.** Relapsed/Refractory Multiple Myeloma: A Review of Available Therapies and Clinical Scenarios Encountered in Myeloma Relapse.. *Curr Oncol.*, 15 February 2023, vol. 30 (2), 2322-2347
- The EBMT/EHA CAR-T Cell Handbook
- **MAZINANI, M. ; RAHBARIZADEH, F.** CAR-T cell potency: from structural elements to vector backbone components.. *Biomark Res*, 2022, vol. 10, 70
- **CHOI T ; KANG Y.** Chimeric antigen receptor (CAR) T-cell therapy for multiple myeloma.. *Pharmacol Ther.*, April 2022, vol. 232, 108007
- **BRUNO B et al.** European Myeloma Network perspective on CAR T-Cell therapies for multiple myeloma.. *Haematologica.*, 01 August 2021, vol. 106 (8), 2054-2065
- **HUANG J ; HUANG X ; HUANG J.** CAR-T cell therapy for hematological malignancies: Limitations and optimization strategies. *Front Immunol.*, 28 September 2022, vol. 13, 1019115
- **LEOW CC ; LOW MSY**. Targeted Therapies for Multiple Myeloma.. *J Pers Med.*, 23 April 2021, vol. 11 (5), 334
- **ZHANG X ; ZHANG H ; LAN H ; WU J ; XIAO Y.** CAR-T cell therapy in multiple myeloma: Current limitations and potential strategies. *Front Immunol.*, 20 February 2023, vol. 14, 1101495
- **YU B ; JIANG T ; LIU D.** BCMA-targeted immunotherapy for multiple myeloma.. *J Hematol Oncol.*, 17 September 2020, vol. 13 (1), 125
- *Future Oncol.*, January 2022, vol. 18 (3), 277-289
- **CHEKOL ABEBE E ; YIBELTAL SHIFERAW M ; TADELE ADMASU F ; ASMAMAW DEJENIE T.** Ciltacabtagene autoleucel: The second anti-BCMA CAR T-cell therapeutic armamentarium of relapsed or refractory multiple myeloma. *Front Immunol.*, 02 September 2022, vol. 13, 991092
- **RAJKUMAR SV**. Multiple myeloma: 2022 update on diagnosis, risk stratification, and management.. *Am J Hematol.*, August 2022, vol. 97 (8), 1086-1107
- **HONIKEL MM ; OLEJNICZAK SH.** Co-Stimulatory Receptor Signaling in CAR-T Cells.. *Biomolecules.*, 15 September 2022, vol. 12 (9), 1303
- **GUERCIO M et al.** CD28.OX40 co-stimulatory combination is associated with long in vivo persistence and high activity of CAR.CD30 T-cells. *Haematologica*, 2020, vol. 106 (4), 987-999
- **CAPPELL, K.M. ; KOCHENDERFER, J.N.** A comparison of chimeric antigen receptors containing CD28 versus 4-1BB costimulatory domains.. *Nat Rev Clin Oncol*, 2021, vol. 18, 715-727
- **ALIYA I SANI ; ZIL-E-RUBAB ; SHUMAILA USMAN ; SYED ZARYAB AHMED ; MERVYN HOSEIN.** Role of OX40 and its ligand as costimulatory modulators in cancer immunotherapy.. *AIMS Molecular Science*, 2021, vol. 8 (3), 161-173
- **GUERCIO M et al.** CD28.OX40 co-stimulatory combination is associated with long in vivo persistence and high activity of CAR.CD30 T-cells.. *Haematologica*, 2020, vol. 106 (4), 987-999
- **MAILANKODY S et al.** Phase I First-in-Class Trial of MCARH109, a G Protein Coupled Receptor Class C Group 5 Member D (GPRC5D) Targeted CAR T Cell Therapy in Patients with Relapsed or Refractory Multiple Myeloma.. *Blood*, 2021, vol. 138 (1), 827
- **WANG Z ; CHEN C ; WANG L ; JIA Y ; QIN Y.** Chimeric antigen receptor T-cell therapy for multiple myeloma. *Front Immunol.*, 22 December 2022, vol. 13, 1050522
- **TAN, J. ; JIA, Y ; ZHOU, M. et al.** Chimeric antigen receptors containing the OX40 signalling domain enhance the persistence of T cells even under repeated stimulation with multiple myeloma target cells.. *J Hematol Oncol*, 2022, vol. 15, 39
- **CAPPELL KM ; KOCHENDERFER JN.** A comparison of chimeric antigen receptors containing CD28 versus 4-1BB costimulatory domains.. *Nat Rev Clin Oncol.*, November 2021, vol. 18 (11), 715-727

- **DAI Q et al.** 4-1BB Signaling Boosts the Anti-Tumor Activity of CD28-Incorporated 2nd Generation Chimeric Antigen Receptor-Modified. *T Cells. Front. Immunol.*, vol. 11, 539654
- **MASCARELLI DE** ; **ROSA RSM** ; **TOSCARO JM** ; **SEMIONATTO IF** ; **RUAS LP** ; **FOGAGNOLO CT** ; **LIMA GC** ; **BAJGELMAN MC**. Boosting Antitumor Response by Costimulatory Strategies Driven to 4-1BB and OX40 T-cell Receptors. *Front. Cell Dev. Biol.*, 2021, vol. 9, 692982
- Front. Immunol.. *Sec. Cancer Immunity and Immunotherapy*, 20 February 2019, vol. 10
- *Blood*, 2013, vol. 121 (4), 573-584
- **ZHOU J** ; **JIN L** ; **WANG F** ; **ZHANG Y** ; **LIU B** ; **ZHAO T.** Chimeric antigen receptor T (CAR-T) cells expanded with IL-7/IL-15 mediate superior antitumor effects.. *Protein Cell.*, October 2019, vol. 10 (10), 764-769
- **GHAFFARI, S.** ; **TORABI-RAHVAR, M.** ; **AGHAYAN, S. et al.** Optimizing interleukin-2 concentration, seeding density and bead-to-cell ratio of T-cell expansion for adoptive immunotherapy. *BMC Immunol*, 2021, vol. 22 (43)
- *J Exp Med.*, 01 February 2021, vol. 218 (2), e20192203
- *Blood*, 2003, vol. 102 (2), 497-505
- **LO PRESTI V** ; **CORNEL AM** ; **PLANTINGA M** ; **DÜNNEBACH E** ; **KUBALL J** ; **BOELENS JJ** ; **NIERKENS S** ; **VAN TIL NP**. Efficient lentiviral transduction method to gene modify cord blood CD8+ T cells for cancer therapy applications. *Mol Ther Methods Clin Dev.*, 23 March 2021, vol. 21, 357-368
- **GARRIDO-RODRIGUEZ V** ; **HERRERO-FERNÁNDEZ I** ; **CASTRO MJ** ; **CASTILLO A** ; **ROSADO-SÁNCHEZ I** ; **GALVÁ MI** ; **RAMOS R** ; **OLIVAS-MARTINEZ I** ; **BULNES-RAMOS A** ; **CAÑIZARES J**. Immunological features beyond CD4/CD8 ratio values in older individuals. *Aging*, 26 May 2021, vol. 13 (10), 13443-13459
- **HUANG, X.** ; **MADAN, A.** CAP3: A DNA sequence assembly program. *Genome Res.*, 1999, vol. 9, 868-877